(19) Europäisches Patentamt
European Patent Office
Office européen des brevets

(11) Numéro de publication : **0 382 629 B1**

(12) # FASCICULE DE BREVET EUROPEEN

(45) Date de publication du fascicule du brevet : **24.11.93 Bulletin 93/47**

(21) Numéro de dépôt : **90400325.8**

(22) Date de dépôt : **06.02.90**

(51) Int. Cl.[5] : **C07C 317/22,** A61K 31/135, C07D 295/088, A61K 31/495, C07D 207/36, C07D 307/82, C07D 209/43, A61K 31/34, A61K 31/40, A61K 31/415, // (C07D463/00, 231:00, 221:00), (C07D221/00, 209:00)

(54) **Dérivés aminoalkoxyphényle, leur procédé de préparation ainsi que les compositions en contenant.**

(30) Priorité : **07.02.89 FR 8901554**

(43) Date de publication de la demande : **16.08.90 Bulletin 90/33**

(45) Mention de la délivrance du brevet : **24.11.93 Bulletin 93/47**

(84) Etats contractants désignés : **AT BE CH DE DK ES FR GB GR IT LI LU NL SE**

(56) Documents cités :
**EP-A- 0 235 111**
**EP-A- 0 302 792**
**US-A- 4 117 128**
**JOURNAL OF MEDICINAL CHEMISTRY, vol. 21, no. 2, février 1978, pages 182-188, American Chemical Society, Washington, US; M.T. COX et al.: "Linked aryl aryloxypropanolamines as a new class of lipid catabolic agents"**

(73) Titulaire : **ELF SANOFI**
**32-34, rue Marbeuf**
**F-75008 Paris (FR)**
(84) **CH DE DK ES FR GB GR IT LI LU NL SE AT**
Titulaire : **S.A. SANOFI - PHARMA N.V.**
**Avenue De Béjar, 1**
**B-1120 Bruxelles (BE)**
(84) **BE**

(72) Inventeur : **Gubin, Jean**
**Avenue des Citronniers, 24**
**B-1020 Bruxelles 2 (BE)**
Inventeur : **Chatelain, Pierre**
**Avenue du Haras 111**
**B-1150 Bruxelles (BE)**
Inventeur : **Inion, Henri**
**Molenweg 101**
**B-1810 Bruxelles (BE)**
Inventeur : **Rosseels, Gilbert**
**C. Van Campenhoutstraat 23**
**B-1810 Emmel (BE)**

(74) Mandataire : **Le Guen, Gérard et al**
**CABINET LAVOIX 2, place d'Estienne d'Orves**
**F-75441 Paris Cédex 09 (FR)**

**EP 0 382 629 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention se rapporte, d'une manière générale à de nouveaux dérivés cycliques et plus particulièrement à de nouveaux dérivés aminoalkoxyphényle ainsi qu'à leur procédé de préparation.

Plus particulièrement, les nouveaux dérivés aminoalkoxyphényle de l'invention peuvent être représentés par la formule générale :

Cy-B-Ph-O-A-Am (1)

dans laquelle :

B représente un groupement -S-, -SO- ou $-SO_2-$,

Ph représente un radical de formule :

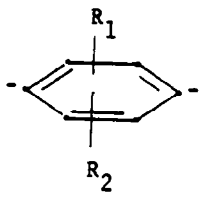

(D)

ou

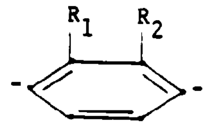

(E)

$R_1$ et $R_2$, qui sont identiques ou différents, représentent chacun l'hydrogène, le radical méthyle ou éthyle ou un halogène tel que chlore, brome ou iode,

A représente un radical alkylène, linéaire ou ramifié, ayant de 2 à 5 atomes de carbone ou le radical hydroxy-2 propylène dans lequel l'hydroxy est éventuellement substitué par un radical alkyle inférieur,

Am représente un groupement :

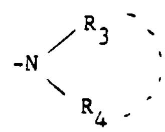

(F)

ou

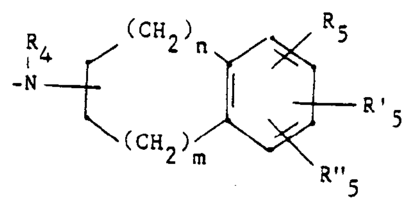

(G)

ou

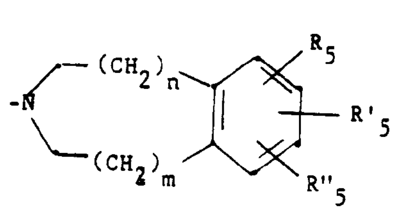

(H)

dans lequel :

$R_3$ représente un radical alkyle, cycloalkyle ou un radical de formule :

-Alk-Ar

dans laquelle Alk représente une liaison simple ou un radical alkylène, linéaire ou ramifié, ayant de 1 à 5 atomes de carbone et Ar représente un radical pyridyle, phényle, méthylènedioxy-2,3 phényle ou méthylènedioxy-3,4 phényle ou un groupement phényle substitué par un ou plusieurs substituants, identiques ou différents, sélectionnés parmi des atomes d'halogène, des groupements alkyles inférieurs ou des groupements alkoxy inférieurs,

$R_4$ représente l'hydrogène, un radical alkyle, ou $R_3$ et $R_4$, lorsqu'ils sont pris ensemble, représentent un radical alkylène ou alkénylène ayant de 3 à 6 atomes de carbone et éventuellement substitué par un radical phényle ou éventuellement interrompu par -O-, -N= ou

$$-\overset{|}{N}-R_6,$$

$R_6$ représentant l'hydrogène, un radical alkyle inférieur, cycloalkyle, phényle éventuellement substitué par un atome d'halogène ou par un groupement alkyle inférieur ou alkoxy inférieur,

$R_6$, $R'_5$ et $R''_5$, qui sont identiques ou différents, représentent chacun l'hydrogène, un atome d'halogène tel que chlore ou brome, un groupement alkyle inférieur ou un groupement alkoxy inférieur, n et m, identiques ou différents, représentent chacun 0,1, 2 ou 3, Cy représente l'un des groupements de formule :

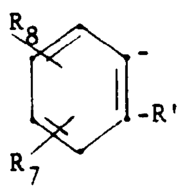

(I)

ou

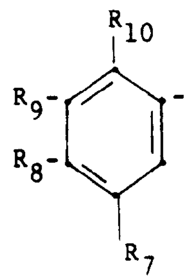

(I')

ou

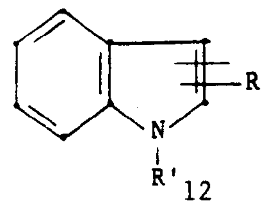

(K')

ou

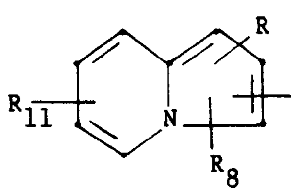

(J)

ou

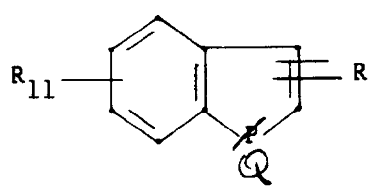

(K)

ou

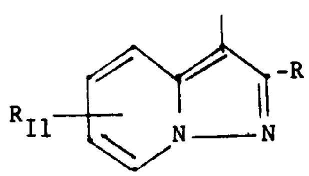

(L)

ou

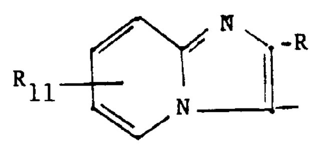

(M)

ou

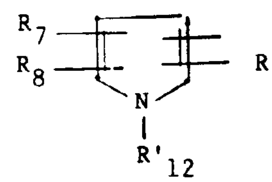

(N)

ou

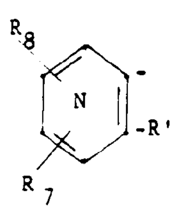

(Q)

ou

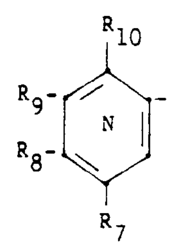

(Q')

dans lequel :

R représente l'hydrogène, un radical alkyle, cycloalkyle, benzyle ou phényle éventuellement substitué par un ou plusieurs substituants, qui peuvent être identiques ou différents, sélectionnés parmi des atomes d'halogène, par exemple fluor, chlore ou brome ou parmi des groupements alkyles inférieurs, alkoxy inférieurs ou nitro,

R' représente un radical alkyle, cycloalkyle, benzyle ou phényle éventuellement substitué par un ou plusieurs substituants, qui peuvent être identiques ou différents, sélectionnés parmi des atomes d'halogène ou parmi des groupements alkyles inférieurs, alkoxy inférieurs ou nitro,

$R_7$, $R_8$ et $R_9$, identiques ou différents, représentent chacun l'hydrogène, un radical alkyle inférieur, alkoxy inférieur, un atome d'halogène tel que chlore ou brome, un groupement hydroxy, benzyloxy, nitro, amino, alkylamino inférieur, dialkylamino inférieur, sulfonamido, alkylsulfonamido inférieur, phénylsulfonamido, cyano, alkoxycarbonyle inférieur ou alkylcarbonyle inférieur,

$R_{10}$ représente l'hydrogène, un radical alkoxy inférieur, un atome d'halogène, un groupement hydroxy, benzyloxy, nitro, amino, alkylamino inférieur, dialkylamino inférieur, sulfonamido, alkylsulfonamido inférieur, phénylsulfonamido, cyano, alkoxycarbonyle inférieur ou alkylcarbonyle inférieur, à condition que :

les groupements $R_7$ et $R_8$ d'une part et $R_7$, $R_8$, $R_9$ et $R_{10}$ d'autre part ne représentant jamais simultanément l'hydrogène, et que le radical (I) ou (I') ne représente pas un radical monohalophényle;

$R_{11}$ représente un radical alkyle inférieur, alkoxy inférieur un atome d'halogène, un grou- pement hydroxy, benzyloxy, nitro, amino, alkylamino inférieur, dialkylamino inférieur, sulfonamido, alkylsulfonamido inférieur, phénylsulfonamido, cyano, alkoxycarbonyle inférieur ou alkylcarbonyle inférieur. Q représente -N-$R_{12}$ dans lequel $R_{12}$ représente l'hydrogène, un groupement alkyle inférieur, cycloalkyle, benzyle, halogénobenzyle, un groupement -A-Am tel que défini précédemment, un groupement alkylsulfonyle inférieur ou phénylsulfonyle éventuellement substitué par un ou plusieurs atomes d'halogène, groupements alkyles inférieurs ou alkoxy inférieurs,

$R'_{12}$ représente un groupement -A-Am tel que défini précédemment, un groupement alkylsulfonyle éventuellement substitué par un ou plusieurs atomes d'halogène, groupements alkyles inférieurs ou alkoxy inférieurs.

Dans le présent contexte, aussi bien dans la description que dans les revendications, les termes repris ci-dessus comportent la signification suivante :

"alkyle" désigne les restes d'hydrocarbures aliphatiques saturés ayant jusqu'à 8 atomes de carbone tels que méthyle, éthyle, n-propyle, isopropyle, n-butyle, isobutyle, tertiobutyle, n-pentyle, néopentyle, n-hexyle, n-heptyle ou n-octyle,

"alkyle inférieur" désigne les restes d'hydrocarbures aliphatiques saturés ayant jusqu'à 4 atomes de carbone tels que méthyle, éthyle, n-propyle, isopropyle, n-butyle, isobutyle, tertiobutyle ou méthyl-l propyle,

"alkoxy inférieur" désigne un groupement hydroxy substitué par un groupement alkyle inférieur tel que défini précédemment,

"cycloalkyle désigne un groupement alicyclique ayant de 3 à 6 atomes de carbone, tels que cyclopropyle ou cyclohexyle.

Ainsi, en tenant compte des valeurs données ci-dessus :

| | |
|---|---|
| R ou R' | peut représenter notamment un radical méthyle, éthyle, n-propyle, isopropyle, n-butyle, isobutyle, tertiobutyle, méthyl-1 propyle, n-pentyle, néopentyle, phényle, mono-fluoro-, mono-chloro- ou mono-bromo-phényle, di-fluoro, di-chloro- ou di-bromo-phényle, mono-méthyl- ou di-méthyl-phényle, mono-méthoxy- ou di-méthoxy-phényle ou un radical méthyl-phényle substitué par un atome d'halogène, |
| A | peut représenter notamment une chaîne éthylène-1,2; propylène-1,3; méthyl-2 propylène-1,3; tétraméthylène-1,4 ou pentaméthylène-1,5. |
| $R_3$ | peut représenter notamment un radical méthyle, éthyle, n-propyle, isopropyle, n-butyle, isobutyle, tertiobutyle, méthyl-1 propyle, n-pentyle, n-hexyle, n-heptyle, n-octyle, phényle, benzyle, phénéthyle, méthoxy-phényle, diméthoxy-phénéthyle par exemple diméthoxy-3,4 phénéthyle, diméthylphénéthyle, di-méthoxybenzyle, pyridyléthyle ou un radical phénéthyle substitué dans la partie aromatique par des radicaux méthyle et méthoxy, |
| $R_4$ | peut représenter notamment un radical méthyle, éthyle, n-propyle, isopropyle, n-butyle, isobutyle, tertiobutyle, n-pentyle, néopentyle, n-hexyle, n-heptyle ou n-octyle, |
| $R_3$ et $R_4$ | pris ensemble peuvent représenter notamment un radical tétraméthylène-1,4; |

pentaméthylène-1,5; oxo-3 pentaméthylène-1,5; aza-3 pentaméthylène-1,5; méthylaza-3 pentaméthylène-1,5; phénylaza-3 pentaméthylène-1,5 ou -CH=CH-N=CH- de sorte que $R_3$ et $R_4$, pris avec l'atome d'azote auquel ils sont attachés, peuvent représenter notamment un radical pyrrolidinyle, pipéridinyle, morpholinyle, pipérazinyle, méthyl-4 pipérazinyle, phényl-4 pipérazinyle ou 1H-imidazolyle.

$R_5$, $R'_5$ et $R''_5$ peuvent représenter notamment le radical méthyle ou méthoxy ou un atome de chlore,

$R_7$, $R_5$, $R_9$, $R_{10}$ et $R_{11}$ peuvent représenter notamment un radical méthyle, éthyle, méthoxy, éthoxy, un atome de chlore, un groupement méthylsulfonamido, éthylsulfonamido, méthylamino, éthylamino, diméthylamino, diéthylamino, méthoxycarbonyle, éthoxycarbonyle, méthylcarbonyle ou éthylcarbonyle.

Une classe particulière de composés de formule (1) est constituée par ceux dans lesquels Cy représente un groupement indolizinyle.

Une autre classe de composés de formule (1) est représentée par ceux dans lesquels $R_1$ et $R_2$ représentent chacun l'hydrogène.

Une autre classe de composés de formule (1) est formée par ces composés dans lesquels $R_3$ représente un radical -Alk-Ar ou $R_3$ représente l'hydrogène et $R_4$ représente tertiobutyle ou $R_3$ ou $R_4$ représentent chacun n-propyle ou n-butyle. Une classe de composés de formule (1) est représentée par ces composés dans lesquels la chaine

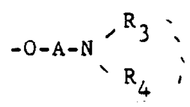

représente un groupement [N-méthyl N(diméthoxy-3,4 β-phénéthyl)amino]propoxy ou [N-méthyl N-(diméthoxy-3,5 β-phénéthyl)amino]propoxy .

D'autres composés de formule (1) particulièrement intéressants sont ceux dans lesquels R représente un groupement isopropyle ou cyclopropyle.

L'invention se rapporte également aux sels pharmaceutiquement acceptables des composés de formule (1) formés à partir d'un acide organique ou inorganique.

Comme exemples de sels organiques de ce genre, on peut citer les oxalate, maléate, fumarate, méthanesulfonate, benzoate, ascorbate, pamoate, succinate, hexamate, bisméthylènesalicylate, éthanedisulfonate, acétate, propionate, tartrate, salicylate, citrate, gluconate, lactate, malate, cinnamate, mandélate, citraconate, aspartate, palmitate, stéarate, itaconate, glycolate, p-aminobenzoate, glutamate, benzènesulfonate et théophylline acétate ainsi que les sels formés à partir d'un acide aminé tel que le sel de lysine ou d'histidine.

Comme exemples de sels inorganiques de ce genre, on peut citer les chlorhydrate, bromhydrate, sulfate, sulfamate, phosphate et nitrate.

Les composés de formule (1) peuvent exister dans certains cas sous la forme d'isomères optiques notamment en raison du carbone asymétrique présent lorsque A représente une chaîne hydroxy-2 propylène.

L'invention se rapporte à la fois à l'ensemble des isomères des composés de formule (1), isomères considérés sous forme dextrogyre ou lévogyre ou, sous forme de mélange, par exemple sous forme de mélange racémique.

On a trouvé que les composés de l'invention possèdent de remarquables propriétés pharmacologiques notamment des propriétés inhibitrices de la translocation calcique ainsi que des propriétés bradycardisantes, hypotensives et antiadrénergiques.

De ce point de vue, les composés préférés de l'invention sont ceux dans lesquels B représente un groupement $-SO_2-$.

Ces propriétés sont capables de rendre les composés en question très utiles dans le traitement de certains syndromes pathologiques du système cardiovasculaire en particulier dans le traitement de l'angine de poitrine, de l'hypertension, de l'arythmie, de l'insuffisance circulatoire cérébrale.

De même, les composés de l'invention pourront être utilisés seuls ou en association avec un agent antiinflammatoire pour réduire et/ou contrôler la pression intraocculaire excessive. A cet effet, les composés de l'invention pourront être utilisés pour le traitement d'affections pathologiques occulaires en particulier dans le traitement du glaucome.
Généralement, on administrera, à chaque oeil, de 5 ng à 0,5 mg de principe actif selon l'invention, la fréquence journalière d'administration dépendant de la gravité de l'affection à traiter.

Dans le domaine antitumoral, les composés de l'invention pourront être utiles comme potentialisateurs d'anticancéreux.

En conséquence, l'invention se rapporte également à des compositions pharmaceutiques ou vétérinaires contenant, comme principe actif, au moins un composé de formule (1) ou un sel pharmaceutiquement acceptable de ce composé, en association avec un véhicule pharmaceutique ou un excipient approprié.

Selon la voie d'administration choisie, la posologie journalière pour un être humain pesant 60kg se situera entre 2 et 500mg de principe actif.

Les composés de l'invention peuvent être obtenus comme suit :

I. - Lorsque $R_7$, $R_8$, $R_9$, ou $R_{10}$représentent l'hydrogène, un groupement alkyle inférieur, alkoxy inférieur, un atome d'halogène, un groupement benzyloxy, nitro, cyano, alkoxycarbonyle inférieur ou alkylcarbonyle inférieur et $R_{11}$a la même valeur que $R_7$ci-dessus à l'exception d'hydrogène.

A. - Les composés de formule (1) dans laquelle B représente un groupement -S- ou -$SO_2$-, et A représente un radical alkylène peuvent être préparés, selon l'invention, en condensant, en présence d'un accepteur d'acide et dans un solvant polaire tel que le diméthylsulfoxyde ou un alcool par exemple le butanol, une cétone telle que la méthyl-éthyl-cétone, ou un solvant non polaire tel qu'un hydrocarbure aromatique, par exemple le benzène, le toluène ou un xylène, un dérivé alkoxy-4 phényle de formule générale :

Cy-B'-Ph-O-AX (2)

dans laquelle B' représente un groupement -S- ou -$SO_2$-, Cy et Ph ont la même signification que précédemment, A représente un radical alkylène tel que défini dans la formule (1) et X représente un atome d'halogène, de préférence le brome, ou un groupement alkylsulfonyloxy ayant de 1 à 4 atomes de carbone tel que par exemple méthanesulfonyloxy ou arylsulfonyloxy ayant de 6 à 10 atomes de carbone tel que benzènesulfonyloxy ou p-toluènesulfonyloxy, avec une amine de formule générale :

H-Am (3)

dans laquelle Am a la même signification que précédemment pour former le dérivé souhaité de formule (1) sous forme de base libre.

Généralement, la condensation en question est effectuée à une température comprise entre la température ambiante et la température de reflux du milieu, l'accepteur d'acide pouvant être par exemple un carbonate ou hydroxyde de métal alcalin ou un excès d'amine de formule (3).

Les composés de formule (2) en question peuvent être obtenus :

a) Lorsque X est un halogène, par condensation d'un dérivé hydroxy-4 phényle de formule générale :

Cy-B'-Ph-OH (4)

dans laquelle Cy, B' et Ph ont la même signification que précédemment, avec un dihalogénoalcane de formule générale :

Hal-A-Hal (5)

dans laquelle A représente un radical alkylène tel que défini dans la formule (1) et Hal représente un atome d'halogène de préférence le brome et ce, au reflux, dans un solvant tel que la méthyl-éthylcétone ou le N,N-diméthylformamide et en présence d'un agent basique tel qu'un carbonate de métal alcalin par exemple le carbonate de potassium, un hydrure de métal alcalin tel que l'hydrure de sodium, un hydroxyde de métal alcalin par exemple l'hydroxyde de sodium ou de potassium ou un alcoolate de métal alcalin par exemple le méthylate ou l'éthylate de sodium,

b) lorsque X représente un groupement alkylsulfonyloxy ou arylsulfonyloxy par condensation d'un halogénure de formule générale :

Hal-W

dans laquelle W représente un radical alkylsulfonyle ayant de 1 à 4 atomes de carbone, par exemple méthanesulfonyle ou arylsulfonyle ayant de 6 à 10 atomes de carbone, par exemple benzènesulfonyle ou p-toluènesulfonyle, dans un solvant accepteur d'acide, par exemple la pyridine, avec un dérivé hydroxy-alkoxy de formule générale :

Cy-B'-Ph-O-A-OH (6)

dans laquelle Cy, B' et Ph ont la même signification que précédemment et A représente un radical alkylène tel que défini dans la formule (1).

Quant aux composés de formule (6), ceux-ci peuvent être préparés en condensant dans un solvant approprié tel que le N,N-diméthylformamide et en présence d'un agent basique tel qu'un carbonate de métal alcalin par exemple le carbonate de potassium, un hydroxyde de métal alcalin tel que l'hydroxyde de sodium ou de potassium, un hydrure de métal alcalin tel que l'hydrure de sodium ou un alcoolate de métal alcalin par exemple le méthylate ou l'éthylate de sodium, un dérivé hydroxy-phényle de formule (4) ci-dessus, avec un alcool halogéné de formule générale :

Hal-A-OH (7)

dans laquelle A représente un radical alkylène tel que défini dans la formule (1) et Hal a la même signification que précédemment.

Les amines de formule (3) sont des composés connus. Par exemple, des amines couvertes par la formule (3) ont été décrites dans les demandes de brevets européens Nos. 219.813, 227.986 et 235.111 ou peuvent être préparées selon les méthodes y décrites.

De même, certains composés de formule (4) sont des composés connus, par exemple deux dans lesquels Cy représente un groupement benzofuryle ou benzothiényle, ces groupements étant substitués ou non en position 4 par un atome d'halogène ou un groupement alkyle inférieur et B' représente $-SO_2-$.
Ces composés sont décrits dans le brevet U.S. No. 4.117.128.
Généralement, les autres composés de formule (4) peuvent être préparés en adaptant, au composé désiré, la méthode décrite dans le susdit brevet U.S. ou des méthodes classiques telles que celles décrites ci-dessous.

Dans la plupart des cas, les composés de formule (4) peuvent être obtenus en fixant, sur le carbocyle ou l'hétérocycle approprié, une chaîne benzènesulfonyle protégée en position 4 ou phénylthio en position 4 et ce, en utilisant une réaction de Friedel-Crafts et en déprotégeant l'oxygène en position 4 du groupement benzènesulfonyle ou phénylthio au moyen de procédés connus pour régénérer le groupement hydroxyle.
On a décrit par la suite des exemples de méthodes qui peuvent être généralement utilisées pour préparer de tels composés de formule (4).

a) Composés de formule (4) dans laquelle Cy représente un radical de formule (I) ou (I').

Pour obtenir de tels composés dans lesquels B représente un groupement $-SO_2-$, on peut, par exemple utiliser la méthode décrite dans Communications, avril 1984 p. 323 qui consiste à faire réagir un dérivé benzénique de formule générale :

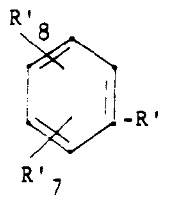

(8)

ou

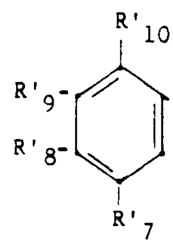

(8')

dans laquelle R' a la même signification que précédemment et $R'_7$, $R'_8$, $R'_9$ et $R'_{10}$ représentent l'hydrogène, un radical alkyle inférieur, alkoxy inférieur, un atome d'halogène, un groupement benzyloxy, nitro, cyano, alkoxycarbonyle inférieur ou alkylcarbonyle inférieur, avec un dérivé sulfonate de formule générale :

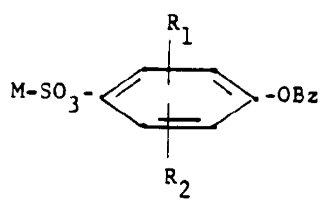

(9)

dans laquelle $R_1$ et $R_2$ ont la même signification que précédemment, M représente un atome de métal alcalin par exemple sodium et Bz représente un radical benzyle, en présence d'acide méthanesulfonique et d'anhydride phosphorique, pour obtenir les dérivés de formule générale :

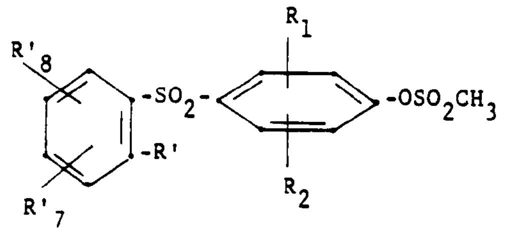

(10)

ou

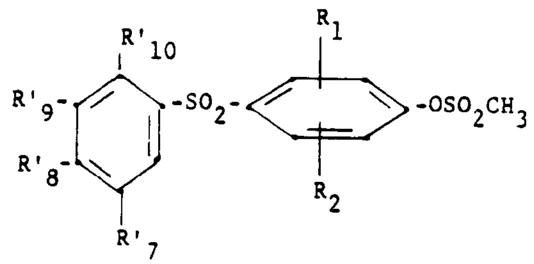

(10')

ou

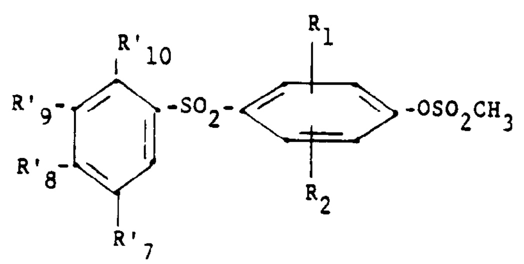

(10')

dans laquelle R', $R_1$, $R_2$, $R'_7$, $R'_8$, $R'_9$ et $R'_{10}$ ont la même signification que précédemment, dérivés que l'on hydrolyse en présence d'un agent basique tel qu'un hydroxyde de métal alcalin, par exemple l'hydroxyde de sodium pour former les composés désirés de formule (4).

b) Composés de formule (4) dans laquelle Cy représente un radical de formule (J).

D'une manière générale, de tels composés peuvent être obtenus en adaptant des méthodes connues par exemple celles décrites dans la demande de brevet européen No. 235.111, le brevet britannique No. 1.174.124, Berichte 60, p. 1607 (1927), Austr.J. Chem. 25, p. 1549 (1972), Khim. Geterotsikl. Soedin. 1972, No. 9 ou 1976, No. 4, 506-510, J.A.C.S. 81, 1456 (1959) ou C.A. 84, 179888 (1976).

Par exemple, on peut préparer des composés de formule (4) dans laquelle Cy représente un groupement R-2 indolizinyl-1, en faisant réagir un dérivé de pyridine de formule générale :

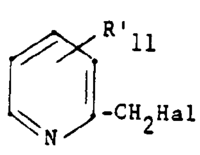

(11)

dans laquelle $R'_{11}$ représente un radical alkyle inférieur, alkoxy inférieur, un atome d'halogène, un groupement benzyloxy, nitro, cyano, alkoxycarbonyle inférieur ou alkylcarbonyle inférieur et Hal représente un atome d'halogène, par exemple chlore , avec un dérivé de formule générale :

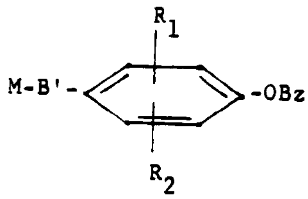

(12)

dans laquelle Bz, $R_1$, $R_2$, B' et M ont la même signification que précédemment, pour obtenir un composé de formule générale :

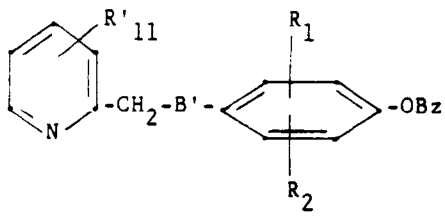

(13)

dans laquelle B', $R_1$, $R_2$, $R'_{11}$ et Bz ont la même signification que précédemment.

On traite alors le composé de formule (13) avec une cétone de formule générale :

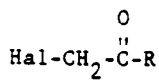

(14)

dans laquelle R et Hal ont la même signification que précédemment, ce qui fournit les dérivés d'indolizine de formule générale :

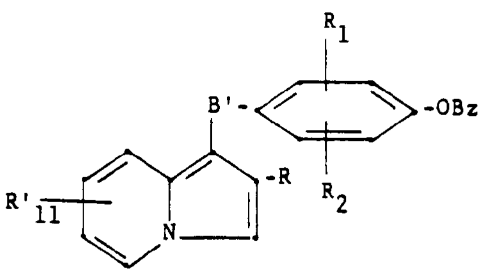

(15)

dans laquelle B', R, $R_1$, $R_2$, $R'_{11}$ et Bz ont la même signification que précédemment.

On fait alors réagir ces dérivés de formule (15) avec le formiate d'ammonium en présence de charbon palladié, ce qui fournit les composés désirés de formule (4), composés que l'on peut si nécessaire faire réagir avec par exemple un agent approprié capable d'engendrer un groupement électrophile pour former des composés de formule (4) dans laquelle $R_8$ se trouve en position 3 et est autre qu'hydrogène.

De même, on peut obtenir des composés de formule (4) dans laquelle Cy représente un groupement R-2 indolizinyl-2 en faisant réagir un dérivé d'indolizine de formule générale :

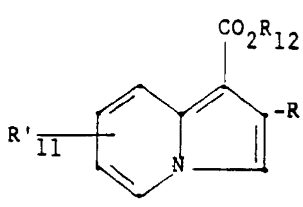

(16)

dans laquelle $R'_{11}$ a la même signification que précédemment et $R_{12}$ représente un groupement alkyle inférieur,

par exemple éthyle, avec un halogénure de formule générale :

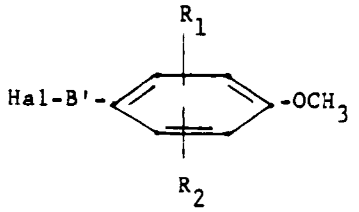

(17)

dans laquelle B', $R_1$, $R_2$ et Hal ont la même signification que précédemment et en présence d'un catalyseur de Friedel-Crafts tel que le chlorure d'aluminium, ce qui fournit le composé de formule générale :

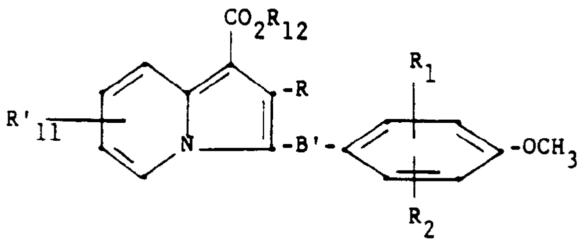

(18)

dans laquelle B', R, $R_1$, $R_2$, $R'_{11}$ et $R_{12}$ ont la même signification que précédemment.
On déméthyle ensuite le composé de formule (18) au moyen d'un mélange éthanethiol/chlorure d'aluminium ce qui fournit les dérivés méthoxy-4 phényle de formule générale :

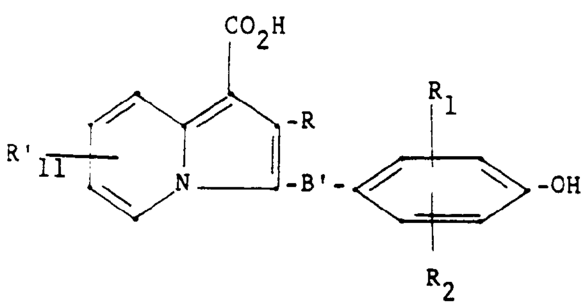

(19)

dans laquelle B', R, $R_1$, $R_2$ et $R_{11}$ ont la même signification que précédemment, composés qui, après chauffage à environ 200°C, fournissent les composés désirés de formule (4).

Les composés de formule (16) sont soit des composés connus ayant été publiés dans J. Chem. Soc. 1962, pp. 2627-2629, soit des composés qui peuvent être préparés par la méthode y décrite.

c) Composés de formule (4) dans laquelle Cy représente un radical de formule (K) ou (K').

Ces composés peuvent être préparés en adaptant la méthode décrite dans le brevet U.S. No. 4.117.128.

Toutefois, ils peuvent être obtenus également selon la méthode décrite ci-après :

1) Cy représente un radical de formule (K) dans lequel P représente -O-, -S- ou -NH :
en faisant réagir un composé de formule générale :

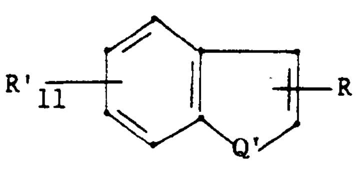

(20)

dans laquelle R et $R'_{11}$ ont la même signification que précédemment et Q' représente -NH, avec un composé de formule générale :

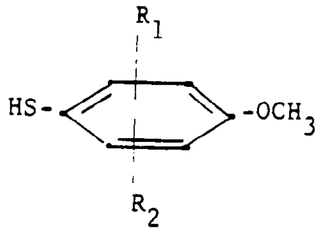

(21)

dans laquelle $R_1$ et $R_2$ ont la même signification que précédemment et ce, en présence d'iode pour obtenir les composés de formule générale :

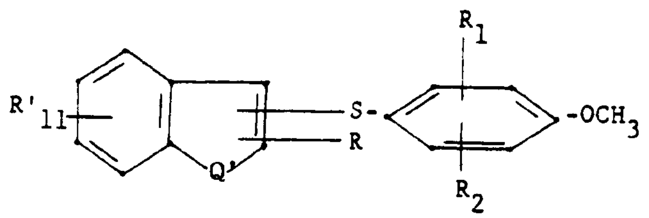

(22)

dans laquelle R, $R_1$, $R_2$, P' et $R'_{11}$ ont la même signification que précédemment, qui, après oxydation avec l'acide chloro-3 perbenzoïque, fournissent les composés de formule générale :

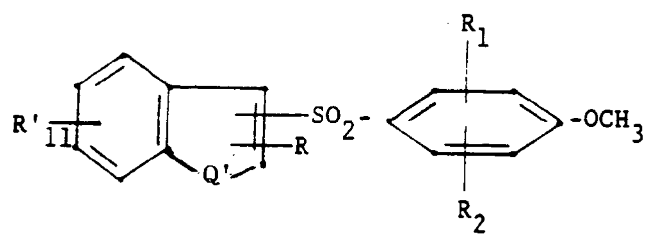

(23)

dans laquelle R, $R_1$, $R_2$, $R'_{11}$ et Q' ont la même signification que précédemment . On peut alors déméthyler les composés de formules (22) et (23) au moyen de mercapto-2 éthanol en présence d'hydrure de sodium, pour obtenir les composés souhaités de formule (4).

2) Cy représente un radical de formule (K) dans lequel Q représente un groupement

$$-\overset{|}{N}-R_{12}$$

dans lequel $R_{12}$ est autre qu'hydrogène:

en traitant un composé de formule (22) ou (23) dans lequel Q' représente -NH, éventuellement sous forme de dérivé métallique, avec un halogénure de formule $R_{12}$-Hal dans laquelle Hal a la même signification que précédemment, par exemple iode, et $R_{12}$ a la même signification que précédemment à l'exception d'hydrogène et en déméthylant le dérivé substitué en position 1 ainsi obtenu, avec le mercapto-2 éthanol en présence d'hydrure de sodium, pour obtenir le composé désiré de formule (4).

Les composés de formule (20) sont des produits connus pouvant être préparés par des méthodes connues. Par exemple, les dérivés indoliques de formule (20) peuvent être obtenus selon les méthodes décrites dans le brevet français No. 2.117.878.

3) Cy représente un radical de fromule (K') : an adaptant au composé désiré la méthode décrite au paragraphe 2) ci-dessus.

4) Cy représente un radical de formule (N) : en adaptant au composé désiré l'une des méthodes décrites aux paragraphes 1) et 2) ci-dessus.

d) Composés de formule (4) dans laquelle Cy représente un radical de formule (L)

On fait réagir, selon la méthode décrite dans la demande de brevet européen No. 121.197, une R-2 pyrazolo[1,5-a]pyridine de formule générale

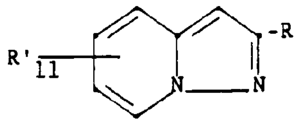

(24)

dans laquelle R et $R'_{11}$ ont la même signification que précédemment, avec un halogénure de formule générale :

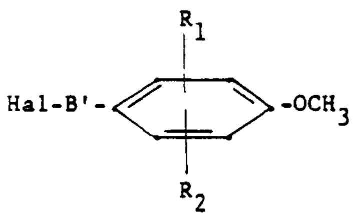

(25)

dans laquelle Hal, B', $R_1$ et $R_2$ ont la même signification que précédemment en présence d'un catalyseur de Friedel-Crafts tel que le chlorure d'aluminium, pour obtenir un dérivé méthoxy-4 phényle de formule générale :

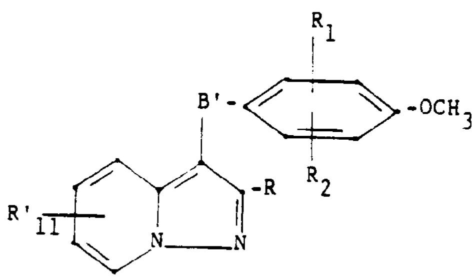

(26)

dans laquelle R, $R_1$, $R_2$, $R'_{11}$ et B' ont la même signification que précédemment.

On déméthyle alors le dérivé pyrazolopyridine de formule (26) au moyen par exemple de chlorhydrate de pyridine à 200-220°C, ce qui fournit le composé désiré de formule (4).

Certains composés de formule (24) sont des produits connus ayant été publiés dans le brevet U.S. n° 4.028.370. Ils peuvent être obtenus, par exemple, en adaptant au composé désiré la méthode décrite dans J. Org. Chem. 33 (10) pp. 3766-3770 ou Org. Synth. 43 p. 1 (1963).

e) Composés de formule (4) dans laquelle Cy représente un radical de formule (M)

On fait réagir une R-2 imidazo[1,2-a] pyridine de formule générale :

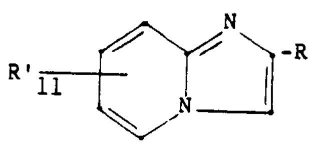

(26')

dans laquelle R et $R'_{11}$ ont la même signification que précédemment, avec un halogénure de formule (17) et en présence d'un catalyseur de Friedel-Crafts tel que le chlorure d'aluminium, pour obtenir un composé de formule générale :

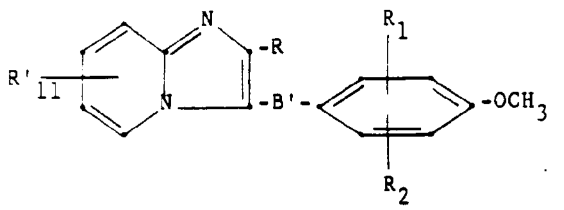

(27)

dans laquelle R, $R_1$, $R_2$, $R'_{11}$ et B' ont la même signification que précédemment.

On déméthyle alors le composé de formule (27) en utilisant un agent approprié par exemple l'acide bromhydrique ou un mélange éthanethiol/chlorure d'aluminium pour obtenir le composé désiré de formule (4). Des aryl-2 imidazo[1,2-a]pyridines sont déjà connues d'après J. Med. Chem. 8 p. 305 (1965). Les autres composés de formule (26') peuvent être obtenus selon la méthode décrite dans cette référence ou en utilisant des procédés classiques.

f) Composés de formule (4) dans laquelle Cy représente un radical de formule (Q) ou (Q')

De tels dérivés de pyridine peuvent être obtenus en déméthylant avec un agent approprié tel que l'acide bromhydrique aqueux, un dérivé méthoxy-4 phényle de formule générale :

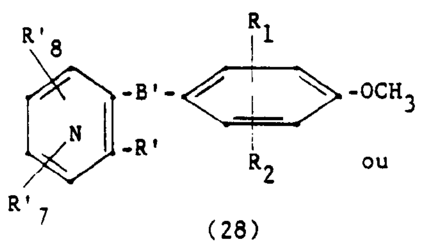

ou

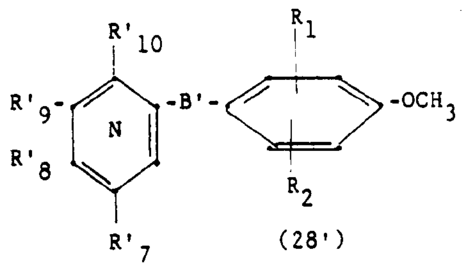

(28) (28')

dans laquelle R', $R_1$, $R_2$, $R'_7$, $R'_8$, $R'_9$, et $R'_{10}$ ont la même signification que précédemment, ces dérivés de méthoxy-4 phényle pouvant être préparés à partir d'un composé de formule générale :

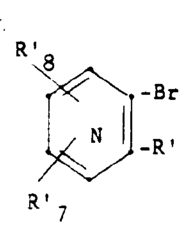

(29)

ou

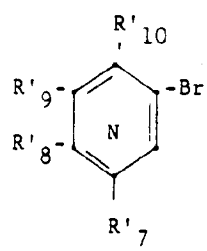

(29')

dans laquelle R, $R'_7$, $R'_8$, $R'_9$ et $R'_{10}$ ont la même signification que précédemment, que l'on traite avec un dérivé thiophénol de formule générale :

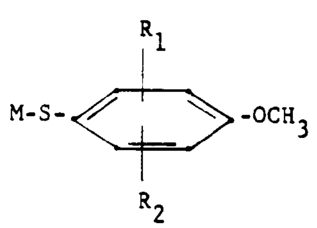

(30)

dans laquelle M, $R_1$ et $R_2$ ont la même signification que précédemment. Les composés de formules (29) et (29') sont des composés connus et peuvent être préparés selon des méthodes connues, par exemple en adaptant la méthode décrite dans Nippon Kagaku Zasshi 86 (10) pp. 1060-1067 (1967) [C.A. 16936h (1966)].

Suivant une méthode alternative, on peut également obtenir les composés de formule (1) dans laquelle B représente un groupement -S- ou $-SO_2-$ et A représente un radical alkylène de préférence ceux dans lesquels A représente un radical propylène, en faisant réagir, en présence d'un agent basique tel qu'un carbonate de

métal alcalin, par exemple le carbonate de potassium, un hydroxyde de métal alcalin tel que l'hydroxyde de sodium ou de potassium, un hydrure de métal alcalin tel que l'hydrure de sodium ou un alcoolate de métal alcalin par exemple le méthylate ou l'éthylate de sodium, un dérivé hydroxy-4 phényle de formule (4) ci-dessus, avec un composé de formule générale :

X-A-Am (31)

dans laquelle X et Am ont la même signification que précédemment et représentent de préférence le chlore ou un radical benzènesulfonyloxy ou p-toluènesulfonyloxy et a représente un radical alkylène, la réaction ayant lieu au reflux et dans un solvant polaire tel que la méthyl-étylcétone ou le diméthylsulfoxyde pour former le dérivé désiré de formule (1) sous forme de base libre.

Les composés de formule (31) sont des produits connus ou pouvant être préparés par des méthodes connues.

Cette méthode, lorsqu'elle est appliquée à un dérivé métallique de formule :

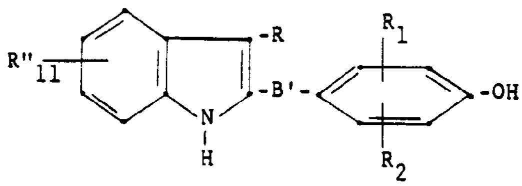

(32)

dans laquelle B', R, $R_1$ et $R_2$ ont la même signification que précédemment et $R''_{11}$ représente l'hydrogène ou un groupement de formule $R'_{11}$ ci-dessus, permet d'obtenir directement des dérivés indoliques de formule (1) dans laquelle Cy représente un groupement (K) dans lequel P représente un groupement

$-N-R_{12}$

dans lequel $R_{12}$ représente un groupement -A-Am ou de formule (1) dans laquelle Cy représente un groupement (K') dans lequel $R'_{12}$ représente un groupement -A-Am.

B. - Les composés de formule (1) dans laquelle B représente un groupement -SO- peuvent être préparés en traitant, avec un agent oxydant, un sulfure de formule (1) dans laquelle B représente un groupement -S-, ce composé de formule (1) étant sous forme de base libre ou d'un sel de façon à obtenir le dérivé désiré de formule (1) sous forme de base libre ou de sel.

Lorsque le composé désiré est obtenu sous forme de sel, on peut régénérer la base libre par traitement avec un agent basique tel qu'un carbonate de métal alcalin par exemple le carbonate de potassium ou un bicarbonate de métal alcalin par exemple le bicarbonate de sodium.

Généralement, la réaction a lieu dans l'eau ou dans un solvant organique tel que le chlorure de méthylène et en présence d'un agent oxydant approprié tel que par exemple le périodate de sodium, le permanganate de potassium ou l'acide chloro-3 perbenzoïque.

Selon l'agent oxydant utilisé, on peut obtenir des mélanges de sulfoxydes ou de sulfones. Ces mélanges peuvent être séparés par des procédés conventionnels par exemple par chromatographie.

C. - Les composés de formule (1) dans laquelle B représente un groupement -S- ou $-SO_2-$ et A représente une chaîne hydroxy-2 propylène peuvent être obtenus en faisant réagir au reflux un dérivé de formule (4) avec une épihalohydrine telle que l'épichlorhydrine ou l'épibromhydrine sous forme dextrogyre, lévogyre ou sous forme de mélange de ces isomères par exemple sous forme racémique et en présence d'un agent basique tel qu'un carbonate de métal alcalin par exemple le carbonate de potassium, un hydroxyde de métal alcalin par exemple l'hydroxyde de sodium ou de potassium, un hydrure de métal alcalin tel que l'hydrure de sodium ou un alcoolate de métal alcalin par exemple le méthylate ou l'éthylate de sodium et dans un solvant polaire tel que la méthyl-éthyl-cétone, pour donner les dérivés oxyranylméthoxy de formule générale :

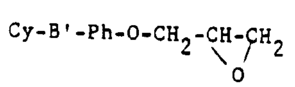

(33)

dans laquelle Cy, B' et Ph ont la même signification que précédemment.

Les dérivés oxyranylméthoxy de formule (33) sont alors traités au reflux avec une amine de formule (3) et ce, dans un solvant polaire tel que la méthyl-éthyl-cétone ou dans un excès d'amine de formule (3) pour donner le dérivé désiré aminoalkoxyphényle de formule (1) sous forme de base libre dans laquelle A représente

une chaîne hydroxy-2 propylène, dérivé que l'on peut faire réagir, si on le désire, avec un halogénure d'alkyle inférieur en présence d'une base forte pour former les composés de formule (1) sous forme de base libre dans laquelle A représente une chaîne hydroxy-2 propylène dans laquelle l'hydroxy est substitué par un radical alkyle inférieur.

Dans certains cas, des produits secondaires peuvent se former parallèlement aux composés de formule (33) ci-dessus, par exemple des dérivés (halogéno-3 hydroxy-2 propoxy)-4 benzènesulfonyles. Par réaction avec l'amine de formule (3) , ces dérivés donneront naissance néanmoins aux composés désirés de formule (1) dans laquelle A représente une chaîne hydroxy-2 propylène.

II. - Lorsque $R_7$, $R_8$, $R_9$, $R_{10}$ou $R_{11}$représente un groupement hydroxy.

On hydrogène dans un solvant approprié, par exemple un alcool ou une cétone, et en présence d'un catalyseur approprié tel que le nickel de Raney ou le charbon palladié, un composé de formule (1) dans laquelle $R_7$, $R_8$, $R_9$, $R_{10}$ ou $R_{11}$ représente un groupement benzyloxy, ce qui fournit le composé désiré de formule (1) sous forme de base libre.

III . - Lorsque $R_7$, $R_8$, $R_9$, $R_{10}$ou $R_{11}$représente un groupement amino, alkylamino inférieur ou dialkylamino inférieur.

On hydrogène dans un solvant approprié, par exemple un alcool ou une cétone, et en présence d'un catalyseur approprié, tel que l'oxyde de platine, un composé de formule (1) dans laquelle $R_7$, $R_8$, $R_9$, $R_{10}$ ou $R_{11}$ représente un groupement nitro, ce qui fournit le composé désiré de formule (1) sous forme de base libre, dans laquelle $R_7$, $R_8$, $R_9$, $R_{10}$ ou $R_{11}$ représente un groupement amino, composé que l'on peut faire réagir, si nécessaire, avec une quantité appropriée d'un halogénure d'alkyle inférieur par exemple le bromure, en présence d'un agent alcalin, pour obtenir les composés de formule (1), sous forme de base libre, dans laquelle $R_7$, $R_8$, $R_9$, $R_{10}$ ou $R_{11}$ représente un groupement alkylamino inférieur ou dialkylamino inférieur.

IV. - Lorsque $R_7$, $R_8$, $R_9$, $R_{10}$ou $R_{11}$représente un groupement sulfamido, alkylsulfamido inférieur ou phénylsulfamido.

On fait réagir, dans un solvant approprié par exemple le dichlorométhane, et en présence d'un accepteur d'acide par exemple la triéthylamine, un composé de formule (1) dans laquelle $R_7$, $R_8$, $R_9$, $R_{10}$ ou $R_{11}$ représente un groupement amino, avec un halogénure de sulfonyle, d'alkylsulfonyle inférieur ou de phénylsulfonyle, ce qui fournit le composé désiré de formule (1) sous forme de base libre.

Les composés de foemule (1) ainsi obtenus sous forme de base libre peuvent ensuite être transformés en sels pharmaceutiquement acceptables par réaction avec un acide organique ou inorganique approprié par exemple l'acide oxalique, maléique, fumarique, méthanesulfonique, benzoïque, ascorbique, pamoïque, hexamique, bisméthylènesalicylique, éthanedisulfonique, acétique, propionique, tartrique, salicylique, citrique, gluconique, lactique, malique, cinnamique, mandélique, citraconique, aspartique, palmitique, stéarique, itaconique, glycolique, p-aminobenzoïque, glutamique, benzènesulfonique, théophylinne acétique ou avec la lysine ou l'histidine.

EP-0 235 111 décrit des dérivés d'indolizine ne comportant pas de substituant sur le cycle à 6 chaînons du noyau indolizin-1-yle

On a décrit dans le brevet FR-A-2.594.438 des dérivés (alkylamino- ou aralkylamino-alkoxy-4 benzènesulfonyl)-1 indolizine doués de propriétés actives sur le système cardiovasculaire notamment des propriétés inhibitrices de la translocation calcique et antiadrénergiques de type $\alpha$ et $\beta$. Ce brevet couvre notamment de tels dérivés d'indolizine substitués en l'une des positions 5,6,7 ou 8 par un atome d'halogène, un groupement hydroxy, alkyle inférieur, alkoxy inférieur, trifluorométhyle, nitro, cyano, carboxy, carbamoyle, (alkoxy inférieur)-carbonyle ou benzyloxy.

Cependant aucun composé de ce type n'est cité ni décrit spécifiquement tant au point de vue chimique que pharmacologique.

EP-0 302 792 opposable au titre de l'article 54(3) CBE décrit des dérivés alkylaminoalkoxyphényles comportant des groupes Cy différemment substitués par rapport aux groupes Cy de la formule I de la présente invention.

J. Med. Chem. 1978, vol. 21, N° 2, p. 182-188 décrit des agents actifs sur le catabolisme des lipides comportant un groupe phénylthio, phénylsulfinyle ou phénylsulfonyle substitué par un atome de chlore.

On a maintenant trouvé, de manière surprenante, que les dérivés indolizinyl-1 de formule I ci-dessus, en l'occurrence des dérivés (alkylamino- ou aralkylamino-alkoxy-4 benzènesulfonyl)-1 indolizine substitués en

position 5, 6, 7 ou 8, présentent de remarquables propriétés pharmacologiques se traduisant notamment par des propriétés inhibitrices calciques et antiadrénergiques largement supérieures à celles des dérivés analogues non substitués en position 5, 6, 7 ou 8.

De même, on a cité, dans le brevet U.S. No. 4.117.128, des dérivés alkylaminoalkoxybenzènesulfonyl-benzofuranne ou benzothiophène actifs sur le système cardiovasculaire.

Ce brevet se rapporte à des dérivés de ce type comportant ou non en position 5 un atome d'halogène ou un groupement alkyle. Bien que de tels dérivés non substitués en position 5 puissent être considérés comme réellement connus, rien ne permet de conclure, à priori, que les composés analogues substitués en position 5 ont réellement été préparés et que leur activité pharmacologique a effectivement été recherchée.

Comme il a été rapporté en détail par R. CHARLIER dans "Bruxelles Médical", No. 9 septembre 1969, pages 543-560, il est admis qu'une médication antiangineuse doit être capable notamment d'antagoniser les réactions cardiovasculaires de type adrénergique. A cet effet, on a proposé des agents aptes à bloquer les récepteurs $\alpha$.

Cependant l'application clinique de tels composés au traitement de l'angor resta sans succès très probablement par le fait que les antagonistes des récepteurs $\alpha$ n'induisent qu'une neutralisation très partielle du système adrénergique, l'activité des récepteurs $\beta$ étant respectée.

Or, les manifestations hémodynamiques les plus indésirables qui surviennent chez l'angineux de poitrine au cours de ses accès douloureux sont avant tout cardiaques et relèvent de ce fait des récepteurs $\beta$.

Parallèlement, on a proposé des médications antagonistes des récepteurs adrénergiques $\beta$. Ces composés, d'un intérêt clinique réel, diminuent les crises d'angor en réduisant le travail cardiaque par ralentissement de la fréquence du rythme. Cependant, il n'y a pas de chute des résistances artérielles périphériques qui s'élèvent au contraire par libération du tonus $\alpha$.

Ces médications modifient toutefois certains paramètres hémodynamiques dans un sens qui, sur le plan fondamental, constitue une contrepartie défavorable à l'angineux de poitrine en particulier et au cardiaque en général.

Si l'on considère l'aspect antiadrénergique des $\beta$-bloquants, il devient évident que seules la tachycardie et l'augmentation de la puissance et de la vélocité de la contraction cardiaque sont susceptibles d'être neutralisées, l'hypertension artérielle relevant d'une stimulation des récepteurs $\alpha$ sur lesquels les antagonistes $\beta$ n'ont pas d'action.

Or, si les perturbations cardiovasculaires entraînées par la stimulation des récepteurs $\beta$ sont les plus préjudiciables à l'angineux, il m'en reste pas moins que l'hypertension artérielle joue également un rôle qui n'est pas négligeable.

Au surplus, le blocage des récepteurs $\beta$ comporte un risque privant l'insuffisant cardiaque d'un mécanisme compensateur qu'il met normalement en jeu pour limiter son insuffisance circulatoire.

Ce mécanisme réflexe dont la composante principale emprunte la voie du système $\beta$-adrénergique aboutit notamment à une augmentation de la puissance et de la vélocité de la contraction cardiaque. Par conséquent, si ce système est bloqué, l'insuffisant cardiaque voit s'aggraver sa défaillance fonctionnelle., Il est donc logique de considérer que l'emploi d'un $\beta$-bloquant donc l'action est <u>pure</u> et <u>complète</u> comportera toujours un risque cardiaque.

Il paraît donc souhaitable de ne pas rechercher des propriétés antagonistes $\alpha$ ou $\beta$ complètes, étant donné les effets secondaires qu'elles peuvent entraîner en clinique. Il semble plus logique de viser à amortir plutôt qu'à supprimer les perturbations cardiovasculaires qui caractérisent l'hyperstimulation du système adrénergique dans sa totalité.

Les composés de l'invention répondent à cet objectif puisqu'ils présentent des propriétés antiadrénergiques de type $\alpha$ et $\beta$ incomplètes. Ils peuvent donc être considérés, non comme des $\beta$-bloquants mais comme des adrénofreinateurs c'est-à-dire des antagonistes partiels des réactions adrénergiques $\alpha$ et $\beta$ potentiellement dépourvues des désavantages énumérés ci-dessus pour les $\beta$-bloquants.

En outre, la composante inhibitrice calcique mise en évidence chez les composés de l'invention complétera d'une manière remarquable leur spectre pharmacologique cardiovasculaire.

On sait, en effet, que la translocation des ions calcium est une des composantes essentielles du potentiel d'action au niveau des cellules cardiaques et que, à ce titre, elle joue un rôle primordial dans la conduction électrique ainsi que dans ses troubles éventuels (arythmie). En outre, il est connu que les ions calcium sont impliqués dans le couplage excitation-contraction lequel contrôle, au niveau de la musculature lisse le degré de vasoconstriction et, par la même occasion, joue un rôle critique dans la crise d'angine de poitrine.

Les composés antagonistes du calcium agissent au niveau de la membrane cellulaire en empêchant sélectivement le calcium d'intervenir dans le processus de contraction au sein de la cellule artérielle.

Or, il apparaît de plus en plus évident, à l'heure actuelle, que les résultats cliniques apportés par l'association d'inhibiteurs calciques et d'inhibiteurs $\beta$-adrénergiques sont meilleurs que lorsque chaque inhibiteur est

utilisé isolément (J.A.M.A. 1982, 247, pages 1911-1917).

Il semble, en outre, qu'il n'existe, à l'heure actuelle, aucun β-bloquant exerçant en plus une action inhibitrice appréciable au niveau de la translocation calcique.

De ce point de vue, les composés de l'invention présentant à la fois une composante anticalcique et une composante antiadrénergique α et β seront d'un intérêt primordial car susceptibles d'applications thérapeutiques plus étendues qu'un β-bloquant isolé ou qu'un inhibiteur calcique isolé. A titre d'exemple on citera :

- l'isopropyl-2 méthyl-8 {[N-méthyl N-(diméthoxy-3,5 β-phénéthyl)amino]-3 propoxy-4 benzènesulfonyl}-1 indolizine (Ex. 76).
- l'isopropyl-2 méthyl-8 {[N-méthyl N-(diméthoxy-3,4 β-phénéthyl)amino]-3 propoxy-4 benzènesulfonyl}-1 indolizine (Ex. 15).
- le {[(di-n-butylamino)-3 propoxy]-4 benzènesulfonyl}-1 isopropyl]-2 méthyl-8 indolizine (Ex. 75)
- l'isopropyl-2 méthyl-8{[(diméthoxy-6,7 tétrahydro-1,2,3,4 isoquinoléinyl-2)-3 propoxy]-4 benzènesulfonyl}-1 indolizine (Ex. 2).
- l'isopropyl-2 méthyl-5 {[N-méthyl N-(diméthoxy-3,4 β-phénéthyl)amino]-3 propoxy-4 benzènesulfonyl}-1 indolizine (Ex. 79).
- le{[N-méthyl N-(diméthoxy-3,4 β-phénéthyl)amino]-3 propoxy-4 benzènesulfonyl}-2 chloro-5 isopropyl-3méthyl-1 indole (Ex. 59).

Cependant l'intérêt majeur de ces composés résidera dans le fait qu'ils pourront, grâce à leur composante anticalcique, être utilisés dans le traitement de l'angine au repos, syndrome provoqué par l'apparition d'un spasme au niveau des coronaires lequel est combattu actuellement par des composés tels que le diltiazem, le vérapamil ou la nifédipine.

Au surplus, les composés de l'invention se sont également montrés capables de provoquer une augmentation du débit coronaire de manière importante.

Les résultats de tests pharmacologiques effectués en vue de déterminer les propriétés cardiovasculaires des composés de l'invention sont répertoriés ci-dessous :

I. Propriétés inhibitrices calciques

Les propriétés inhibitrices de la translocation calcique au niveau membranaire présentées par les composés de l'invention ont été mises en évidence par mesure de leur action antagoniste vis-à-vis de la réponse contractile à la dépolarisation provoquée par le potassium sur l'aorte isolée de rat. Il est bien établi que la dépolarisation de la membrane d'un muscle lisse par le potassium rend cette dernière perméable au calcium extracellulaire et provoque la contraction musculaire.

Dès lors, la mesure de l'inhibition de la réponse contractile à la dépolarisation par le potassium ou la mesure d'un relachement de la contraction tonique à la dépolarisation potassique peut constituer une évaluation de la puissance d'un composé en tant qu'inhibiteur de la perméabilité membranaire aux ions $Ca^{++}$.

La technique utilisée est la suivante :

Sur des rats mâles Wistar pesant environ 300 g, on prélève l'aorte et on la coupe en bandelettes d'environ 40mm de longueur et 3 mm de largeur. On place ces fragments dans une cuve à organe isolé de 25ml contenant une solution de Krebs-bicarbonate modifiée (NaCl 112 mM; KCl 5mM; $NaHCO_3$ 25 mM; $KH_2PO_4$ 1 mM; $MgSO_4$ 1,2mM; $CaCl_2$ 2,5 mM; glucose 11,5 mM; eau distillée jusqu'à 1000 ml, parcourue par un courant de carbogène et maintenue à 37°C. On relie la préparation à un microcapteur de force et on enregistre la réponse contractile après amplifition sur un enregistreur.

On applique une tension de 2g à la préparation. On maintient celle-ci durant 60 minutes dans la solution de Krebs-bicarbonate modifiée puis on provoque les contractions en remplaçant la solution de Krebs-bicarbonate par une solution de Krebs-potassique (NaCl 17mM; KCl 100mM; $NaHCO_3$ 25mM; $KH_2PO_4$ 1mM; $MgSO_4$ 1,2mM; $CaCl_2$ 2,5mM; glucose 1,5mM; eau distillée jusqu'à 1000ml). Lorsque la réponse contractile de la préparation est devenue reproductible, on introduit, dans le bain, une quantité donnée d'un composé de l'invention. Soixante minutes plus tard, un nouveau spasme est provoqué par la dépolarisation potassique.

Les résultats obtenus sur l'aorte éprouvée sont alors exprimés en % de l'effet contracturant maximal avant l'incubation avec la substance à tester.

A titre d'exemples, les résultats suivants ont été obtenus, les composés de formule (1) étant sous la forme de base, de chlorhydrate, d'oxalate ou de fumarate.

A. Dérivés de benzofuranne

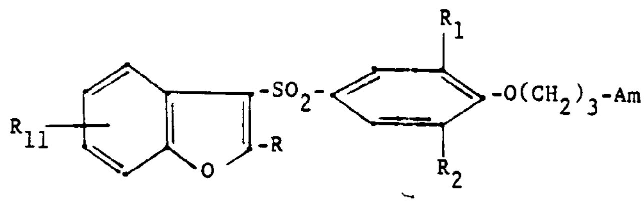

| Composés | R | $R_1$ et $R_2$ | $R_{11}$ | Am | % effet contracturant max. $10^{-6}M$ | % effet contracturant max. $10^{-7}M$ |
|---|---|---|---|---|---|---|
| Ex. 1 | $n-C_4H_9$ | H | $-NO_2-5$ | 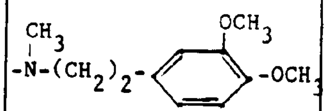 | 45,4 | 70,1 |
| Ex. 3 | $n-C_4H_9$ | H | $-NH_2-5$ | 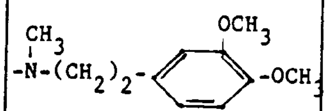 | 24,8 | 71,1 |
| Ex. 4 | $n-C_4H_9$ | H | $-NHSO_2CH_3-5$ | 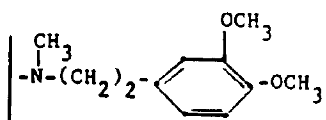 | 49,9 | 92,7 |
| Ex. 8 | $n-C_4H_9$ | H | Br-5 | 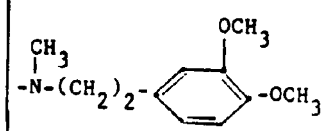 | 61,5 | 79,4 |
| Ex. 7 | $n-C_4H_9$ | Br | Cl-5 | 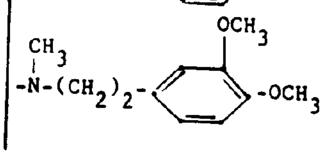 | 80,3 | 76,8 |

B. Dérivés d'indole

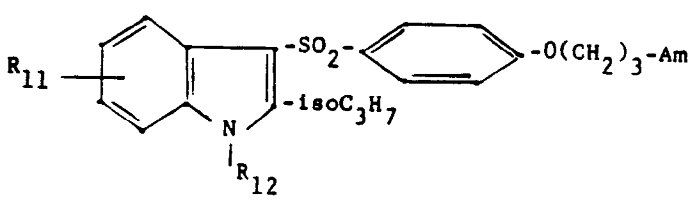

| Composés | $R_{11}$ | $R_{12}$ | Am | % effet contracturant maximal | | | |
|---|---|---|---|---|---|---|---|
| | | | | $10^{-7}M$ | $10^{-8}M$ | $10^{-9}M$ | $10^{-1}$ |
| Ex. 11 | H | $-(CH_2)_2-N(CH_3)_2$ | 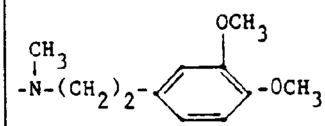 | 66,7 | 85,9 | - | - |
| Ex. 12 | H | $-(CH_2)_2-N(C_2H_5)_2$ | 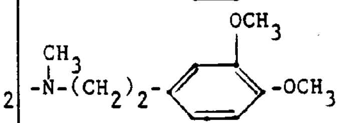 | 74,9 | 84,5 | 90,4 | - |
| Ex. 13 | Cl-5 | $CH_3$ | 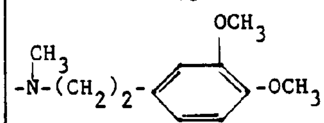 | 35,5 | 79,2 | - | - |
| Ex. 44 | Cl-5 | $CH_3$ | $-N(n-C_4H_9)_2$ | 69,6 | 85,7 | - | - |
| Ex. 45 | $CH_3$-6 | $CH_3$ | 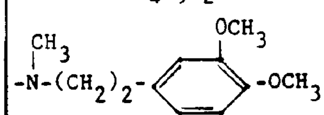 | 15,7 | 75,2 | 83,2 | - |
| Ex. 46 | $CH_3$-6 | $CH_3$ | $-N(n-C_4H_9)_2$ | 41,7 | 67,2 | 79,5 | - |
| Ex. 47 | Cl-5 | $CH_3$ | 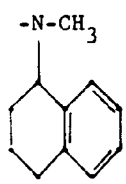 | 75,3 | 88,3 | 91,4 | - |
| Ex. 65 | $CH_3$-4 | $CH_3$ | 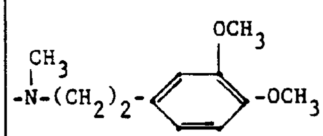 | 14,3 | 29,9 | 76,1 | 83,5 |
| Ex. 66 | $CH_3$-4 | $CH_3$ | $-N(n-C_4H_9)_2$ | 7,1 | 37,1 | 83,1 | - |

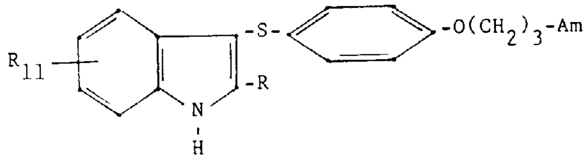

| Composés | $R_{11}$ | R | Am | % effet contracturant maximal | | | |
|---|---|---|---|---|---|---|---|
| | | | | $10^{-7}M$ | $10^{-8}M$ | $10^{-9}M$ | $10^{-10}M$ |
| Ex. 9 | $OCH_3$-5 | $CH_3$ | 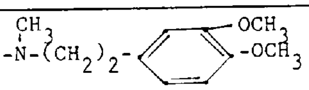 | 70,4 | 84,4 | 88 | - |

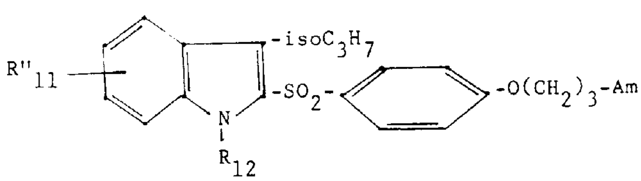

| Composés | $R''_{11}$ | $R_{12}$ | Am | % effet contracturant maximal | | | |
|---|---|---|---|---|---|---|---|
| | | | | $10^{-7}M$ | $10^{-8}M$ | $10^{-9}M$ | $10^{-10}M$ |
| Ex. 10 | H | 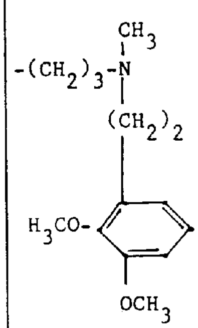 | 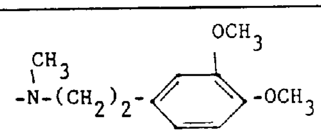 | 78,9 | 90,2 | - | - |
| Ex. 55 | $CH_3$ | $CH_3$ | "" "" | 45,8 | 56,4 | 79 | 85,7 |

| | | | | | | | |
|---|---|---|---|---|---|---|---|
| Ex. 59 | Cl-5 | $CH_3$ | 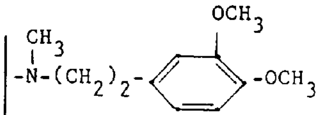 | 41,4 | 55,4 | 68,4 | 81,5 |
| Ex. 61 | $OCH_3$-5 | $CH_3$ | " " | 15,7 | 31,2 | 71,4 | 89,5 |
| Ex. 62 | $OCH_3$-5 | H | " " | 37,1 | 48,7 | 93,7 | 90,3 |
| Ex. 63 | $OCH_3$-5 | 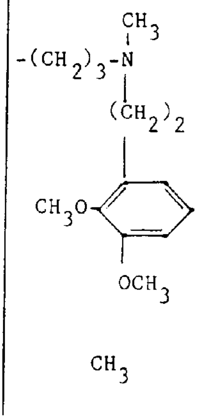 | " " | 69,7 | 82,8 | 90,8 | - |
| Ex. 64 | $OCH_3$-5 | $CH_3$ | $-N(n-C_4H_9)_2$ | 69,3 | 72,6 | 93,9 | - |

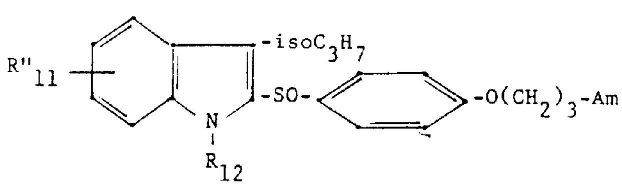

| Composés | R"$_{11}$ | $R_{12}$ | Am | % effet contracturant maximal | | | |
|---|---|---|---|---|---|---|---|
| | | | | $10^{-7}$M | $10^{-8}$M | $10^{-9}$M | $10^{-10}$M |
| Ex. 56 | Cl-5 | $CH_3$ | 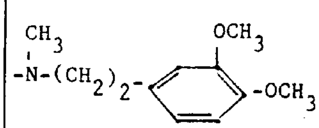 | 19,8 | 61,3 | 78,6 | - |
| Ex. 57 | $CH_3$-7 | $CH_3$ | " " | 56,9 | 76,4 | 89,9 | - |

C. Dérivés d'indolizine

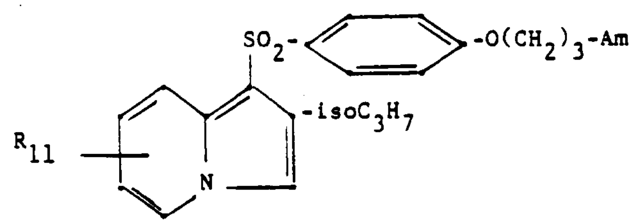

| Composés | $R_{11}$ | Am | % effet contracturant maximal | | |
|---|---|---|---|---|---|
| | | | $10^{-8}M$ | $10^{-9}M$ | $10^{-10}M$ |
| Ex. 2 | $CH_3$-8 | 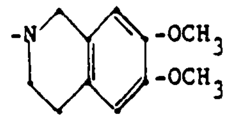 | 11,9 | 72,6 | - |
| Ex. 15 | $CH_3$-8 | 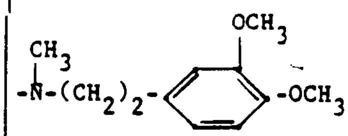 | 11,1 | 41,0 | 85,2 |
| Ex. 75 | $CH_3$-8 | $-N(n-C_4H_9)_2$ | 9,7 | 51,4 | 83,8 |
| Ex. 76 | $CH_3$-8 | 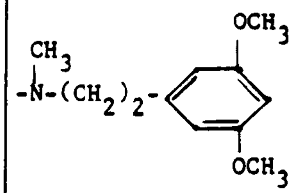 | 12,7 | 41,8 | 64,1 |
| Ex. 79 | $CH_3$-5 | 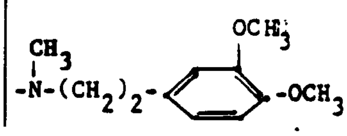 | 29,8 | 77,9 | 87,3 |

A titre de comparaison, on a obtenu les résultats suivants avec des composés connus (brevet FR-A-2.594.438)

| Composés | $R_{11}$ | Am | % effet contracturant maximal | | |
|---|---|---|---|---|---|
| | | | $10^{-8}M$ | $10^{-9}M$ | $10^{-10}M$ |
| B | H | $-N(n-C_4H_9)_2$ | 61 | - | - |
| C | H | 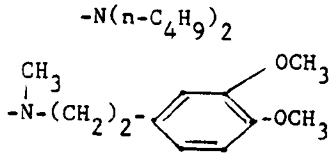 | 40,1 | 81,7 | - |

D. Dérivés de pyrazolo[1,5-a]pyridine

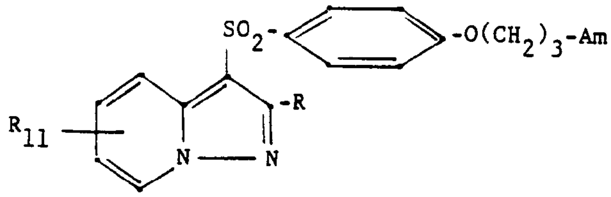

| Composé | R | $R_{11}$ | Am | % effet contractu-rant maximal | | |
|---|---|---|---|---|---|---|
| | | | | $10^{-8}M$ | $10^{-9}M$ | $10^{-10}M$ |
| Ex. 48 | $CH_3$ | $CH_3$-7 | $-N(n-C_4H_9)_2$ | 77,1 | 85,7 | - |
| Ex. 49 | $CH_3$ | $CH_3$-4 | $-N(n-C_4H_9)_2$ | 62,9 | 75,7 | - |
| Ex. 50 | iso$C_3H_7$ | $CH_3$-7 | 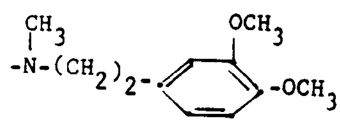 | 29,1 | 65,6 | 86,3 |
| Ex. 51 | $CH_3$ | $CH_3$-4 | 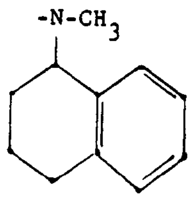 | 67,1 | 85 | - |
| Ex. 78 | $C_2H_5$ | $CH_3$-4 | 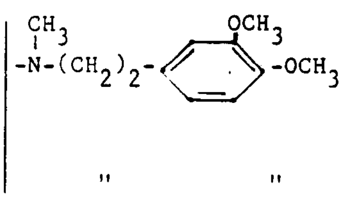 | 17,5 | 72,9 | 78,6 |
| Ex. 81 | $C_2H_5$ | $CH_3$-6 | " " | 78,1 | 85,3 | 91,4 |

E. Dérivés de benzène

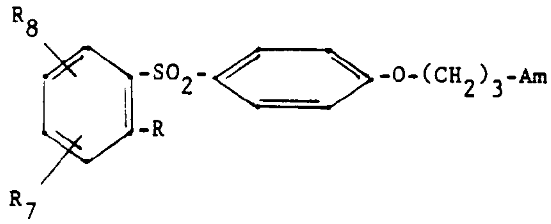

| Composé | R | $R_7$ | $R_8$ | Am | % effet contracturant maximal | | |
|---|---|---|---|---|---|---|---|
| | | | | | $10^{-7}M$ | $10^{-8}M$ | $10^{-9}M$ |
| Ex.6 | $isoC_3H_7$ | $OCH_3$-3 | $OCH_3$-5 | $CH_3$ $-N-(CH_2)_2-$ 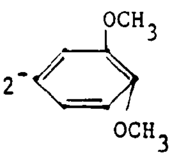 | 13,1 | 63,4 | 85,7 |

II. Propriétés antiadrénergiques

Le but de ce test est de déterminer la capacité des composés de l'invention de réduire l'augmentation de la pression sanguine induite par l'épinéphrine (effet anti-α) et l'accélération de la fréquence cardiaque induite par l'isoprénaline (effet anti-β) chez le chien préalablement anesthésié au pentobarbital et atropiné.

On détermine d'abord pour chaque chien la dose d'épinéphrine (entre 3 et 10 μg/kg) qui provoque une augmentation reproductible de la pression artérielle d'environ $133.10^2$ Pa et la dose d'isoprénaline (1 à 2 μg/kg) qui provoque une augmentation reproductible de la fréquence cardiaque d'environ 70 battements/minute. On injecte alternativement toutes les dix minutes la dose d'épinéphrine et d'isoprénaline ainsi déterminée et après obtention de deux réponses de référence successives, on administre une quantité du composé à étudier par voie intraveineuse.

- Effet anti-α

  On enregistre le pourcentage de réduction de l'hypertension provoquée par le composé à étudier comparativement à l'hypertension de référence obtenue précédemment (environ 100 mm Hg).

- Effet anti-β

  On enregistre le pourcentage de réduction de l'accélération de la fréquence cardiaque provoquée par le composé à étudier comparativement à la tachycardie de référence mesurée précédemment (environ 70 battements).

Dans les deux cas, on a exprimé les résultats de la réduction de la pression artérielle ou de la fréquence cardiaque comme suit :

+ pour une réduction < 50%

++ pour une réduction ≧ 50%

+++ pour une réduction sub-totale (réduction presque complète)

On a enregistré les résultats suivants :

A. - Dérivés de benzofuranne

| Composés | Dose (mg/kg) | Effet anti-α | Effet anti-β |
|---|---|---|---|
| Ex. 1 | 3,23 | +++ | ++ |
| Ex. 3 | 1,31 | +++ | ++ |
| Ex. 8 | 1,5 | +++ | +++ |

B. - Dérivés d'indole

| Composés | Dose (mg/kg | Effet anti-α | Effet anti-β |
|---|---|---|---|
| Ex. 10 | 4,9 | +++ | +++ |
| Ex. 11 | 1,6 | ++ | + |
| Ex. 47 | 6,6 | ++ | ++ |
| Ex. 13 | 0,6 | +++ | ++ |
| Ex. 14 | 0,12 | +++ | + |
| Ex. 65 | 0,1 | +++ | +++ |
| Ex. 44 | 1,2 | ++ | ++ |
| Ex. 66 | 0,05 | +++ | +++ |
| Ex. 55 | 0,07 | +++ | +++ |
| Ex. 56 | 0,67 | +++ | ++ |
| Ex. 61 | 0,035 | +++ | ++ |
| Ex. 62 | 1 | +++ | ++ |
| Ex. 64 | 0,5 | +++ | ++ |

C.- Dérivés d'indolizine

| Composés | Dose (mg/kg) | Effet anti-α | Effet anti-β |
|---|---|---|---|
| Ex. 75 | 0,02 | ++ | ++ |
| Ex. 2 | 0,065 | +++ | +++ |
| Ex. 76 | 0,013 | +++ | +++ |
| Ex. 79 | 0,07 | +++ | ++ |
| Ex. 15 | 0,013 | ++ | + |

A titre de comparaison on a obtenu les résultats suivants avec les composés connus cités ci-après :

| Composés | Dose (mg/kg) | Effet anti-α | Effet anti-β |
|---|---|---|---|
| B | 0,5 | ++ | ++ |
| C | 0,1 | +++ | +++ |

D. - Dérivés de pyrazolo[1,5-a]pyridine

| Composés | Dose (mg/kg) | Effet anti-α | Effet anti-β |
|---|---|---|---|
| Ex. 49 | 0,28 | + | + |
| Ex. 50 | 0,13 | +++ | +++ |

III. - Toxicité

La toxicité des composés de l'invention s'est révélée compatible avec leur emploi en thérapeutique.

Les compositions thérapeutiques selon l'invention peuvent être présentées sous toute forme convenant à l'administration en thérapie humaine ou vétérinaire. Pour ce qui concerne l'unité d'administration, celle-ci peut prendre la forme, par exemple, d'un comprimé, d'une dragée, d'une capsule, d'une gélule, d'une poudre, d'une suspension ou d'un sirop pour l'administration orale, d'un suppositoire pour l'administration rectale ou d'une solution ou suspension pour l'administration parentérale.

Les compositions thérapeutiques de l'invention pourront comprendre, par unité d'administration, par exemple de 50 à 500mg en poids d'ingrédient actif pour l'administration orale, de 50 à 200mg d'ingrédient actif pour l'administration rectale et de 50 à 150 d'ingrédient actif pour l'administration parentérale.

Suivant la voie d'administration choisie, les compositions thérapeutiques ou vétérinaires de l'invention seront préparées en associant au moins un des composés de formula (1) ou un sel d'addition non toxique de ce composé avec un excipient approprié, ce dernier pouvant être constitué par exemple d'au moins un ingrédient sélectionné parmi les substances suivantes : lactose, amidons, talc, stéarate de magnésium, polyvinylpyrrolidone, acide alginique, silice colloïdale, eau distillée, alcool benzylique ou agents édulcorants.

Les Exemples, non limitatifs suivants, illustrent l'invention :

## EXEMPLE 1

Préparation du chlorhydrate de n-butyl-2[{[N-méthyl N-(diméthoxy-3,4 - phénéthyl)amino]-3 propoxy}-4 benzènesulfonyl]-3 nitro-5 benzofuranne (SR 33633 A)

a) n-Butyl-2 (hydroxy-4 benzènesulfonyl)-3 nitro-5 benzofuranne

On agite durant 5 min à 20°C, un mélange de 2,6 g (0,011 mole) de chlorure de p-méthoxybenzènesulfonyle, 1,6 g de chlorure d'aluminium et 40 ml de dichloréthane. On refroidit à 0°C et on ajoute 2,5 g (0,011 mole) de n-butyl-2 nitro-5 benzofuranne dans 3 ml de dichloréthane. On laisse revenir durant 1 h à température ambiante, on ajoute 1,4 g de chlorure d'aluminium et on agite encore durant 2 heures. Après addition supplémentaire de 4,2 g de chlorure d'aluminium, on porte le mélange au reflux durant 20 min. On coule dans un mélange éther éthylique/eau/glace et on lave 3 fois avec de l'eau. On traite au moyen de 300 ml d'une solution d'hydroxyde de sodium à 2% puis on lave la phase aqueuse brune avec de l'éther éthylique. On traite cette phase aqueuse par une solution d'acide chlorhydrique concentrée puis on extrait le dérivé phénolique avec de l'éther éthylique. On agite la phase organique en présence de sulfate de sodium et de noir animal, on filtre et on concentre.

De cette manière on obtient 2,8 g de n-butyl-2 (hydroxy-4 benzènesulfonyl)-3 nitro-5 benzofuranne avec un rendement de 68%.

Spectre U.V. (méthanol) : λ max : 246 $cm^{-1}$, 204 $cm^{-1}$

b) n-Butyl-2[(bromo-3 propoxy)-4 benzènesulfonyl]-3 nitro-5 benzofuranne

On chauffe durant 1 min à 120°C, un mélange de 14 mmoles de n-butyl-2(hydroxy-4 benzènesulfonyl)-3 nitro-5 benzofuranne et 20 mmoles de carbonate de potassium dans 30 ml de N,N-diméthylformamide. On ajoute alors 280 mmoles de dibromo-1,3 propane et on poursuit l'agitation durant 15 minutes à cette température. On coule dans l'eau et on extrait avec de l'éther éthylique. On sèche sur sulfate de sodium, filtre et concentre. On purifie alors sur colonne de silice en utilisant un mélange acétate d'éthyle/n-hexane 4/6.

De cette manière, on obtient 10,8 mmoles de n-butyl-2[(bromo-3 propoxy)-4 benzènesulfonyl]-3 nitro-5 benzofuranne après recristallisation dans un mélange éther éthylique/pentane.

Rendement : 77%.

P.F. : 101°C.

c) Chlorhydrate de n-butyl-2[{[N-méthyl N-(diméthoxy-3,4β-phénéthyl)amino]-3propoxy}4benzènesulfonyl]-3 nitro-5 benzofuranne

On agite durant 10 à 12 heures, à température ambiante, un mélange de 8 mmoles de n-butyl-2[(bromo-3 propoxy)-4 benzènesulfonyl]-3 nitro-5 benzofuranne, 10 mmoles de carbonate de potassium et 8 mmoles de N-méthyl diméthoxy-3,4 phénéthyl amine dans 14 ml de N,N-diméthylformamide. On coule dans l'eau et on extraît avec un mélange acétate d'éthyle/dichlorométhane 8/2. On concentre et on élue sur colonne de silice successivement par du méthanol et de l'acétone pour obtenir 4,8 mmoles (rendement : 60%) du composé désiré sous forme basique. On dissout cette huile dans du toluène et on traite la solution par de l'acide chlorhydrique.

De cette manière, on obtient le chlorhydrate de n-butyl-2{[N-méthyl N-(diméthoxy-3,4β-phénéthyl)amino]-3 propoxy-4 benzènesulfonyl}-3 nitro-5 benzofuranne.

P.F. : 162,9°C (méthyl éthyl cétone)

## EXEMPLE 2

Préparation de l'oxalate acide d'isopropyl-2 méthyl-8{[(diméthoxy-6,7 tetrahydro-1,2,3,4 ,N-isoquinoléinyl-2)-3 propoxy]-4 benzènesulfonyl}-1 indolizine (SR 33873 A)

a) (Méthoxy-4 benzènesulfonyl)méthyl-2 méthyl-3 pyridine

On agite à 80°C pendant 2,5 heures, un mélange de 0,050 mole de chlorhydrate de chlorométhyl-2 méthyl-3 pyridine, 0,050 mole de méthoxy-4 benzènesulfinate de sodium et 0,070 mole de bicarbonate de sodium dans 20 ml de toluène contenant 0,8 g de chlorure de méthyltrioctylammonium. On refroidit, verse dans 100 ml d'eau et extrait avec 3 fois 125 ml d'acétate d'éthyle pour obtenir 17 g d'une pâte brunâtre que l'on purifie sur colonne de silice (solvant : dichlorométhane/acétate d'éthyle 5/1).

De cette manière, on obtient la (méthoxy-4 benzènesulfonyl)méthyl-2 méthyl-3 pyridine sous forme d'un solide blanc avec un rendement de 85,2%.

Pureté : 98,9%.

P.F. : 84-85°C.

b) Isopropyl-2 méthyl-8 (méthoxy-4 benzènesulfonyl)-1 indolizine

On agite au reflux durant 48 heures, un mélange de 11,6 g (0,0495 mole) de (méthoxy-4 benzènesulfonyl)méthyl-2 méthyl-3 pyridine, 7,2 g (0,051 mole) de carbonate de potassium et 21,2 g (0,128 mole) de bromométhyl isopropyl cétone. On élimine sous vide le solvant et la bromocétone en excès et on reprend le résidu dans l'eau. On extrait au dichlorométhane, isole puis purifie le produit obtenu sur colonne de silice (éluant : dichlorométhane).

De cette manière on obtient 10,8 g d'isopropyl-2 méthyl-8 (méthoxy-4 benzènesulfonyl)-1 indolizine sous forme d'un solide blanc.

Rendement : 74%.

P.F. : 103°C (acétate d'éthyle/n-hexane).

Pureté : 99%.

c) Isopropyl-2 méthyl-8(hydroxy-4 benzènesulfonyl)-1 indolizine

On met en suspension dans 20 ml de N,N-diméthylformamide 15 g (0,024 mole) d'hydrure de sodium à 50% dans l'huile. On y ajoute 0,012 mole de thio-2 éthanol et on agite durant 15 minutes à température ambiante. On introduit alors, par petites portions, 2 g (0,00582 mole) d'isopropyl-2 méthyl-8(méthoxy-4 benzènesulfonyl)-1 indolizine et on porte à 130°C. Après 45 minutes, une chromatographie sur couche mince indique que le produit méthoxylé de départ est totalement transformé. On refroidit le mélange, verse dans de l'eau et extrait d'huile obtenue avec de l'hexane. On acidifie la phase aqueuse et on extrait à l'acétate d'éthyle. On purifie alors sur colonne de silice en éluant au moyen d'un mélange dichlorométhane/acétate d'éthyle 80/20.

De cette manière, on obtient l'isopropyl-2 méthyl-8(hydroxy-4 benzènesulfonyl)-1 indolizine sous forme d'un solide blanc.
Rendement : 100%.
P.F. : 191-193°C.
Pureté : 98,72%.

d) Oxalate acide d'isopropyl-2 méthyl-8{[(diméthoxy-6,7 tétrahydro-1,2,3,4 N-isoquinoléinyl-2)-3propoxy]-4 benzènesulfonyl}-1 indolizine

On chauffe durant 0,5 h à 50°C, un mélange de 2 g (6 mmoles) d'isopropyl-2 méthyl-8 (hydroxy-4 benzènesulfonyl)-1 indolizine dans 10 ml de N,N-diméthylformamide. On ajoute 1,6 g (6 mmoles) de (chloro-3 propyl)-2 diméthoxy-6,7 tétrahydro-1,2,3,4 isoquinoléine et on chauffe, sous agitation, à 80-90°C durant 3 heures. On refroidit, verse dans de l'eau et extrait avec du dichlorométhane. On purifie alors, par chromatographie sur silice, l'huile ainsi obtenue (éluant : acétate d'éthyle/méthanol 9/1) ce qui fournit 2,7 g d'une huile formée du composé désiré sous forme basique. Au repos cette huile se solidifie. On dissout alors cette base dans du méthanol et on ajoute un équivalent d'acide oxalique, ce qui provoque la précipitation d'un oxalate après une dizaine de minutes. On réchauffe pour le redissoudre et on laisse cristalliser.

De cette manière, on obtient 3,35 g d'oxalate acide d'isopropyl-2 méthyl-8 {[(diméthoxy-6,7 tétrahydro-1,2,3,4 N-isoquinoléinyl-2)-3 propoxy]-4 benzènesulfonyl}-1 indolizine sous forme d'un solide blanc.
Rendement : 85,5%.
P.F. : 199-200°C.
Pureté : 98,2%.

## EXEMPLE 3

### Préparation du dichlorhydrate d'amino-5[{[N-méthylN-(diméthoxy-3,4β-phénéthyl)amino]-3propoxy}-4 benzènesulfonyl]-3 n-butyl-2 benzofuranne (SR 33668 A)

On agite durant 2 heures sous atmosphère d'hydrogène, une solution de n-butyl-2 {[N-méthyl N-(diméthoxy-3,4β-phénéthyl) amino]-3 propoxy-4 benzènesulfonyl}-3 nitro-5 benzofuranne sous forme de base dans 80 ml d'éthanol absolu en présence de 0,2 g d'oxyde de platine. On filtre et évapore à sec. On reprend dans l'acétate d'éthyle et on purifie sur colonne de silice neutralisée par de la diéthylamine en éluant avec de l'acétate d'éthyle contenant 10% de méthanol ce qui fournit 4,15 g (rendement : 80%) de composé désiré sous forme basique. On traite ensuite, par de l' acide chlorhydrique, 1,5 g de cette base en solution dans de l'éther éthylique sec. On filtre et on recristallise 2 fois dans un mélange acétone/méthanol.

De cette manière, on obtient 0,55 g de dichlorhydrate d'amino-5 {[N-méthyl N-(diméthoxy-3,4β-phénéthyl)amino]-3 propoxy-4 benzènesulfonyl}-3 n-butyl-2 benzofuranne avec un rendement de 33%.
P.F. : 163°C.

## EXEMPLE 4

### Préparation de l'oxalate acide de [{[N-méthylN-(diméthoxy-3,4β-phénéthyl)amino]-3propoxy}-4 benzènesulfonyl]-3 n-butyl-2 méthanesulfonamido-5 benzofuranne (SR 33669 A)

On traite goutte à goutte une solution d'amino-5[{[N-méthyl N-(diméthoxy-3,4β-phénéthyl)amino]-3 propoxy}-4 benzènesulfonyl]-3 n-butyl-2 benzofuranne sous forme de base libre et de triéthylamine dans 25 ml de dichloréthane sec, au moyen de 0,84 g de chlorure de méthanesulfonyle dans 10 ml de dichloréthane. On poursuit l'agitation durant 3 heures, on lave à l'eau et on évapore à sec. On purifie sur colonne de silice traitée par de la diéthylamine en éluant au moyen d'acétate d'éthyle contenant 2,5% de méthanol. On traite alors au moyen d'une solution d'acide oxalique une solution du composé désiré sous forme basique ainsi obtenu.

De cette manière, on obtient 0,5 g d'oxalate acide de [{[N-méthyl N-(diméthoxy-3,4β-phénéthyl)amino]-3 propoxy}-4 benzènesulfonyl]-3 n-butyl-2 méthanesulfonamido-5 benzofuranne.
Rendement : 15%.
P.F. : environ 83°C.

EXEMPLE 5

Préparation de l'oxalate acide de [{[N-méthylN-(diméthoxy-3,4β-phénéthyl)amino]-3propoxy}-4 benzènesulfonyl]-1 isopropyl-2 éthoxy-6 indolizine

a) Acétoxyméthyl-2 éthoxy-5 pyridine

On chauffe à reflux 18,3 g de méthyl-2 éthoxy-5 pyridine-N-oxyde dans 125 ml d'anhydride acétique pendant 3 heures. On élimine l'acide et l'anhydride acétiques sous vide et on distille le résidu huileux sous vide.

De cette manière, on obtient l'acétoxyméthyl-2 éthoxy-5 pyridine avec un rendement de 75,9%.

P.E. : 105-110°C (7,5 x $10^{-2}$ Torr)

b) Chlorhydrate de chlorométhyl-2 éthoxy-5 pyridine

On reflue, pendant 1,5 heure, 20 g (0,102 mole) d'acétoxyméthyl-2 éthoxy-5 pyridine dans 85 ml d'éthanol et 195 ml d'hydroxyde de sodium N. On évapore l'éthanol sous vide, acidifie et extrait la phase aqueuse à l'acétate d'éthyle. On lave à l'eau et on isole ainsi 15 g de carbinol pratiquement pur. On reprend ce carbinol dans 60 ml de chloroforme, on ajoute 0,12 mole de chlorure de thionyle et on porte au reflux pendant 2 heures. On évapore à sec sous vide, on reprend dans 100 ml d'acétone le résidu pâteux obtenu et on agite à température ambiante pendant 10 à 12 heures pour former un produit solide. On dilue avec 100 ml d'éther éthylique anhydre puis on essore.

De cette manière, on obtient 19 g de chlorhydrate de chlorométhyl-2 éthoxy-5 pyridine sous forme d'un solide blanc brunâtre.

Rendement : 89,5%.

P.F. : 75°C (isopropanol/acétone/éther éthylique).

c) (Benzyloxy-4 benzènesulfonyl) méthyl-2 éthoxy-5 pyridine

On agite, sans solvant, 2,6 g (10 mmoles) de chlorhydrate de chlorométhyl-2 éthoxy-5 pyridine, 13 mmoles de bicarbonate de sodium et 10 mmoles de benzyloxy-4 benzènesulfinate de sodium. On ajoute alors 20 gouttes de chlorure de méthyltrioctylammonium, on chauffe à 80°C au bain d'huile et on ajoute 10 ml de toluène pour fluidifier et permettre l'agitation du milieu réactionnel. Après 2 h à cette température, on verse dans l'eau et on extrait à l'acétate d'éthyle. On obtient ainsi 4,4 g d'un solide beige que l'on purifie par chromatographie sur silice en utilisant un mélange dichloréthane/acétate d'éthyle 90/10.

De cette manière, on obtient 2,6 g de (benzyloxy-4 benzènesulfonyl) méthyl-2 éthoxy-5 pyridine.

P.F. : 133-134°C (acétate d'éthyle/n-hexane).

d) (Benzyloxy-4 benzènesulfonyl)-1 isopropyl-2 éthoxy-6 indolizine

On porte au reflux, pendant 24 heures, 16,95 g (0,044 mole) de (benzyloxy-4 benzènesulfonyl)méthyl-2 éthoxy-5 pyridine, 22 g (0,133 mole) de bromométhyl isopropyl cétone et 7,75 g (0,055 mole) de carbonate de potassium dans 150 ml de méthyl éthyl cétone. On réajoute alors 11 g (1,5 équivalent) de bromométhyl isopropyl cétone et 8 g de carbonate de potassium et on poursuit le reflux durant 24 heures supplémentaires. On évapore alors le solvant, on reprend le résidu dans l'eau et on extrait au dichlorométhane. On lave les extraits à l'eau et on isole la phase organique. On élimine la bromocétone en excès sous vide et on obtient ainsi 26 g d'une huile brunâtre. On la triture avec de l'éther anhydre sec pendant quelques heures et on essore puis lave à l'éther éthylique le solide formé.

De cette manière, on obtient la (benzyloxy-4 benzènesulfonyl)-1 isopropyl-2 éthoxy-6 indolizine que l'on recristallise dans un mélange acétone/eau 3/1.

Rendement : 69,9%.

P.F. : 153°C.

e) (Hydroxy-4 benzènesulfonyl)-1 isopropyl-2 éthoxy-6 indolizine

On fait bouillir pendant 2 heures, dans 50 ml de méthanol, 1,3 g de (benzyloxy-4 benzènesulfonyl)-1 isopropyl-2 éthoxy-6 indolizine, 0,9 g de charbon palladié à 10% et 1,5 g de formiate d'ammonium. On filtre et évapore le filtrat à sec pour obtenir 0,8 g d'un solide blanc que l'on purifie sur colonne de silice (éluant : dichlorométhane) puis recristallise dans un mélange méthanol/eau.

De cette manière, on obtient 0,6 g d'(hydroxy-4 benzènesulfonyl)-1 isopropyl-2 éthoxy-6 indolizine avec un rendement de 60%.

P.F. : 220-221°C.

Pureté : 100%.

f) Oxalate acide de [{[N-méthyl N-(diméthoxy-3,4β-phénéthyl)amino]-3propoxy-} 4 benzènesulfonyl]-1 isopropyl-2 éthoxy-6 indolizine (SR 33890 A)

Le composé a été obtenu selon la méthode décrite à l'Exemple 2.

P.F. : 146°C (acétate d'éthyl/méthanol/éther éthylique)

EXEMPLE 6

Préparation de l'oxalate de [{[N-méthyl N-(diméthoxy-3,4β-phénéthyl)amino]-3 propoxy}-4 phenyl](isopropyl-2 diméthoxy-3,5 phényl)sulfone (SR 33771 A)

a) (Hydroxy-4benzènesulfonyl)-2 isopropyl-1 diméthoxy-3,4 benzène

On agite, à 50°C durant 5 heures, un mélange de 2 g (0,011 mole) d'isopropyl-1 diméthoxy-3,4 benzène, 3,2 g (0,011 mole) de benzyloxy-4 benzènesulfonate de sodium, 45 ml d'acide méthanesulfonique anhydre et 4,5 g d'anhydride phosphorique. On verse dans un mélange glace/eau, filtre et lave le produit sur filtre avec de l'eau. On reprend le solide encore humide avec 20 ml d'éthanol et 5 ml d'une solution d'hydroxyde de sodium à 30%. On chauffe à 60°C pendant environ 2 h jusqu'à dissolution complète, on dilue avec de l'eau, filtre et acidifie la solution avec de l'acide acétique. On filtre et lave le produit sur filtre avec de l'eau. On purifie alors par chromatographie sur colonne de silice en utilisant un mélange dichloroéthane/acétate d'éthyle 95/5.

De cette manière, on obtient 1,5 g d'(hydroxy-4 benzènesulfonyl)-2 isopropyl-1 diméthoxy-3,4 benzène. Rendement : 40%.

b) Oxalate de [{[N-méthyl-N-(diméthoxy-3,4β-phénéthyl)amino]-3 propoxy}-4 phényl](isopropyl-2 diméthoxy-3,5 phényl)sulfone

Ce composé a été obtenu selon la méthode décrite à l'Exemple 2.
P.F. : 105°C (acétate d'éthyle/isopropanol).

En utilisant les procédés décrits précédemment, on a préparé les composés suivants :
Oxalate acide de chloro-5 n-butyl-2 { {[N-méthyl N-(diméthoxy-3,4β-phénéthyl)amino]-3 propyl}oxy-4 dibromo-3,5 benzènesulfonyl} -3 benzofuranne (SR 33851 A). (Exemple 7) - P.F. : 114°C (éthanol)
Oxalate acide de bromo-5 n-butyl-2[{[N- méthyl N-(diméthoxy-3,4β-phénéthyl) amino]-3 propoxy]-4 benzènesulfonyl]-3 benzofuranne (SR 33844 A) (Exemple 8). P.F. : 114°C.
Oxalate acide de [{[N-méthyl N-(diméthoxy-3,4β-phénéthyl)amino]-3 propoxy]-4 phénylthio]-3 méthoxy-5 méthyl-2 indole (SR 33875 A) (Exemple 9).
P.F. : 162°C (éthanol/méthanol 1/1).
Dioxalate de [{[N-méthyl N-(diméthoxy-3,4β-phénéthyl)amino]-3 propoxy}-4 benzènesulfonyl]-2 {[N-méthyl N-(diméthoxy-3,4β-phénéthyl)amino]-3 propyl}-1 isopropyl-3 indole (SR 33806 A) (Exemple 10).
P.F. : environ 115°C (isopropanol/acétate d'éthyle).
Dioxalate de [{[N-méthyl N-(diméthoxy-3,4β-phénéthyl)amino]-3 propoxy}-4 benzènesulfonyl]-3 diméthylaminoéthyl-1 isopropyl-2 indole (SR 33788 A) (Exemple 11).
P.F. : 114°C (pâteux).
Dioxalate de [{[N-méthyl N-(diméthoxy-3,4β-phénéthyl)amino]-3 propoxy}-4 benzènesulfonyl]-3 diéthylaminoéthyl-1 isopropyl-2 indole (SR 33782 A) (Exemple 12).
P.F. : pâteux à partir de 115°C (isopropanol/acétate d'éthyle).
[{[N-Méthyl N-(diméthoxy-3,4β-phénéthyl) amino]-3 propoxy}-4 benzènesulfonyl]-3 chloro-5 isopropyl-2 méthyl-1 indole (SR 33867) (Exemple 13)-P.F. : 92,7°C.
Oxalate acide d'isopropyl-2 méthyl-3[{[N-(dimethoxy-3,4β-phénéthyl)amino]-3 propoxy}-4 benzènesulfonyl]-1 indolizine (SR 33762 A) (Exemple 14)
P.F. : 157-158°C.
Oxalate acide d'isopropyl-2 méthyl-8[{[N-méthyl N-(diméthoxy-3,4β-phénéthyl)amino]-3 propoxy}-4 benzènesulfonyl]-1 indolizine (SR 33871 A) (Exemple 15)
P.F. : 161°C.
Dichlorhydrate de [(méthyl-4 benzènesulfonyl)-4 phényl]-oxy-1 (méthyl-4 pipérazinyl-1]-3 propanol-2 (LB 31808) (Exemple 16).
P.F. : 206-208°C.
Oxalate acide de [(diméthylamino-3 propyl)oxy]-4 méthyl-4' diphényl sulfone (LB 31804) (Exemple 17).
P.F. : 131-133°C.
Oxalate acide de [(di-n-propylamino-3 propyl)oxy]-4 méthyl-4' diphénylsulfone (LB 31805) (Exemple 18).
P.F. : 90-92°C.
Oxalate acide de [(diéthylamino-3 propyl)oxy]-4 méthyl-4' diphénylsulfone (LB 31806) (Exemple 19).
P.F. : 118-120°C.
Oxalate acide de [(di-n-butylamino-3 propyl)oxy]-4 méthyl-4 diphénylsulfone (LB 31770) (Exemple 20).
P.F. : 116-118°C.

Chlorhydrate de [(diméthylamino-2 éthyl)oxy]-4 méthyl-4' diphénylsulfone (LB 31771) (Exemple 21).
P.F. : 143-145°C.
Chlorhydrate de [(diéthylamino-2 éthyl)oxy]-4 méthyl-4' diphénylsulfone (LB 31772) (Exemple 22).
P.F. : 153-154°C.
[(Méthoxy-4 phényl)-4 pipérazinyl-1]-3 [(méthyl-4-benzènesulfonyl)-4 phényloxy]-1 propanol-2 (LB 31853) (Exemple 23).
P.F. : 124-127°C.
Chlorhydrate de [(chloro-4 phényl)-4 pipérazinyl-1]-3 [(méthyl-4 benzènesulfonyl)-4 phényloxy]-1 propanol-2 (LB 31902) (Exemple 24).
P.F. : 228-229°C.
Chlorhydrate de [(chloro-2 phényl)-4 pipérazinyl-1]-3[(méthyl-4 benzènesulfonyl)-4 phényloxy]-1 propanol-2 (LB 31903) (Exemple 25)
P.F. : 226-227°C.
Dichlorhydrate de [(méthoxy-2 phényl)-4 pipérazinyl-1]-3 [(méthyl-4 benzènesulfonyl)-4 phényloxy]-1 propanol-2 (LB 31916) (Exemple 26).
P.F. : 198-200°C.
[ Dichloro-2,3 (méthyl-4 benzènesulfonyl)-4 phényl]oxy-1 N-[(méthoxy-2 phényl)-4 pipérazinyl-1]-3 propanol-2 (LB 32776) (Exemple 27).
P.F. : 159-160°C.
Chlorhydrate de [dichloro-2,3 (méthyl-4 benzènesulfonyl)-4 phényl]oxy-1 N-tertiobutylamino-3 propanol-2 (LB 32803) (Exemple 28).
P.F. : 221-223°C.
[(Chloro-2 phényl)-4 pipérazinyl-1]-1 [dichloro-2,3 (méthyl-4 benzènesulfonyl)-4 phényl]oxy-3 propanol-2 (LB 32804) (Exemple 29).
P.F. : 141-145°C.
Chlorhydrate de[dichloro-2,3 (méthyl-4 benzènesulfonyl)-4 phényl]oxy-1 N-isopropylamino-3 propanol-2 (LB 32856) (Exemple 30).
P.F. : 212-213°C.
[ Dichloro-2,3 (méthoxy-4 benzènesulfonyl)-4phényl]oxy-1 [(méthyl-2 phényl) -4 pipérazinyl-1]-3 propanol-2 (LB 32956) (Exemple 31).
P.F. : 147-148°C.
[Dichloro-2,3 (méthyl-4 benzènesulfonyl)-4 phényl]oxy-1 N[(méthyl-2 phényl)-4 pipérazinyl-1]-3 propanol-2 (LB 32957) (Exemple 32)
P.F. : 144-145°C.
Chlorhydrate de [dichloro-2,3 (méthoxy-4 benzènesulfonyl)-6 phényl]oxy-1 [(méthoxy-2 phényl)-4 pipérazinyl-1]-3 propanol-2 (LB 32986) (Exemple 33).
P.F. : 190-192,5°C.
[(Chloro-2 phényl)-4 pipérazinyl-1]-1 [dichloro-2,3 (méthoxy-4 benzènesulfonyl)-4 phényl]oxy-3 propanol-2 (LB 32988) (Exemple 34).
P.F. : 155-157°C.
Chlorhydrate de [dichloro-2,3 (méthoxy-4 benzènesulfonyl)-4 phényl]oxy-1 [(diméthoxy-3,4 phényl)-2 éthylamino]-3 propanol-2 (LB 32989) (Exemple 35).
P.F. : 162-170°C.
Chlorhydrate de [dichloro-2,3 (méthoxy-4 benzènesulfonyl)-6 phényl]oxy-1 [(méthyl-2 phényl)-4 pipérazinyl-1]-3 propanol-2 (LB 33007) (Exemple 36).
P.F. : 135-137°C.
Chlorhydrate de[dichloro-2,3 (méthoxy-4 benzènesulfonyl)-6 phényl]oxy-1 [(chloro-2 phényl)-4 pipérazinyl-1]-3 propanol-2 (LB 32008) (Exemple 37).
P.F. : 140-142°C.
Oxalate acide de [dichloro-2,3 (méthyl-4 benzènesulfonyl)-6 phényl]oxy-1 [(méthoxy-2 phényl)-4 pipérazinyl-1]-3 propanol-2 (LB 33015) (Exemple 38).
P.F. : 181-183°C.
Chlorhydrate de [dichloro-2,3 (méthyl-4 benzènesulfonyl)-4 phényl]oxy-1 [(méthyl-2 phényl)-4 pipérazinyl-1]-3 propanol-2 (LB 32016) (Exemple 39).
P.F. : 178-180°C.
Chlorhydrate de [dichloro-2,5 (méthyl-4 benzènesulfonyl)-4 phényl]oxy-1 N-isopropylamino-3propanol-2 (LB 33097) (Exemple 40).
P.F. : 209-210°C.

Chlorhydrate de [dichloro-2,6 (méthyl-4 benzènesulfonyl)-4 phényl]oxy-1 [ (méthoxy-2 phényl)-4 pipérazinyl-1]-3 propanol-2 (LB 33096) (Exemple 41).
P.F. : 193,5-194°C.
Chlorhydrate de [chloro-2 (méthyl-4 benzènesulfonyl)-4 phényl]oxy-1 N-isopropylamino-3 propanol-2 (LB 33098) (Exemple 42).
P.F. : 205-206°C.
Oxalate acide de N-cyclohexylamino-2 [dichloro-2,3 (méthoxy-4benzènesulfonyl)-6 phényl]oxy-3 propanol-2(LB 33094) (Exemple 43).
P.F. : 209-211°C.
Oxalate acide de méthyl-1 isopropyl-2{[(di-n-butylamino)-3 propoxy]-4 benzènesulfonyl}-3 chloro-5 indole (SR 33879 A) (Exemple 44).
P.F. : 155°C (éthanol).
Oxalate acide de méthyl-1 isopropyl-2[{[N-méthyl N-(diméthoxy-3,4 β-phénéthyl) amino]-3 propoxy}-4 benzènesulfonyl]-3 méthyl-6 indole (SR 33884 A) (Exemple 45).
P.F. : 153°C (isopropanol).
Oxalate acide de méthyl-1 isopropyl-2 {[(di-n-butylamino)-3 propoxy]-4 benzènesulfonyl}-3 méthyl-6 indole (Exemple 46) (SR 33930 A)
P.F. : environ 80°C (pâteux) (isopropanol).
Oxalate acide de méthyl-1 isopropyl-2[{[N-méthyl N-(tétrahydro-1,2,3,4, naphtyl-1) amino]-3 propoxy} -4 benzènesulfonyl]-3 chloro-5 indole (Exemple 47) (SR 33925 A)
P.F. : environ 105°C (acétate d'éthyle/isopropanol).
Oxalate acide de {[(di-n-butylamino)-3 propoxy]-4 benzènesulfonyl}-3 diméthyl-2,7 pyrazolo-[1,5-a] pyridine (SR 33889 A) (Exemple 48).
P.F. : 129°C (isopropanol).
Oxalate acide de {[(di-n-butylamino)-3 propoxy]-4 benzènesulfonyl}-3 diméthyl-2,4 pyrazolo [1,5-a] pyridine (SR 33895 A) (Exemple 49).
P.F. : 148°C (éthanol).
Oxalate acide de [{[N-méthyl N-(diméthoxy-3,4β-phénéthyl)amino]-3 propoxy}-4 benzènesulfonyl]-3 isopropyl-2 méthyl-7 pyrazolo [1,5-a] pyridine (SR 33897 A) (Exemple 50).
P.F. : 164°C (éthanol)
Oxalate acide de [{[N-méthyl N-(tétrahydro-1,2,3,4 naphtyl-1) amino]-3 propoxy}-4 benzènesulfonyl ]-3 diméthyl-2,4 pyrazolo [1,5-a] pyridine (SR 33902 A) (Exemple 51).
P.F. : 121,9°C (éthanol/éther diéthylique).
Oxalate acide de {[(di-n-butylamino)-3 propoxy]-4 benzènesulfonyl}-3 isopropyl-2 méthyl-7 pyrazolo [1,5-a] pyridine (Exemple 52).
{[(Di-n-butylamino)-3 propoxy]-4 benzènesulfonyl}-1 isopropyl-2 éthoxy-6 indolizine (Exemple 53) (SR 33904).
P.F. : 74,5°C (éthanol/eau 2/1)
Chlorhydrate de benzyloxy-6 isopropyl-2 {[(di-n-butylamino)-3 propoxy]-4 benzènesulfonyl}-1 indolizine (SR 33655 A ) (Exemple 54).
P.F. : 168-169°C (acétate d'éthyle/méthanol)
Oxalate acide de [{[ N-méthyl N-(diméthoxy-3,4β-phénétyl) amino]-3 propoxy}-4 benzènesulfonyl]-2 isopropyl-3 diméthyl-1,7 indole (SR 33931 A) (Exemple55)
P.F. : 186°C (méthanol)
Oxalate acide de (chloro-5 isopropyl-3 méthyl-1 indolyl-2)[{[N-méthyl N-(diméthoxy-3,4β-phénétyl)amino]-3 propoxy}-4 phényl] sulfoxyde (SR 33933 A) (Exemple 56).
P.F. : environ 85°C (isopropanol)
Oxalate acide d'(isopropyl-3 diméthyl-1,7 indolyl-2)[{[N-méthyl N-(diméthoxy)-3,4β-phénéthyl)amino]-3 propoxy}-4 phényl]sulfoxyde(SR 33934 A) (Exemple 57). P.F. : 168°C (éthanol).
[{[N-Méthyl N-(diméthoxy-3,4β- phénéthyl)amino]-3 propoxy}-4 benzènesulfonyl]-2 chloro-5 isopropyl-3 méthyl-1 indole

a) base (SR 33941) (Exemple 58).
P.F. : collant à partir de 50°C.
b) Oxalate acide (SR 33941 A) (Exemple 59)
  P.F. : 178,5°C (éthanol)
c) Chlorhydrate (SR 33941 B) (Exemple 60)
  P.F. : 134°C (acétate d'éthyle/éther éthylique)

Fumarate acide de [{[N-méthyl N-(diméthoxy-3,4β-phénéthyl)amino]-3 propoxy}-4 benzènesulfonyl]-2 isopropyl-3 méthoxy-5 méthyl-1 indole (SR 33949 A) (Exemple 61)

P.F. : 150,3°C (éthanol)

Oxalate acide de [{[N-méthyl N-(diméthoxy-3,4β-phénéthyl)amino]-3 propoxy}-4 benzènesulfonyl]-2 isopropyl-3 méthoxy-5 indole (SR 33958 A) (Exemple 62)

P.F. : environ 98°C (éther éthylique)

Oxalate de [{[N-méthyl N-(diméthoxy-3,4β-phénéthyl)amino]-3 propoxy}-4 benzènesulfonyl]-2 {[N-méthyl N-(diméthoxy-3,4β-phénéthyl)amino]-3 propyl}-1 isopropyl-3 méthoxy-5 indole (SR 33959 A) (Exemple 63)

P.F. : environ 96°C (éther éthylique)

Oxalate acide de {[(di-n-butylamino)-3 propoxy]-4 benzènesulfonyl}-2 isopropyl-3 méthoxy-5 méthyl-1 indole (SR 33965 A) (Exemple 64)

P.F. : 93°C (isopropanol/éther éthylique)

Oxalate acide de [{[N-méthyl N-(diméthoxy-3,4β-phénéthyl)amino]-3 propoxy}-4 benzènesulfonyl]-3 isopropyl-2 diméthyl-1,4 indole (SR 33966 A) (Exemple 65)

P.F. : environ 115°C (éther éthylique)

Oxalate acide de {[(di-n-butylamino)-3 propoxy]-4 benzènesulfonyl}-3 isopropyl-2 diméthyl-1,4 indole (SR 33974 A) (Exemple 66)

P.F. : 72°C (éther éthylique)

Fumarate acide de benzyloxy-5[{[N-méthyl N-(diméthoxy-3,4β-phénéthyl)amino]-3 propoxy}-4 benzènesulfonyl]-2 isopropyl-3 indole (SR 33981 A) (Exemple 67)

P.F. : collant à partir de 90°C (isopropanol)

Fumarate acide d'(isopropyl-3 méthoxy-5 méthyl-1 indolyl-2)[{[N-méthyl N-(diméthoxy-3,4β-phénéthyl)amino]-3 propoxy}-4 phényl]sulfoxyde (SR 33982 A) (Exemple 68)

P.F. : collant à partir de 85°C (éthanol/éther éthylique)

Fumarate acide d'isopropyl-2 diméthyl-1,4[{[N-méthyl N-(diméthoxy-3,4 β-phénéthyl)amino]-3 propoxy}-4 p hénylthio]-3 indole (SR 33984 A) (Exemple 69)

P.F. : collant à partir de 90°C (éther éthylique)

Fumarate acide de [{[N-méthyl N-(diméthoxy-3,4β-phénéthyl)amino]-3 propoxy}-4 benzènesulfonyl]-3 isopropyl-2 diméthyl-1,4 indole (SR 33976 A) (Exemple 70)

P.F. : environ 100°C.

Chlorhydrate de {[(di-n-butylamino)-3 propoxy]-4 benzènesulfonyl}-2 méthyl-1 isopropyl-3 chloro-5 indole (SR 34040 A) (Exemple 71)

P.F. : 148°C (acétate d'éthyle/éther éthylique)

Oxalate acide de [{[N-méthyl N-(diméthoxy-3,4β-phénéthyl)amino]-3 propoxy}-4 benzènesulfonyl]-2 méthyl-1 isopropyl-3 hydroxy-5 indole (SR 34028 A) (Exemple 72)

P.F. : environ 115°C (isopropanol)

Chlorhydrate de [{[N-méthyl N-(diméthoxy-3,4β-phénéthyl)amino]-3 propoxy}-4 benzènesulfonyl]-1 isopropyl-2 chloro-5 indolizine (SR 34059 A) (Exemple 73)

P.F. : 130°C (acétate d'éthyle)

Fumarate acide d'isopropyl-2 méthyl-8[{[N-méthyl N-(diméthoxy-3,4β-phénéthyl)amino]-3 propoxy}-4 benzènesulfonyl]-1 indolizine (SR 33871 B) (Exemple 74)

P.F. : environ 120°C (éthanol)

{[(Di-n-butylamino)-3 propoxy]-4 benzènesulfonyl}-1 isopropyl-2 méthyl-8 indolizine (SR 33882) (Exemple 75)

P.F. : 87-88°C (méthanol)

[{[N-Méthyl N-(diméthoxy-3,5β-phénéthyl)amino]-3 propoxy}-4 benzènesulfonyl]-1 isopropyl-2 méthyl-8 indolizine

- a) Oxalate acide (SR 33919 A) (Exemple 76)
  - P.F. : 157°C (acétate d'éthyle, méthanol, éther éthylique)
- b) Fumarate acide (SR 33919 B) (Exemple 77)
  - P.F. : 139-141°C (décomposition)

Oxalate acide de [{[N-méthyl N-(diméthoxy-3,4β-phénéthyl)amino]-3 propoxy}-4 benzènesulfonyl]-3 éthyl-2 méthyl-4 pyrazolo[1,5-a] pyridine (SR 33929 A) (Exemple 78)

P.F. : 180,9°C (méthanol/éthanol 7/3)

Isopropyl-2 méthyl-5[{[N-méthyl N-(diméthoxy-3,4β-phénéthyl) amino]-3 propoxy}-4 benzènesulfonyl]-1 indolizine

- a) Oxalate acide (SR 33940 A) (Exemple 79) P.F. 112-115°C (acétate d'éthyle/méthanol/éther éthylique)
- b) Chlorhydrate (SR 33940 B) (Exemple 80) Solide amorphe

Oxalate acide de [{[N-méthyl N-(diméthoxy-3,4β-phénéthyl)amino]-3 propoxy}-4 benzènesulfonyl]-3 éthyl-2 méthyl-6 pyrazolo [1,5-a] pyridine (SR 33954 A) (Exemple 81)

P.F. : 140°C (isopropanol)
Oxalate acide de [{[N-méthyl N-(diméthoxy-3,4β-phénéthyl)amino]-3 propoxy}-4 benzènesulfonyl]-1 isopropyl-2 méthyl-6 indolizine (SR 33960 A) (Exemple 82)
P.F. : 144-146°C (méthyl éthyl cétone)
[{[N-Méthyl N-(diméthoxy-3,4β-phénéthyl)amino]-3 propoxy}-4 benzènesulfonyl]-1 isopropyl-2 benzyloxy-6 indolizine (SR 34060) (Exemple 83)
P.F. : 73-74°C (méthanol)
Chlorhydrate de [{[N-méthyl N-(diméthoxy-3,4β-phénéthyl)amino]-3 propoxy}-4 benzènesulfonyl]-1 phényl-2 méthyl-8 indolizine (SR 34050 A) (Exemple 84)
P.F. : entre 100°C et 140°C (décomposition)
Oxalate acide de [{[N-méthyl N-(diméthoxy-3,4β-phénéthyl)amino]-3 propoxy}-4 benzènesulfonyl]-3 triméthyl-1,2,5pyrrole (SR 33997 A) (Exemple 85)
P.F. : 165°C (isopropanol)
Oxalate acide de {[(di-n-butylamino)-3 propoxy]-4 benzènesulfonyl}-3 triméthyl-1,2,5 pyrrole(SR 34020 A) (Exemple 86)
P.F. : 94°C (acétate d'éthyle)
Oxalate acide de (triméthyl-1,2,5-pyrrolyl)[{[N-méthyl N-(diméthoxy-3,4β-phénéthyl)amino]-3 propoxy}-4 phényl]sulfoxyde (SR 34033 A) (Exemple 87)
P.F. : 135°C (isopropanol)
Chlorhydrate de [{[N-méthyl N-(diméthoxy-3,4β-phénéthyl)amino]-3 propoxy}-4 benzènesulfonyl]-2 éthyl-4 diméthyl-3,5 pyrrole (SR 34035 A) (Exemple 88)
P.F. : environ 100°C (éther éthylique)
Fumarate de benzyloxy-5[{[N-méthyl N-(diméthoxy-3,4β-phénéthyl)amino]-3 propoxy}-4 benzènesulfonyl]-2 isopropyl-3 [N-méthyl N-(diméthoxy-3,4β-phénéthyl)amino-3 propyl-1]-1 indole (SR 33983 A) (Exemple 89)
P.F. : collant à partir de 100°C.
Chloro-5 isopropyl-2 {[(di-n-butylamino)-3 propoxy]-4 benzènesulfonyl}-3 méthyl-1 indole (SR 34052) (Exemple 90)
P.F. : 105°C (heptane)
Chlorhydrate d'isopropyl-2 méthyl-8 [(tertiobutylamino-3 propoxy)-4 benzènesulfonyl]-1 indolizine (Exemple 91)
P.F. : 199-200°C (éthanol)
Chlorhydrate d'isopropyl-2 méthyl-8 [(n-butylamino-3 propoxy)-4 benzènesulfonyl]-1 indolizine (Exemple 92)
P.F. : 165°C (isopropanol)
Oxalate acide d'isopropyl-2 méthyl-8 [{[N-triméthyl-3,4,5β-phénéthyl)amino]-3 propoxy}-4 benzènesulfonyl]-1 indolizine (SR 34066 A) (Exemple 93)
P.F. : 206°C (méthanol)
Citrate d'isopropyl-2 méthyl-8[{[N-méthyl N-(diméthoxy-3,4 méthyl-5β-phénéthyl) amino]-3 propoxy}-4 benzènesulfonyl]-1 indolizine (Example 94)
P.F. : environ 50°C (isopropanol/éther éthylique)
Chlorhydrate de triméthyl-1,3,5[{[N- méthyl N-(diméthoxy-3,4β-phénéthyl)amino]-3 propoxy}-4 benzènesulfonyl]-2 éthyl-4 pyrrole (Exemple 95)
Fumarate acide d'isopropyl-2 méthyl-8[{[N-diméthoxy-3,4β-phénéthyl)amino]-3 propoxy}-4 benzènesulfonyl]-1 indolizine (Exemple 96)
Produit pâteux.
Oxalate acide d'isopropyl-2 méthyl-8[{[N-méthyl N-(diméthoxy-3,4 méthyl-5β -phénéthyl) amino]-3 propoxy}-4 benzènesulfonyl]-1 indolizine (Exemple 97)
P.F. : 174-177°C (acétate d'éthyle).

## Revendications

### Revendications pour les Etats contractants suivants : CH, DE, DK, FR, GB, IT, LI, LU, NL, SE, AT

1. Dérivés aminoalkoxyphényle de formule générale :

Cy-B-Ph-O-A-Am (1)

ainsi que leurs sels pharmaceutiquement acceptables, dans laquelle :
B représente un groupement -S-, -SO- ou $-SO_2$, Ph représente un radical de formule :

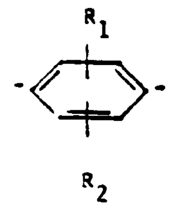

(D)

ou

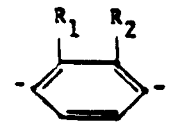

(E)

$R_1$ et $R_2$, qui sont identiques ou différents représentent chacun l'hydrogène, le radical méthyle ou éthyle ou un halogène,

A représente un radical alkylène, linéaire ou ramifié, ayant de 2 à 5 atomes de carbone ou le radical hydroxy-2 propylène dans lequel l'hydroxy est éventuellement substitué par un radical alkyle en $C_1$-$C_4$,

Am représente un groupement :

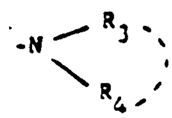

(F)

ou

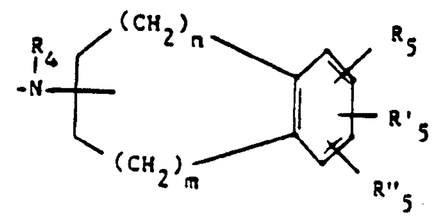

(G)

ou

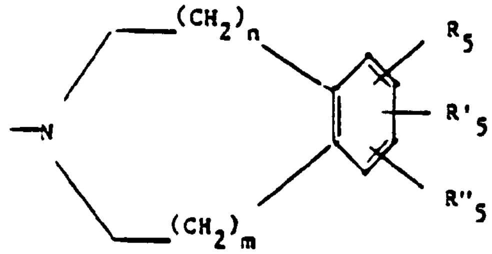

(H)

dans lequel :

$R_3$ représente un radical alkyle en $C_1$-$C_8$, cycloalkyle en $C_3$-$C_6$ ou un radical de formule :

-Alk-Ar

dans laquelle Alk représente une liaison simple ou un radical alkylène, linéaire ou ramifié, ayant de 1 à 5 atomes de carbone et Ar représente un radical pyridyle, phényle, méthylènedioxy-2,3 phényle ou méthylènedioxy-3,4 phényle ou un groupement phényle substitué par un ou plusieurs substituants, identiques ou différents, choisis parmi des atomes d'halogène, des groupements alkyle en $C_1$-$C_4$ ou des groupements alkoxy en $C_1$-$C_4$,

$R_4$ représente l'hydrogène ou un radical alkyle en $C_1$-$C_8$, ou $R_3$ et $R_4$, lorsqu'ils sont pris ensemble. représentant un radical alkylène ou alkénylène ayant de 3 à 6 atomes de carbone et éventuellement substitué par un radical phényle ou éventuellement interrompu par -O-, -N= ou

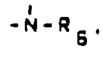

$R_6$ représentant l'hydrogène, un radical alkyle en $C_1$-$C_4$, cycloalkyle en $C_3$-$C_6$, phényle éventuellement substitué par un atome d'halogène ou par un groupement alkyle en $C_1$-$C_4$ ou alkoxy en $C_1$-$C_4$,

$R_5$, $R'_5$ et $R''_5$, qui sont identiques ou différents. représentent chacun l'hydrogène, un atome d'halogène, un groupement alkyle en $C_1$-$C_4$ ou alkoxy en $C_1$-$C_4$

n et m, identiques ou différents représentent chacun 0, 1, 2 ou 3, Cy représente l'un des groupements de formule :

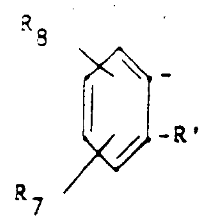

(I)

ou

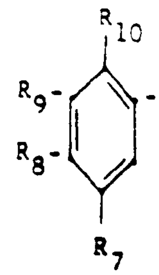

(I')

ou

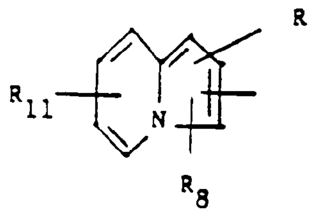

(J)

ou

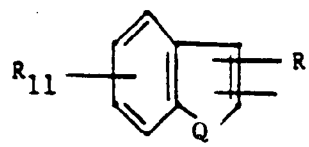

(K)

ou

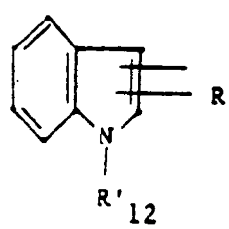

(K')

ou

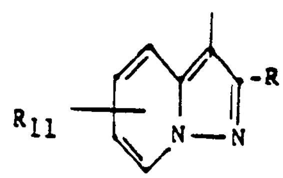

(L)

ou

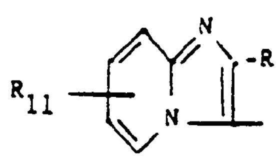

(M)

ou

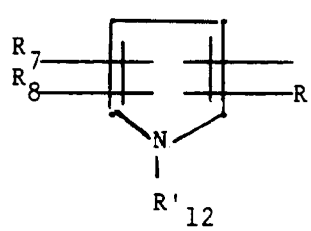

(N)

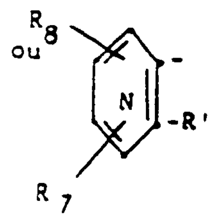

(Q)

ou

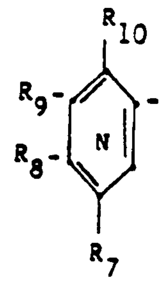

(Q')

dans lequel :

R représente l'hydrogène, un radical alkyl en $C_1$-$C_8$, cycloalkyle en $C_3$-$C_6$, benzyle ou phényle éventuellement substitué par un ou plusieurs substituants, qui, peuvent être identiques ou différents, choisis parmi des atomes d'halogène ou parmi des groupements alkyle en $C_1$-$C_4$, alkoxy en $C_1$-$C_4$ ou nitro,

R' représente un radical alkyle en $C_1$-$C_8$, cycloalkyle en $C_3$-$C_6$, benzyle ou phényle éventuellement substitué par un ou plusieurs substituants, qui peuvent être identiques ou différents choisis parmi des atomes d'halogène ou parmi des groupements alkyle en $C_1$-$C_4$, alkoxy en $C_1$-$C_4$ ou nitro,

$R_7$, $R_8$ et $R_9$, identiques ou différents, représentent chacun l'hydrogène, un radical alkyle en $C_1$-$C_4$, alkoxy en $C_1$-$C_4$, un atome d'halogène, un groupement hydroxy, benzyloxy, nitro, amino, (alkyl en $C_1$-$C_4$)amino, di(alkyl en $C_1$-$C_4$)amino, sulfonamido, (alkyl en $C_1$-$C_4$)sulfonamido, phénylsulfonamido, cyano, (alkoxy en $C_1$-$C_4$)carbonyl ou (alkyl en $C_1$-$C_4$)carbonyle,

$R_{10}$ représente l'hydrogène, un radical alkoxy en $C_1$-$C_4$, un atome d'halogène, un groupement hydroxy, benzyloxy, nitro, amino, (alkyl en $C_1$-$C_4$)amino, di(alkyl en $C_1$-$C_4$)amino, sulfonamido, (alkyl en $C_1$-$C_4$)sulfonamido, phénylsulfonamido, cyano, (alkoxy en $C_1$-$C_4$)carbonyle ou (alkyl en $C_1$-$C_4$)carbonyle,

les groupements $R_7$ et $R_8$ d'une part et $R_7$, $R_8$, $R_9$ et $R_{10}$ d'autre part ne représentant jamais simultanément l'hydrogène et le radical (I) ou (I') ne représentant pas un radical monohalogénophényle. $R_{11}$ représente un radical alkyle en $C_1$-$C_4$, un atome d'halogène, un groupement hydroxy, benzyloxy, nitro, amino, (alkyl en $C_1$-$C_4$)amino, di(alkyl en $C_1$-$C_4$)amino, sulfonamido, (alkyl en $C_1$-$C_4$)sulfonamido, phénylsulfonamido, cyano, (alkoxy en $C_1$-$C_4$)carbonyle ou (alkyl en $C_1$-$C_4$)carbonyle,

Q représente

$$-\overset{|}{N}-R_{12}$$

dans lequel $R_{12}$ représente l'hydrogène, un groupement alkyle en $C_1$-$C_4$, cycloalkyle en $C_3$-$C_6$, benzyle, halogénobenzyle, un groupement -A-Am tel que défini précédemment, un groupement (al- kyl en $C_1$-$C_4$) sulfonyle ou phénylsulfonyle éventuellement substitué par un ou plusieurs atomes d'halogènes, groupements alkyle en $C_1$-$C_4$ ou alkoxy en $C_1$-$C_4$,

$R'_{12}$ représente un groupement -A-Am tel que défini précédemment, un groupement alkylsulfonyle éventuellement substitué par un ou plusieurs atomes d'halogène, groupements alkyle en $C_1$-$C_4$ ou alkoxy en $C_1$-$C_4$.

**2.** Dérivés aminoalkoxyphényle selon la revendication 1 dans laquelle B représente un groupement $-SO_2-$.

**3.** Dérivés aminoalkoxyphényle selon la revendication 1 ou 2, caractérisés en ce que $R_1$ et $R_2$ représentent chacun l'hdyrogène.

**4.** Dérivés aminoalkoxyphényle selon l'une quelconque des revendications 1 à 3, caractérisés en ce que R représente le groupement isopropyle ou cyclopropyle.

**5.** Dérivés aminoalkoxyphényle selon l'une quelconque des revendications 1 à 4 caractérisés en ce que $R_5$, $R'_5$ et $R''_5$, identiques ou différents, représentent chacun l'hydrogène, le chlore, le groupement méthyle ou le groupement méthoxy.

**6.** Dérivés aminoalkoxyphényle selon une des revendications 1 à 5 caractérisés en ce que $R_3$ représente un radical -Alk-Ar.

7. Dérivés aminoalkoxyphényle selon l'une quelconque des revendications 1 à 6, caractérisés en ce que la chaîne -0-A-$NR_3$-$R_4$ représente un groupement [N-méthyl N-(diméthoxy-3,4β-phénéthyl)amino]propoxy ou [N-méthyl N-(diméthoxy-3,5β-phénéthylamino] propoxy.

8. Dérivés aminoalkoxyphényle selon l'une quelconque des revendications 1 à 7, caractérisés en ce que Ph représente un groupement (D).

9. Dérivés aminoalkoxyphényle selon l'une quelconque des revendications 1 à 7, caractérisés en ce que Cy représente un groupement (J).

10. Dérivés aminoalkoxyphényle selon l'une quelconque des revendications 1 à 7, caractérisés en ce que Cy représente un groupement (K) ou (K').

11. Dérivés aminoalkoxyphényle selon l'une quelconque des revendications 1 à 7, caractérisés en ce que Cy représente un groupement (L).

12. Dérivés aminoalkoxyphényle selon l'une quelconque des revendications 1 à 11, caractérisés en ce que le sel pharmaceutiquement acceptable est l'oxalate, le chlorhydrate ou le fumarate.

13. Dérivés aminoalkoxyphényle selon la revendication 1 choisis parmi les composés suivants :
isopropyl-2 méthyl-8 [{[N-méthyl-N-(diméthoxy-3,5β-phénéthyl)amino]-3 propoxy}-4 benzènesulfonyl]-1 indolizine
isopropyl-2 méthyl-8 [{[N-méthyl N-(diméthoxy-3,4β-phénéthyl)amino]-3 propoxy}-4 benzènesulfonyl]-1 indolizine
{[(di-n-butylamino)-3 propoxy]-4-benzènesulfonyl}-1 isopropyl-2 méthyl-8 indolizine
isopropyl-2 méthyl-8{[(diméthoxy-6,7 tétrahydro-1,2,3,4 isoquinoléinyl-2)-3 propoxy]-4 benzènesulfonyl]-1 indolizine
isopropyl-2 méthyl-5[{[N-méthyl N-(diméthoxy-3,4β-phénéthyl)amino]-3 propoxy}-4 benzènesulfonyl]-1 indolizine
[{[N-méthyl N-(diméthoxy-3,4β-phénéthyl)amino]-3 propoxy}-4 benzènesulfonyl]-2 chloro-5 isopropyl-3 méthyl-1 indole
et leurs sels pharmaceutiquement acceptables.

14. Procédé de préparation de dérivés aminoalkoxyphényle de formule (1) selon la revendication 1 dans laquelle A représente un radical alkylène et B représente un groupement -S- ou -$SO_2$-, caractérisé en ce que l'on condense, en présence d'un accepteur d'acide, dans un solvant polaire ou non polaire et à une température comprise entre la température ambiante et la température de reflux, un dérivé alkoxy-4 phényle de formule générale :

Cy-B'-Ph-O-A-X (2)

dans laquelle B' représente un groupement -S- ou -$SO_2$-, Cy et Ph ont la même signification que dans la revendication 1, A a la même signification que précédemment et X représente un atome d'halogène ou un groupement alkylsulfonyloxy ayant de 1 à 4 atomes de carbone ou arylsulfonyloxy ayant de 6 à 10 atomes de carbone, avec une amine de formule générale :

H-Am (3)

dans laquelle Am a la même signification que dans la revendication 1, pour former le dérivé désiré que l'on peut faire réagir, si nécessaire, avec un acide organique ou inorganique pour former un sel pharmaceutiquement acceptable de ce dérivé.

15. Procédé de préparation de dérivés aminoalkoxyphényle de formule (1) selon la revendication 1 dans laquelle B représente un groupement -S- ou -$SO_2$-, caractérisé en ce que l'on fait réagir, en présence d'un agent basique, un dérivé hydroxy-4 phényle de formule générale :

Cy-B'-Ph-OH (4)

dans laquelle B' représente un groupement -S- ou -$SO_2$- et Cy et Ph ont la même signification que dans la revendication 1, avec un composé de formule générale :

X-A-Am (31)

dans laquelle X représente un atome d'halogène ou un groupement alkylsulfoxy ayant de 1 à 4 atomes de carbone ou arylsulfoxy ayant de 6 à 10 atomes de carbone. A représente un radical alkylène et Am a la même valeur que dans la revendication 1, la réaction ayant lieu au reflux et dans un solvant approprié pour obtenir le dérivé désiré que l'on peut, si on le désire, faire réagir avec un acide organique ou inorganique approprié, pour former un sel pharmaceutiquement acceptable.

**16.** Procédé de préparation de dérivés amino- alkoxyphényle de formule (1) selon la revendication 1 dans laquelle A repré- sente le radical hydroxy-2 propylène éventuellement substitué et B représente un groupement -S- ou -$SO_2$-, caractérisé en ce que l'on traite au reflux un dérivé oxyranylméthoxy de formule générale :

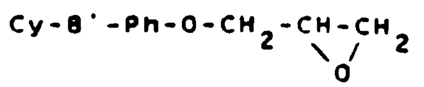 (33)

dans laquelle B' représente un groupement -S- ou -$SO_2$- et Cy et Ph ont la même signification que dans la revendication 1, avec une amine de formule générale :

H-Am (3)

dans laquelle Am a la même signification que dans la revendication 1 et ce, dans un solvant polaire ou dans un excès de ladite amine pour donner :

- le dérivé désiré, sous forme de base libre, dans lequel A représente un radical hydroxy-2 propylène,
- un dérivé aminoalkoxyphényle que l'on peut faire réagir avec un halogénure d'alkyle ayant de 1 à 4 atomes de carbone et en présence d'une base forte, ce qui fournit le dérivé dérisé sous forme de base libre, dans lequel A représente un radical hydroxy-2 propylène dans lequel l'hydroxy est substitué par un alkyle ayant de 1 à 4 atomes de carbone,

le dérivé aminoalkoxyphényle ainsi obtenu pouvant, si on le désire, être mis en réaction avec un acide organique ou inorganique pour former un sel pharmaceutiquement acceptable.

**17.** Procédé de préparation de dérivés aminoalkoxyphényle de formule (1) selon la revendication 1 dans laquelle B représente un groupement -SO-, caractérisé en ce que l'on traite, avec un agent oxydant, un sulfure de formule générale :

Cy-S-Ph-O-A-Am (1)

dans laquelle Cy, Ph, A et Am ont la même signification que dans la revendication 1, ce sulfure étant sous forme de base libre ou de sel, pour former le dérivé désiré sous forme de base libre ou de sel que l'on traite avec un agent basique pour fournir le dérivé désiré sous forme de base libre, la base libre ainsi obtenue étant, si on le désire, mise en réaction avec un acide organique ou inorganique pour former un sel pharmaceutiquement acceptable de ce dérivé.

**18.** Procédé selon la revendication 17, caractérisé en ce que l'agent oxydant est le périodate de sodium, le permangante de potassium ou l'acide chloro-3 perbenzoïque.

**19.** Procédé de préparation de dérivés aminoalkoxyphényle de formule (1) selon la revendication 1 dans laquelle $R_7$, $R_8$, $R_9$, $R_{10}$ ou $R_{11}$ représentent un groupement hydroxy, caractérisé en ce que l'on hydrogène, dans un solvant approprié et en présence d'un catalyseur un composé de formule (1) selon la revendication 1 dans laquelle $R_7$, $R_8$, $R_9$, $R_{10}$ ou $R_{11}$ représentent un groupement benzyloxy, pour obtenir le dérivé désiré que l'on peut, si nécessaire faire réagir avec un acide organique ou inorganique approprié pour former un sel pharmaceutiquement acceptable.

**20.** Procédé de préparation de dérivés aminoalkoxyphényle de formule (1) selon la revendication 1 dans laquelle $R_7$, $R_8$, $R_9$, $R_{10}$ ou $R_{11}$ représentent un groupement amino, alkylamino inférieur ou dialkylamino inférieur, caractérisé en ce que l'on hydrogène dans un solvant approprié et en présence d'un catalyseur, un composé de formule (1) selon la revendication 1 dans laquelle $R_7$, $R_8$, $R_9$, $R_{10}$ ou $R_{11}$ représentent un groupement nitro, ce qui fournit :

- le composé désiré sous forme de base libre dans laquelle $R_7$, $R_8$, $R_9$, $R_{10}$ ou $R_{11}$ représentent un groupement amino.
- un dérivé aminoalkoxyphényle que l'on peut faire réagir avec un quantité appropriée d'un halogénure d'alkyle ayant de 1 à 4 atomes de carbone et en présence d'un agent alcalin, ce qui fournit le dérivé désiré sous forme de base libre, dans lequel $R_7$, $R_8$, $R_9$, $R_{10}$ ou $R_{11}$ représentent un groupement (alkyl en $C_1$-$C_4$)amino ou (dialkyl en $C_1$-$C_4$)amino,

le dérivé aminoalkoxyphényle ainsi obtenu pouvant, si on le désire, être mis en réaction avec un acide organique ou inorganique pour former un sel pharmaceutiquement acceptable.

**21.** Compositions pharmaceutiques ou vétérinaires contenant, comme principe actif, au moins un dérivé aminoalkoxyphényle selon l'une quelconque des revendications 1 à 13, en association avec un véhicule pharmaceutique ou un excipient approprié.

**22.** Compositions pharmaceutiques ou vétérinaires selon la revendication 21 pour le traitement de syndromes pathologiques du système cardiovasculaire contenant de 50 mg à 500 mg de principe actif.

**23.** Utilisation d'au moins un dérivé aminoalkoxyphényle selon l'une quelconque des revendications 1 à 13 pour la fabrication d'un médicament destiné au traitement de syndromes pathologiques du système cardiovasculaire.

**24.** Utilisation d'au moins un dérivé aminoalkoxyphényle selon l'une quelconque des revendications 1 à 13 pour la fabrication d'un médicament destiné au traitement d'affections pathologiques oculaires.

**Revendications par l'Etat contractant suivant : ES**

**1.** Procédé de préparation de dérivés aminoalkoxyphényle de formule générale :

Cy-B-Ph-O-A-Am (1)

ainsi que leurs sels pharmaceutiquement acceptables, dans laquelle :
B représente un groupement -S-, -SO- ou $-SO_2$, Ph représente un radical de formule :

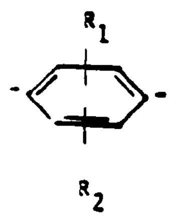

(D)

ou

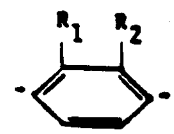

(E)

$R_1$ et $R_2$, qui sont identiques ou différents représentent chacun l'hydrogène, le radical méthyle ou éthyle ou un halogène,
A représente un radical alkylène, linéaire ou ramifié, ayant de 2 à 5 atomes de carbone ou le radical hydroxy-2 propyléne dans lequel l'hydroxy est éventuellement substitué par un radical alkyle en $C_1$-$C_4$,
Am représente un groupement :

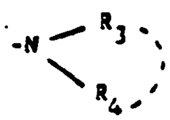

(F)

ou

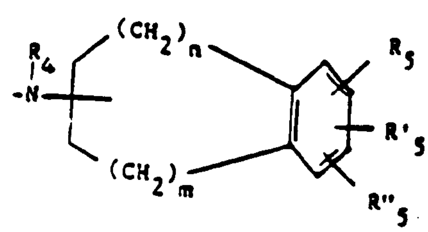

(G)

ou

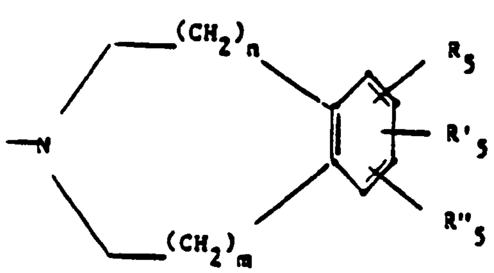

(H)

dans lequel :

$R_3$ représente un radical alkyle en $C_1$-$C_8$, cycloalkyle en $C_3$-$C_6$ ou un radical de formule :

Alk-Ar

dans laquelle Alk représente une liaison simple ou un radical alkylène, linéaire ou ramifié, ayant de 1 à 5 atomes de carbone et Ar représente un radical pyridyle, phényle, méthylènedioxy-2,3 phényle ou méthylènedioxy-3,4 phényle ou un groupement phényle substitué par un ou plusieurs substituants, identiques ou différents, choisis parmi des atomes d'halogène, des groupements alkyle en $C_1$-$C_4$ ou des groupements alkoxy en $C_1$-$C_4$,

$R_4$ représente l'hydrogène ou un radical alkyle en $C_1$-$C_8$, ou $R_3$ et $R_4$, lorsqu'ils sont pris ensemble, représentant un radical alkylène ou alkénylène ayant de 3 à 6 atomes de carbone et éventuellement substitué par un radical phényle ou éventuellement interrompu par -O- -N= ou

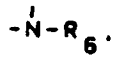

$R_6$ représentant l'hydrogène un radical alkyle en $C_1$-$C_4$, cycloalkyle en $C_3$-$C_6$, phényle éventuellement substitué par un atome d'halogène ou par un groupement alkyle en $C_1$-$C_4$ ou alkoxy en $C_1$-$C_4$,

$R_5$, $R'_5$ et $R''_5$, qui sont identiques ou différents, représentent chacun l'hydrogène, un atome d'halogène, un groupement alkyle en $C_1$-$C_4$ ou alkoxy en $C_1$-$C_4$,

n et m, identiques ou différents représentent chacun 0, 1, 2 ou 3, Cy représente l'un des groupements de formule :

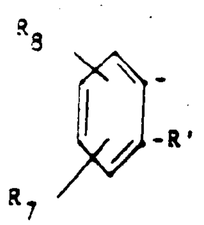

(I)

ou

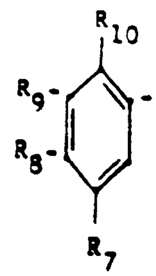

(I')

ou

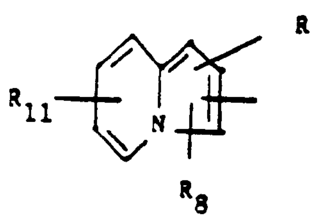

(J)

ou

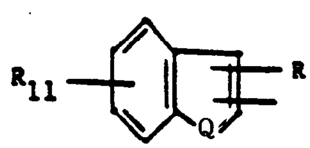

(K)

ou

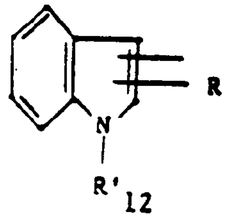

(K')

ou

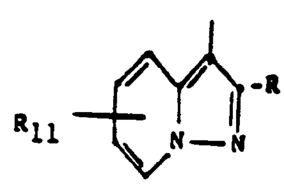

(L)

ou 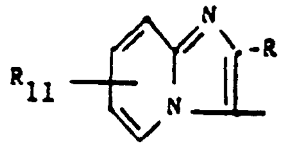

(M)

ou 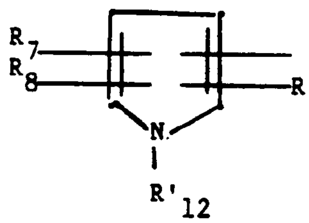

(N)

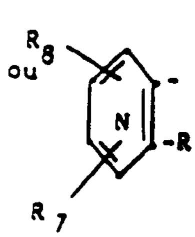

ou

(Q)

ou 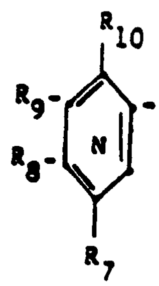

(Q')

dans lequel :

R représente l'hydrogène, un radical alkyle en $C_1$-$C_8$, cycloalkyle en $C_3$-$C_6$, benzyle ou phényle éventuellement substitué par un ou plusieurs substituants, qui, peuvent être identiques ou différents, choisis parmi des atomes d'halogène ou parmi des groupements alkyle en $C_1$-$C_4$, alkoxy en $C_1$-$C_4$ ou nitro,

R' représente un radical alkyle en $C_1$-$C_8$, cycloalkyle en $C_3$-$C_6$, benzyle ou phényle éventuellement substitué par un ou plusieurs substituants, qui peuvent être identiques ou différents choisis parmi des atomes d'halogène ou parmi des groupements alkyle en $C_1$-$C_4$, alkoxy en $C_1$-$C_4$ ou nitro,

$R_7$, $R_8$ et $R_9$, identiques ou différents, représentent chacun l'hydrogène, un radical alkyle en $C_1$-$C_4$, alkoxy en $C_1$-$C_4$, un atome d'halogène, un groupement hydroxy, benzyloxy, nitro, amino, (alkyl en $C_1$-$C_4$)amino, di(alkyl en $C_1$-$C_4$)amino, sulfonamido, (alkyl en $C_1$-$C_4$) sulfonamido, phénylsulfonamido, cyano, (alkoxy en $C_1$-$C_4$)carbonyle ou (alkyl en $C_1$-$C_4$)carbonyle,

$R_{10}$ représente l'hydrogène, un radical alkoxy en $C_1$-$C_4$, un atome d'halogène, un groupement hydroxy, benzyloxy, nitro, amino, (alkyl en $C_1$-$C_4$)amino, di(alkyl en $C_1$-$C_4$)amino, sulfonamido, (alkyl en $C_1$-$C_4$)sulfonamido, phénylsulfonamido, cyano, (alkoxy en $C_1$-$C_4$)carbonyle ou (alkyl en $C_1$-$C_4$)carbonyle,

les groupements $R_7$ et $R_8$ d'une part et $R_7$, $R_8$, $R_9$ et $R_{10}$ d'autre part ne représentant jamais simultanément l'hydrogène et le radical (I) ou (I') ne représentant pas un radical monohalogénophényle.

$R_{11}$ représente un radical alkyle en $C_1$-$C_4$, un atome d'halogène, un groupement hydroxy, benzyloxy, nitro, amino, (alkyl en $C_1$-$C_4$)amino, di(alkyl en $C_1$-$C_4$)amino, sulfonamido, (alkyl en $C_1$-$C_4$)sulfonamido, phénylsulfonamido, cyano, (alkoxy en $C_1$-$C_4$)carbonyl ou (alkyl en $C_1$-$C_4$) carbonyle,

Q représente

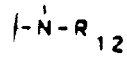

dans lequel $R_{12}$ représente l'hydrogène, un groupement alkyle en $C_1$-$C_4$, cycloalkyle en $C_3$-$C_6$, benzyle, halogénobenzyle, un groupement -A-Am tel que défini précédemment, un groupement (al- kyl en $C_1$-$C_4$)sulfonyle ou phénylsulfonyle éventuellement substitué par un ou plusieurs atomes d'halogènes, groupements alkyle en $C_1$-$C_4$ ou alkoxy en $C_1$-$C_4$,

$R'_{12}$ représente un groupement -A-Am tel que défini précédemment, un groupement alkylsulfonyle éventuellement substitué par un ou plusieurs atomes d'halogène, groupements alkyle en $C_1$-$C_4$ ou alkoxy en $C_1$-$C_4$,

caractérisé en ce que:

A/ Lorsque A représente un radical alkylène et B représente un groupement -S- ou $-SO_2-$. soit l'on condense, en présence d'un accepteur d'acide, dans un solvant polaire ou non polaire et à une température comprise entre la température ambiante et la température de reflux, un dérivé alkoxy-4 phényle de formule générale :

Cy-B'-Ph-O-A-X (2)

dans laquelle B' représente un groupement -S- ou $-SO_2-$, Cy et Ph ont la même signification que dans la revendication 1, A a la même signification que précédemment et X représente un atome d'halogène ou un groupement alkylsulfonyloxy ayant de 1 à 4 atomes de carbone ou arylsulfonyloxy ayant de 6 à 10 atomes de carbone, avec une amine de formule générale :

H - Am (3)

dans laquelle Am a la même signification que dans la revendication 1, pour former le dérivé désiré ,

- soit l'on fait réagir, en présence d'un agent basique, un dérivé hydroxy-4 phényle de formule générale :

Cy-B'-Ph-OH (4)

dans laquelle B' représente un groupement -S- ou $-SO_2-$ et Cy et Ph ont la même signification que dans la revendication 1, avec un composé de formule générale :

X - A - Am (31)

dans laquelle X représente un atome d'halogène ou un groupement alkylsulfoxy ayant de 1 à 4 atomes de carbone ou arylsulfoxy ayant de 6 à 10 atomes de carbone, A représente un radical alkylène et Am a la même valeur que dans la revendication 1, la réaction ayant lieu au reflux et dans un solvant approprié,

B/ Lorsque A représente le radical hydroxy-2 propylène éventuellement substitué et B représente un groupement -S- ou $-SO_2-$ l'on traite au reflux un dérivé oxyranylméthoxy de formule générale

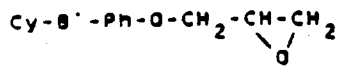 (33)

dans laquelle B' représente un groupement -S- ou $-SO_2-$ et Cy et Ph ont la même signification que dans la revendication 1, avec une amine de formule générale :

H-Am (3)

dans laquelle Am a la même signification que dans la revendication 1 et ce, dans un solvant polaire ou dans un excès de ladite amine pour donner :

- le dérivé désiré, sous forme de base libre, dans lequel A représente un radical hydroxy-2 propylène,
- un dérivé aminoalkoxyphényle que l'on peut faire réagir avec un halogénure d'alkyle ayant de 1 à 4 atomes de carbone et en présence d'une base forte, ce qui fournit le dérivé désiré sous forme de base libre dans lequel A représente un radical hydroxy-2 propylène dans lequel l'hydroxy est substitué par un alkyle ayant de 1 à 4 atomes de carbone,et

C/ Lorsque B représente un groupement -SO, l'on traite avec un agent oxydant un sulfure de formule générale

Cy-S-Ph-O-A-Am (1)

dans laquelle Cy, Ph, A et Am ont la même signification que dans la revendication 1, ce sulfure étant sous forme de base libre ou de sel, pour former le dérivé désiré sous forme de base libre ou de sel que l'on traite avec un agent basique pour fournir le dérivé désiré sous forme de base libre, la base libre ainsi obtenue étant, si on le désire, mise en réaction avec un acide organique ou inorganique pour former un sel pharmaceutiquement acceptable de ce dérivé.

2. Procédé selon la revendication 1, caractérisé en ce que l'agent oxydant est le périodate de sodium, le permanganate de potassium ou l'acide chloro-3 perbenzoïque.

3. Procédé de préparation de dérivés aminoalkoxyphényle de formule (1) selon la revendication 1 dans laquelle $R_7$, $R_8$, $R_9$, $R_{10}$ ou $R_{11}$ représentent un groupement hydroxy, caractérisé en ce que l'on hydrogène, dans un solvant approprié et en présence d'un catalyseur un composé de formule (1) selon la revendication 1 dans laquelle $R_7$, $R_8$, $R_9$,$R_{10}$ ou $R_{11}$ représentent un groupement benzyloxy, pour obtenir le dérivé désiré que l'on peut, si nécessaire,faire réagir avec un acide organique ou inorganique approprié pour former un sel pharmaceutiquement acceptable.

4. Procédé de préparation de dérivés aminoalkoxyphényle de formule (1) selon la revendication 1 dans laquelle $R_7$, $R_8$, $R_9$, $R_{10}$ ou $R_{11}$ représentent un groupement amino, alkylamino inférieur ou dialkylamino inférieur, caractérisé en ce que l'on hydrogène dans un solvant approprié et en présence d'un catalyseur, un composé de formule (1) selon la revendication 1 dans laquelle $R_7$, $R_8$, $R_9$, $R_{10}$ ou $R_{11}$ représentent un groupement nitro, ce qui fournit :
   - le composé désiré sous forme de base libre dans laquelle $R_7$, $R_8$, $R_9$, $R_{10}$ ou $R_{11}$ représentent un groupement amino,
   - un dérivé aminoalkoxyphényle que l'on peut faire réagir avec une quantité appropriée d'un halogénure d'alkyle ayant de 1 à 4 atomes de carbone et en présence d'un agent alcalin, ce qui fournit le dérivé désiré sous forme de base libre, dans lequel $R_7$, $R_8$, $R_9$, $R_{10}$ ou $R_{11}$ représentent un groupement (alkyl en $C_1$-$C_4$)amino ou (dialkyl en $C_1$-$C_4$)amino,

   le dérivé aminoalkoxyphényle ainsi obtenu pouvant, si on le désire, être mis en réaction avec un acide organique ou inorganique pour former un sel pharmaceutiquement acceptable.

5. Procédé selon la revendication 1, caractérisé en ce que l'agent oxydant est le périodate de sodium, le permanganate de potassium ou l'acide chloro-3 perbenzoïque.

6. Procédé selon la revendication 1, caractérisé en ce que l'on prépare des dérivés aminoalkoxyphényle de formule (1) dans laquelle B représente un groupement $-SO_2-$

7. Procédé selon la revendication 1 caractérisé en ce que l'on prépare des dérivés aminoalkoxyphényle de formule (1) dans laquelle $R_1$ et $R_2$ représentent chacun l'hydrogène.

8. Procédé selon la revendication 1, caractérisé en ce que l'on prépare des dérivés aminoalkoxyphényle de formule (1) dans laquelle R représente le groupement isopropyle ou cyclopropyle.

9. Procédé selon la revendication 1 caractérisé en ce que l'on prépare des dérivés aminoalkoxyphényle de formule (1) dans laquelle $R_5$, $R'_5$ et $R''_5$ identiques ou différents, représentent chacun l'hydrogène, le chlore, le groupement méthyle ou le groupement méthoxy.

10. Procédé selon la revendication 1 caractérisé en ce que l'on prépare des dérivés aminoalkoxyphényle de formule (1) dans laquelle $R_3$ représente un radical -Alk-Ar.

11. Procédé selon la revendication 1 caractérisé en ce que l'on prépare des dérivés aminoalkoxyphényle de formule (1) dans laquelle la chaine $-O-A-NR_3-R_4$ représente un groupement [N-méthyl N-(diméthoxy-3,4β-phénéthyl)amino]propoxy ou [N-méthyl N-(diméthoxy-3,5β-phénéthylamino] propoxy.

12. Procédé selon la revendication 1 caractérisé en ce que l'on prépare des dérivés aminoalkoxyphényle de formule (1) dans laquelle Ph représente un groupement (D).

13. Procédé selon la revendication 1 caractérisé en ce que l'on prépare des dérivés aminoalkoxyphényle de formule (1) dans laquelle Cy représente un groupement (J).

14. Procédé selon la revendication 1, caractérisé en ce que l'on prépare des dérivés aminoalkoxyphényle de formule (1) dans laquelle Cy représente un groupement (K) ou (K').

15. Procédé selon la revendication 1 caractérisé en ce que l'on prépare des dérivés aminoalkoxyphényle de formule (1) dans laquelle Cy représente un groupement (L).

16. Procédé selon la revendication 1, caractérisé en ce que l'on prépare des dérivés aminoalkoxyphényle de formule (1) , le sel pharmaceutiquement acceptable étant l'oxalate, le chlorhydrate ou le fumarate.

17. Procédé selon la revendication 1, caractérisé en ce que l'on prépare des dérivés aminoalkoxyphényle de formule (1) choisis parmi les composés suivants :
   isopropyl-2 méthyl-8[{[N-méthyl N-(diméthoxy-3,5 β-phénéthyl) amino] -3 propoxy}-4 benzènesulfonyl}-1 indolizine
   isopropyl-2 méthyl-8[{[N-méthyl N-(diméthoxy-3,4β-phénéthyl) amino] -3 propoxy}-4 benzènesulfonyl] -1 indolizine
   {[(di-n-butylamino)-3 propoxy]-4 benzènesulfonyl} -1 isopropyl-2 méthyl-8 indolizine
   isopropyl-2 méthyl-8{[(diméthoxy-6,7 tétrahydro-1,2,3,4 isoquinoléinyl-2)-3 propoxy]-4 benzènesulfonyl}

-1 indolizine
isopropyl-2 méthyl-5[{N-méthyl N-(diméthoxy-3,4β-phénéthyl) amino]- 3 propoxy}-4 benzènesulfonyl]-1 indolizine
[{[N-méthyl N-(diméthoxy-3,4β-phénéthyl)amino] -3 propoxy}-4 benzenesulfony]-2 chloro-5 isopropyl-3 méthyl-1 indole et leurs sels pharmaceutiquement acceptables.

**18.** Procédé de préparation de compositions pharmaceutiques ou vétérinaires caractérisé en ce que l'on ajoute en tant que principe actif au moins un dérivé aminoalkoxyphényle préparé suivant l'une quelconque des revendications précédentes à un véhicule pharmaceutique ou un excipient approprié.

**19.** Procédé de préparation de compositions pharmaceutiques ou vétérinaires selon la revendication 18 contenant de 50 à 500 mg de principe actif pour le traitement de syndromes pathologiques du système cardiovasculaire.

**20.** Procédé de préparation de compositions pharmaceutiques ou vétérinaires selon la revendication 18 pour le traitement d'affections pathologiques o culaires.

**Revendications pour l'Etat contractant suivant : GR**

**1.** Dérivés aminoalkoxyphényle de formule générale :

Cy-B-Ph-O-A-Am (1)

ainsi que leurs sels pharmaceutiquement acceptables, dans laquelle :
B représente un groupement -S-, -SO- ou -$SO_2$,
Ph représente un radical de formule :

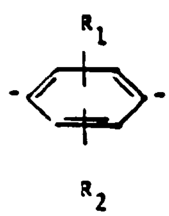

(D)

ou

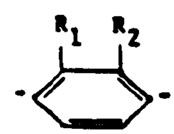

(E)

$R_1$ et $R_2$, qui sont identiques ou différents représentent chacun l'hydrogène, le radical méthyle ou éthyle ou un halogène.
A représente un radical alkylène, linéaire ou ramifié, ayant de 2 à 5 atomes de carbone ou le radical hydroxy-2 propylène dans lequel l'hydroxy est éventuellement substitué par un radical alkyle en $C_1$-$C_4$,
Am représente un groupement :

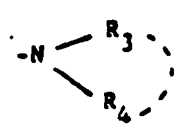

(F)

ou

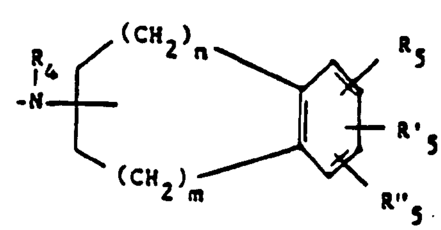

(G)

ou

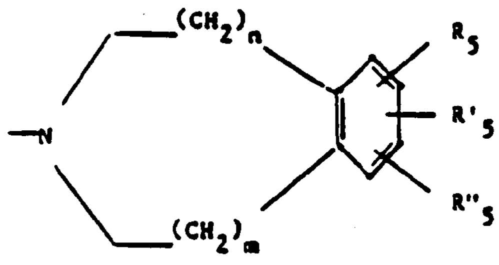

(H)

dans lequel :

$R_3$ représente un radical alkyle en $C_1$-$C_8$, cycloalkyle en $C_3$-$C_6$ ou un radical de formule :

-Alk-Ar

dans Laquelle Alk représente une liaison simple ou un radical alkylène, linéaire ou ramifié, ayant de 1 à 5 atomes de carbone et Ar représente un radical pyridyle, phényle, méthylènedioxy-2,3 phényle ou méthylènedioxy-3,4 phényle ou un groupement phényle substitué par un ou plusieurs substituants, identiques ou différents, choisis parmi des atomes d'halogène, des groupements alkyle en $C_1$-$C_4$ ou des groupements alkoxy en $C_1$-$C_4$,

$R_4$ représente l'hydrogène ou un radical alkyle en $C_1$-$C_8$, ou $R_3$ et $R_4$, lorsqu'ils sont pris ensemble, représentant un radical alkylène ou alkénylène ayant de 3 à 6 atomes de carbone et éventuellement substitué par un radical phényle ou éventuellement interrompu par -O-, -N= ou

$-\overset{|}{N}-R_6$.

$R_6$ représentant l'hydrogène, un radical alkyle en $C_1$-$C_4$, cycloalkyle, en $C_3$-$C_6$, phényle éventuellement substitué par un atome d'halogène ou par un groupement alkyle en $C_1$-$C_4$ ou alkoxy en $C_1$-$C_4$,

$R_5$, $R'_5$ et $R''_5$, qui sont identiques ou différents, représentent chacun l'hydrogène, un atome d'halogène, un groupement alkyle en $C_1$-$C_4$ ou alkoxy en $C_1$-$C_4$,

n et m, identiques ou différents représentent chacun 0, 1, 2 ou 3, Cy représente l'un des groupements de formule :

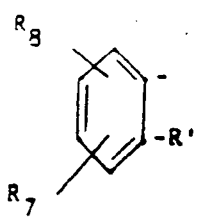

(I)

ou

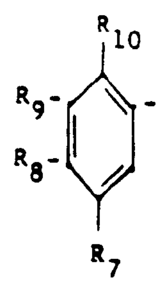

(I')

ou

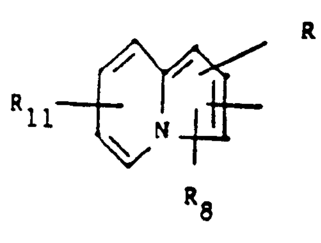

(J)

ou

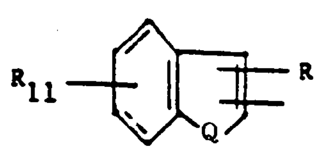

(K)

ou

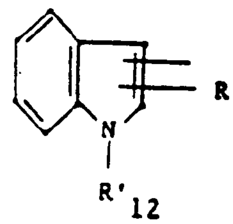

(K')

ou

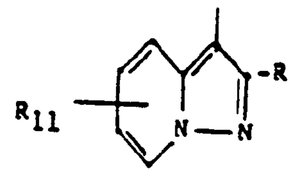

(L)

ou

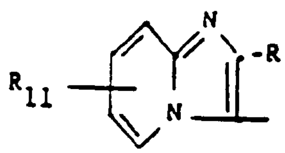

(M)

ou

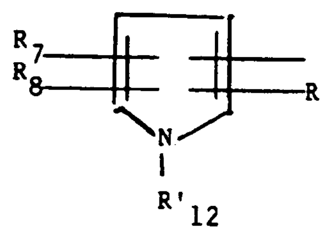

(N)

ou

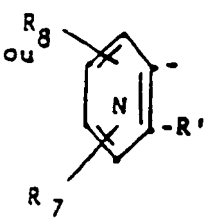

(Q)

ou

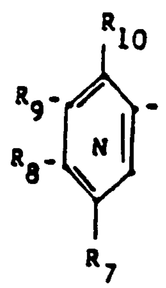

(Q')

dans lequel :

R représente l'hydrogène, un radical alkyle en $C_1$-$C_8$, cycloalkyle en $C_3$-$C_6$, benzyle ou phényle éventuellement substitué par un ou plusieurs substituants, qui, peuvent être identiques ou différents, choisis parmi des atomes d'halogène ou parmi des groupements alkyle en $C_1$-$C_4$, alkoxy en $C_1$-$C_4$ ou nitro,

R' représente un radical alkyle en $C_1$-$C_8$, cycloalkyle en $C_3$-$C_6$, benzyle ou phényle éventuellement substitué par un ou plusieurs substituants, qui peuvent être identiques ou différents choisis parmi des atomes d'halogène ou parmi des groupements alkyle en $C_1$-$C_4$, alkoxy en $C_1$-$C_4$ ou nitro,

$R_7$, $R_8$ et $R_9$, identiques ou différents, représentent chacun l'hydrogène, un radical alkyle en $C_1$-$C_4$, alkoxy en $C_1$-$C_4$, un atome d'halogène, un groupement hydroxy, benzyloxy, nitro, amino, (alkyl en $C_1$-$C_4$)amino, di(alkyl en $C_1$-$C_4$)amino, sulfonamido, (alkyl en $C_1$-$C_4$)sulfonamido, phénylsulfonamido, cyano, (alkoxy en $C_1$-$C_4$)carbonyle ou (alkyl en $C_1$-$C_4$)carbonyle,

$R_{10}$ représente l'hydrogène, un radical alkoxy en $C_1$-$C_4$, un atome d'halogène, un groupement hydroxy, benzyloxy, nitro, amino, (alkyl en $C_1$-$C_4$)amino, di(alkyl en $C_1$-$C_4$)amino, sulfonamido, (alkyl en $C_1$-$C_4$)sulfonamido, phénylsulfonamido, cyano, (alkoxy en $C_1$-$C_4$)carbonyle ou (alkyl en $C_1$-$C_4$)carbonyle,

les groupements $R_7$ et $R_8$ d'une part et $R_7$, $R_8$, $R_9$ et $R_{10}$ d'autre part ne représentant jamais simultanément l'hydrogène et le radical (I) ou (I') ne représentant pas un radical monohalogénophenyle.

$R_{11}$ représente un radical alkyle en $C_1$-$C_4$, un atome d'halogène, un groupement hydroxy, benzyloxy, nitro, amino, (alkyl en $C_1$-$C_4$)amino, di(alkyl en $C_1$-$C_4$)amino, sulfonamido, (alkyl en $C_1$-$C_4$)sulfonamido, phényl-

sulfonamido, cyano, (alkoxy en $C_1$-$C_4$)carbonyle ou (alkyl en $C_1$-$C_4$)carbonyle,
Q représente

$$-\overset{|}{N}-R_{12}$$

dans lequel $R_{12}$ représente l'hydrogène, un groupement alkyle en $C_1$-$C_4$, cycloalkyle en $C_3$-$C_6$, benzyle, halogénobenzyle, un groupement -A-Am tel que défini précédemment, un groupement (al- kyle en $C_1$-$C_4$) sulfonyle ou phénylsulfonyle éventuellement ment substitué par un ou plusieurs atomes d'halogènes, groupements alkyle en $C_1$-$C_4$ ou alkoxy en $C_1$-$C_4$,
$R'_{12}$ représente un groupement -A-Am tel que défini précédemment, un groupement alkylsulfonyle éventuelle- ment substitué par un ou plusieurs atomes d'halogène, groupements alkyle en $C_1$-$C_4$ ou alkoxy en $C_1$-$C_4$.

2. Dérivés aminoalkoxyphényle selon la revendication 1 dans laquelle 8 représente un groupement $-SO_2-$.

3. Dérivés aminoalkoxyphényle selon la re- vendication 1 ou 2, caractérisés en ce que $R_1$ et $R_2$ représentent chacun l'hdyrogène.

4. Dérivés aminoalkoxyphényle selon l'une quelconque des revendications 1 à 3 caractérisés en ce que R représente le groupement isopropyle ou cyclopropyle.

5. Dérivés aminoalkoxyphényle selon l'une quelconque des revendications 1 à 4, caractérisés en ce que $R_5$, $R'_5$ et $R''_5$, identiques ou différents, représentent chacun l'hydrogène, le chlore, le groupement méthyle ou le groupement méthoxy.

6. Dérivés aminoalkoxyphényle selon une des revendications 1 à 5 caractérisés en ce que $R_3$ représente un radical -Alk-Ar.

7. Dérivés aminoalkoxyphényle selon l'une quelconque des revendications 1 à 6, caractérisés en ce que la chaîne -0-A-$NR_3$-$R_4$ représente un groupement [N-méthyl N-(diméthoxy-3,4β-phénéthyl)amino]propoxy ou [N-méthyl N-(diméthoxy-3,5β-phénéthylamino] propoxy.

8. Dérivés aminoalkoxyphényle selon l'une quelconque des revendications 1 à 7, caractérisés en ce que Ph représente un groupement (D).

9. Dérivés aminoalkoxyphényle selon l'une quelconque des revendications 1 à 7, caractérisés en ce que Cy représente un groupement (J).

10. Dérivés aminoalkoxyphényle selon l'une quelconque des revendications 1 à 7, caractérisés en ce que Cy représente un groupement (K) ou (K').

11. Dérivés aminoalkoxyphényle selon l'une quelconque des revendications 1 à 7, caractérisés en ce que Cy représente un groupement (L).

12. Dérivés aminoalkoxyphényle selon l'une quelconque des revendications 1 à 11, caractérisés en ce que le sel pharmaceutiquement acceptable est l'oxalate, le chlorhydrate ou le fumarate.

13. Dérivés aminoalkoxyphényle selon la revendication 1 choisis parmi les composés suivants :
isopropyl-2 méthyl-8[{[N-méthyl N-(diméthoxy-3,5β-phénéthyl)amino]-3 propoxy}-4 benzénesulfonyl]-1 indolizine
isopropyl-2 méthyl-8[{[N-méthyl N-(diméthoxy-3,4β-phénéthyl)amino]-3 propoxy}-4 benzénesulfonyl]-1 indolizine
{[(di-n-butylamino)-3 propoxy]-4 benzènesulfonyl}-1 isopropyl-2 méthyl-8 indolizine
isopropyl-2 méthyl-8{[(diméthoxy-6,7 tétrahydro-1,2,3,4 isoquinoléinyl-2)-3 propoxy]-4 benzènesulfonyl}-1 indolizine
isopropyl-2 méthyl-5[{[N-méthyl N-(diméthoxy-3,4β-phénéthyl)amino]-3 propoxy}-4 benzènesulfonyl]-1 indolizine
[{[N-méthyl N-(diméthoxy-3,4β-phénéthyl)amino]-3 propoxy}-4 benzènesulfonyl]-2 chloro-5 isopropyl-3 méthyl-1 indole
et leurs sels pharmaceutiquement acceptables.

**14.** Procédé de préparation de dérivés aminoalkoxyphényle de formule (1) selon la revendication 1 dans laquelle A représente un radical alkylène et B représente un groupement -S- ou -$SO_2$-, caractérisé en ce que l'on condense, en présence d'un accepteur d'acide, dans un solvant polaire ou non polaire et à une température comprise entre la température ambiante et la température de reflux, un dérivé alkoxy-4 phényle de formule générale :

Cy-B'-Ph-O-A-X (2)

dans laquelle B' représente un groupement -S- ou -$SO_2$-, Cy et Ph ont la même signification que dans la revendication 1, A a la même signification que précédemment et X représente un atome d'halogéne ou un groupement alkylsulfonyloxy ayant de 1 à 4 atomes de carbone ou arylsulfonyloxy ayant de 6 à 10 atomes de carbone, avec une amine de formule générale :

H-Am (3)

dans laquelle Am a la même signification que dans la revendication 1, pour former le dérivé désiré que l'on peut faire réagir, si nécessaire, avec un acide organique ou inorganique pour former un sel pharmaceutiquement acceptable de ce dérivé.

**15.** Procédé de préparation de dérivés aminoalkoxyphényle de formule (1) selon la revendication 1 dans laquelle B représente un groupement -S- ou -$SO_2$-. caractérisé en ce que l'on fait réagir, en présence d'un agent basique. un dérivé hydroxy-4 phényle de formule générale :

Cy-B'-Ph-OH (4)

dans laquelle B' représente un groupement -S- ou -$SO_2$- et Cy et Ph ont la même signification que dans la revendication 1, avec un composé de formule générale :

X-A-Am (31)

dans laquelle X représente un atome d'halogène ou un groupement alkylsulfoxy ayant de 1 à 4 atomes de carbone ou arylsulfoxy ayant de 6 à 10 atomes de carbone. A représente un radical alkylène et Am a la même valeur que dans la revendication 1, la réaction ayant lieu au reflux et dans un solvant approprié pour obtenir le dérivé désiré que l'on peut, si on le désire, faire réagir avec un acide organique ou inorganique approprié. pour former un sel pharmaceutiquement acceptable.

**16.** Procédé de préparation de dérivés amino- alkoxyphényle de formule (1) selon la revendication 1 dans laquelle A repré- sente le radical hydroxy-2 propyléne éventuellement substitué et 8 représente un groupement -S- ou -$SO_2$-. caractérisé en ce que l'on traite au reflux un dérivé oxyranylméthoxy de formule générale :

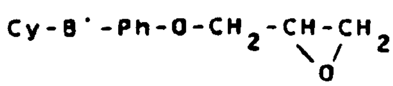

(33)

dans laquelle B' représente un groupement -S- ou -$SO_2$- et Cy et Ph ont la même signification que dans la revendication 1, avec une amine de formule générale :

H-Am (3)

dans laquelle Am a la même signification que dans la revendication 1 et ce, dans un solvant polaire ou dans un excès de ladite amine pour donner :

- le dérivé désiré, sous forme de base libre, dans lequel A représente un radical hydroxy-2 propylène,
- un dérivé aminoalkoxyphényle que l'on peut faire réagir avec un halogénure d'alkyl ayant de 1 à 4 atomes de carbone et en présence d'une base forte, ce qui fournit le dérivé dérisé sous forme de base libre, dans lequel A représente un radical hydroxy-2 propylène dans lequel l'hydroxy est substitué par un alkyle ayant de 1 à 4 atomes de carbone,

le dérivé aminoalkoxyphényle ainsi obtenu pouvant, si on le désire, être mis en réaction avec un acide organique ou inorganique pour former un sel pharmaceutiquement acceptable.

**17.** Procédé de préparation de dérivés aminoalkoxyphényle de formule (1) selon la revendication 1 dans laquelle B représente un groupement -SO-, caractérisé en ce que l'on traite, avec un agent oxydant, un sulfure de formule générale :

Cy-S-Ph-O-A-Am (1)

dans laquelle Cy, Ph, A et Am ont la même signification que dans la revendication 1, ce sulfure étant sous forme de base libre ou de sel, pour former le dérivé désiré sous forme de base libre ou de sel que l'on traite avec un agent basique pour fournir le dérivé désiré sous forme de base libre, la base libre ainsi obtenue étant, si on le désire, mise en réaction avec un acide organique ou inorganique pour former un sel pharmaceutiquement acceptable de ce dérivé.

**18.** Procédé selon la revendication 17, caractérisé en ce que l'agent oxydant est le périodate de sodium, le permangante de potassium ou l'acide chloro-3 perbenzoïque.

**19.** Procédé de préparation de dérivés aminoalkoxyphényle de formule (1) selon la revendication 1 dans laquelle, $R_7$, $R_8$, $R_9$, $R_{10}$ ou $R_{11}$ représentent un groupement hydroxy, caractérisé en ce que l'on hydrogène, dans un solvant approprié et en présence d'un catalyseur un composé de formule (1) selon la revendication 1 dans laquelle $R_7$, $R_8$, $R_9$, $R_{10}$ ou $R_{11}$ représentent un groupement benzyloxy, pour obtenir le dérivé désiré que l'on peut, si nécessaire faire réagir avec un acide organique ou inorganique approprié pour former un sel pharmaceutiquement acceptable.

**20.** Procédé de préparation de dérivés aminoalkoxyphényle de formule (1) selon la revendication 1 dans laquelle $R_7$, $R_8$, $R_9$, $R_{10}$ ou $R_{11}$ représentent un groupement amino, alkylamino inférieur ou dialkylamino inférieur, caractérisé en ce que l'on hydrogène dans un solvant approprié et en présence d'un catalyseur, un composé de formule (1) selon la revendication 1 dans laquelle $R_7$, $R_8$, $R_9$, $R_{10}$ ou $R_{11}$ représentent un groupement nitro, ce qui fournit :

- le composé désiré sous forme de base libre dans laquelle $R_7$, $R_8$, $R_9$, $R_{10}$ ou $R_{11}$ représentent un groupement amino
- un dérivé aminoalkoxyphényle que l'on peut faire réagir avec une quantité appropriée d'un halogénure d'alkyle ayant de 1 à 4 atomes de carbone et en présence d'un agent alcalin, ce qui fournit le dérivé désiré sous forme de base libre, dans lequel $R_7$, $R_8$, $R_9$, $R_{10}$ ou $R_{11}$ représentent un groupement (alkyl en $C_1$-$C_4$)amino ou (dialkyl en $C_1$-$C_4$)amino, le dérivé aminoalkoxyphényle ainsi obtenu pouvant, si on le désire, être mis en réaction avec un acide organique ou inorganique pour former un sel pharmaceutiquement acceptable.

**21.** Procédé de préparation de compositions pharmaceutiques ou vétérinaires caractérisé en ce que l'on additionne au moins un dérivé aminoalkoxyphényle selon l'une quelconque des revendications 1 à 13, avec un véhicule pharmaceutique ou un excipient approprié.

**22.** Procédé de préparation de compositions pharmaceutiques ou vétérinaires selon la revendication 21 pour le traitement de syndromes pathologiques du système cardiovasculaire contenant de 50 mg à 500 mg de principe actif.

**23.** Procédé de préparation de compositions pharmaceutiques ou vétérinaires selon la revendication 21 destinées au traitement d'affections pathologiques oculaires.

## Claims

### Claims for the following Contracting States : CH, DE, DK, FR GB, IT, LI, LU, NL, SE, AT

**1.** Aminoalkoxyphenyl derivatives of general formula:

Cy-B-Ph-O-A-Am (1)

as well as the pharmaceutically acceptable salts thereof, in which:
B represents a -S-, -SO- or $-SO_2$ group,
Ph represents a radical of formula:

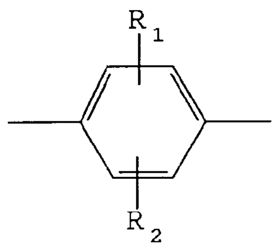

or

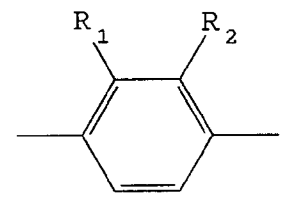

(D) (E)

$R_1$ and $R_2$, which are identical or different, each represents hydrogen, a methyl or ethyl radical or halogen,
A represents a straight-chained or branched alkylene radical having from 2 to 5 carbon atoms or a 2-hydroxypropylene radical in which the hydroxy is optionally substituted by a $C_1$-$C_4$ alkyl radical,
Am represents a group:

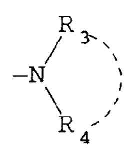

or

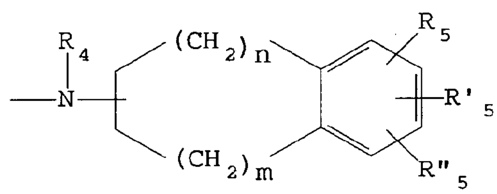

(F) (G)

or

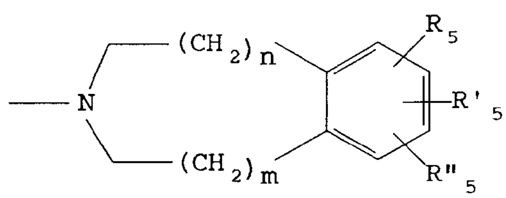

(H)

in which
$R_3$ represents a $C_1$-$C_8$ alkyl or a $C_3$-$C_6$ cycloalkyl radical, or a radical of formula:

-Alk-Ar

in which Alk represents a single bond or a straight-chained or branched alkylene radical having from 1 to 5 carbon atoms and Ar represents a pyridyl, phenyl, 2,3-methylenedioxyphenyl or 3,4-methylenedioxyphenyl radical or a phenyl group substituted by one or more identical or different substituents selected from halogen atoms, $C_1$-$C_4$ alkyl groups or $C_1$-$C_4$ alkoxy groups,
$R_4$ represents hydrogen or a $C_1$-$C_8$ alkyl radical, or $R_3$ and $R_4$, when they are taken together, represent an alkylene or alkenylene radical having from 3 to 6 carbon atoms and optionally substituted by a phenyl radical or optionally interrupted by -O-, -N= or

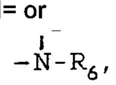,

$R_6$ representing hydrogen, a $C_1$-$C_4$ alkyl radical, a $C_3$-$C_6$ cycloalkyl radical, a phenyl radical optionally substituted by a halogen atom or by a $C_1$-$C_4$ alkyl group or a $C_1$-$C_4$ alkoxy group,
$R_5$, $R'_5$ and $R''_5$, which are identical or different, each represents hydrogen, a halogen atom, a $C_1$-$C_4$ alkyl group or a $C_1$-$C_4$ alkoxy group,
n and m, identical or different, each denotes 0, 1, 2, 3, Cy represents one of the groups of formula:

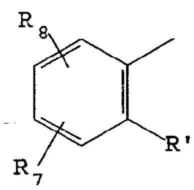

(I)

or

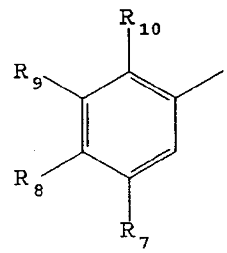

(I')

or

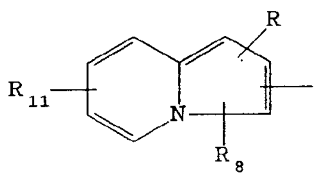

(J)

or

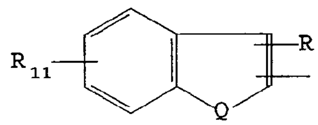

(K)

or

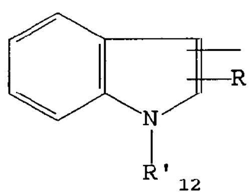

(K')

or

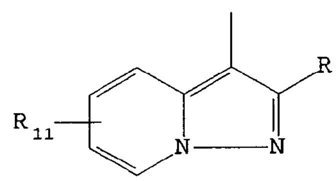

(L)

or

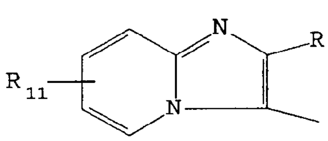

(M)

or

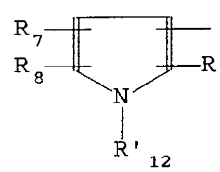

(N)

or

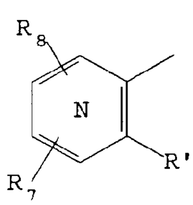

(Q)

or

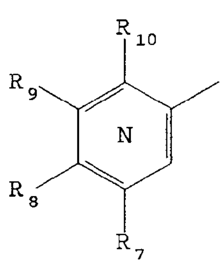

(Q')

in which:

R represents hydrogen, a $C_1$-$C_8$ alkyl radical, a $C_3$-$C_6$ cycloalkyl radical, a benzyl radical or a phenyl radical optionally substituted by one or more substituents which may be identical or different, selected from ha-

logen atoms or from $C_1$-$C_4$ alkyl, $C_1$-$C_4$ alkoxy or nitro groups,
R' represents a $C_1$-$C_8$ alkyl radical, a $C_3$-$C_6$ cycloalkyl radical, a benzyl radical or a phenyl radical optionally substituted by one or more substituents which may be identical or different, selected from halogen atoms or from $C_1$-$C_4$ alkyl, $C_1$-$C_4$ alkoxy or nitro groups,
$R_7$, $R_8$ and $R_9$, identical or different, each represents hydrogen, a $C_1$-$C_4$ alkyl radical, a $C_1$-$C_4$ alkoxy radical, a halogen atom, a hydroxy, benzyloxy, nitro, amino, ($C_1$-$C_4$ alkyl)-amino, di($C_1$-$C_4$ alkyl)-amino, sulfonamido, ($C_1$-$C_4$ alkyl)-sulfonamido, phenylsulfonamido, cyano, ($C_1$-$C_4$ alkoxy)-carbonyl group or ($C_1$-$C_4$ alkyl)-carbonyl group, $R_{10}$ denotes hydrogen, a $C_1$-$C_4$ alkoxy radical, a halogen atom, a hydroxy, benzyloxy, nitro, amino, ($C_1$-$C_4$ alkyl)amino, di($C_1$-$C_4$ alkyl)amino, sulfonamido, ($C_1$-$C_4$ alkyl)-sulfonamido, phenylsulfonamido, cyano, ($C_1$-$C_4$ alkoxy)carbonyl or ($C_1$-$C_4$ alkyl)carbonyl group,
the groups $R_7$ and $R_8$, on the one hand, and $R_7$, $R_8$, $R_9$ and $R_{10}$, on the other, never simultaneously representing hydrogen and the radical (I) or (I') not representing a monohalogenophenyl radical;
$R_{11}$ represents a $C_1$-$C_4$ alkyl radical, a halogen atom, a hydroxy, benzyloxy, nitro, amino, ($C_1$-$C_4$ alkyl)amino, di($C_1$-$C_4$ alkyl)amino, sulfonamido, ($C_1$-$C_4$ alkyl)sulfonamido, phenylsulfonamido, cyano, ($C_1$-$C_4$ alkoxy)carbonyl or ($C_1$-$C_4$ alkyl)carbonyl group,
Q represents -N-$R_{12}$ in which $R_{12}$ represents hydrogen, a $C_1$-$C_4$ alkyl group, a $C_3$-$C_6$ cycloalkyl group, benzyl, halogenobenzyl, an -A-Am group as defined above, a ($C_1$-$C_4$ alkyl)sulfonyl group or a phenylsulfonyl group optionally substituted by one or more halogen atoms or $C_1$-$C_4$ alkyl or $C_1$-$C_4$ alkoxy groups,
$R'_{12}$ represents an -A-Am group as defined above, an alkylsulfonyl group optionally substituted by one or more halogen atoms, $C_1$-$C_4$ alkyl or $C_1$-$C_4$ alkoxy groups.

2. Aminoalkoxyphenyl derivatives according to Claim 1, in which B represents an -$SO_2$- group.

3. Aminoalkoxyphenyl derivatives according to Claim 1 or 2, characterised in that $R_1$ and $R_2$ each represents hydrogen.

4. Aminoalkoxyphenyl derivatives according to any one of Claims 1 to 3, characterised in that R represents an isopropyl or a cyclopropyl group.

5. Aminoalkoxyphenyl derivatives according to any one of Claims 1 to 4, characterised in that $R_8$, $R'_5$ and $R''_5$, identical or different, each represents hydrogen, chlorine, or a methyl or methoxy group.

6. Aminoalkoxyphenyl derivatives according to any one of Claims 1 to 5, characterised in that $R_3$ represents an -Alk-Ar radical.

7. Aminoalkoxyphenyl derivatives according to any one of Claims 1 to 6, characterised in that the -O-A-$NR_3$-$R_4$ chain represents an
(N-methyl-N-3,4-dimethoxy-β-phenethylamino)-propoxy or (N-methyl-N-3,5-dimethoxy-β-phenethylamino)-propoxy group.

8. Aminoalkoxyphenyl derivatives according to any one of Claims 1 to 7, characterised in that Ph represents a (D) group.

9. Aminoalkoxyphenyl derivatives according to any one of Claims 1 to 7, characterised in that Cy represents a (J) group.

10. Aminoalkoxyphenyl derivatives according to any one of Claims 1 to 7, characterised in that Cy represents a (K) or (K') group.

11. Aminoalkoxyphenyl derivatives according to any one of Claims 1 to 7, characterised in that Cy represents an (L) group.

12. Aminoalkoxyphenyl derivatives according to any one of Claims 1 to 11, characterised in that the pharmaceutically acceptable salt is the oxalate, hydrochloride or fumarate.

13. Aminoalkoxyphenyl derivatives according to Claim 1 selected from the following compounds:
2-isopropyl-8-methyl-1-{4-[3-(N-methyl-N-3,5-dimethoxy-β-phenethyl-amino)-propoxy] benzenesulfonyl} indolizine;
2-isopropyl-8-methyl-1-{4-[3-(N-methyl N-3,4-dimethoxy-β-phenethyl-amino)-propoxy] benzenesulfonyl} indolizine;

1-{4-[3-(di-n-butylamino)-propoxy] benzenesulfonyl}-2-isopropyl-8-methyl-indolizine;
2-isopropyl-8-methyl-1-{4-[3-(6,7-dimethoxy-1,2,3,4-tetrahydroisoquinolin-2-yl)-propoxy] benzenesulfonyl} indolizine;
2-isopropyl-5-methyl-1-{4-[3-(N-methyl-N-3,4-dimethoxy-β-phenethyl-amino)-propoxy] benzenesulfonyl} indolizine;
2-{4-[3-(N-methyl-N-3,4-dimethoxy-β-phenethylamino)-propoxy] benzenesulfonyl}-5-chloro-3-isopropyl-1-methyl indole
and the pharmaceutically acceptable salts thereof.

**14.** Process for the preparation of aminoalkoxyphenyl derivatives of formula (1) according to Claim 1 in which A denotes an alkylene radical and B denotes a -S- or $-SO_2-$ group, characterised in that, in the presence of an acid acceptor in a polar or non-polar solvent and at a temperature between room temperature and reflux temperature, a 4-alkoxyphenyl derivative of general formula:

Cy-B'-Ph-O-A-X (2)

in which B' represents an -S- or $-SO_2-$ group, Cy and Ph have the same meaning as in Claim 1, A has the same meaning as above and X represents a halogen atom or an alkylsulfonyloxy group having from 1 to 4 carbon atoms or an arylsulfonyloxy group having from 6 to 10 carbon atoms, is condensed with an amine of general formula:

H-Am (3)

in which Am has the same meaning as in Claim 1, to form the desired derivative which can be reacted, if necessary, with an organic or inorganic acid to form a pharmaceutically acceptable salt of this derivative.

**15.** Process for the preparation of aminoalkoxyphenyl derivatives of formula (1) according to Claim 1 in which B represents an -S- or $-SO_2-$ group, characterised in that, in the presence of a basic agent, a 4-hydroxyphenyl derivative of general formula:

Cy-B'-Ph-OH (4)

in which B' represents an -S- or $-SO_2-$ group and Cy and Ph have the same meaning as in Claim 1, is reacted with a compound of general formula:

X-A-Am (31)

in which X represents a halogen atom or an alkylsulfoxy group having from 1 to 4 carbon atoms or an arylsulfoxy group having from 6 to 10 carbon atoms, A represents an alkylene radical and Am has the same meaning as in Claim 1, the reaction being carried out by refluxing in a suitable solvent to give the desired derivative which can be reacted, if desired, with a suitable organic or inorganic acid to form a pharmaceutically acceptable salt.

**16.** Process for the preparation of aminoalkoxyphenyl derivatives of formula (1) according to claim 1, in which A represents an optionally substituted 2-hydroxypropylene radical and B represents an -S- or $-SO_2-$ group, characterised in that an oxiranylmethoxy derivative of general formula:

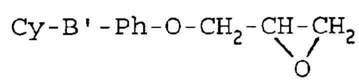 (33)

in which B' represents an -S- or $-SO_2-$ group and Cy and Ph have the same meaning as in Claim 1, is refluxed with an amine of general formula:

H-Am (3)

in which Am has the same meaning as in Claim 1, in a polar solvent or in an excess of the said amine to give:

- the required derivative, in the form of the free base, in which A represents a 2-hydroxypropylene radical,
- an aminoalkoxyphenyl derivative which can be reacted with an alkyl halide having from 1 to 4 carbon atoms and in the presence of a strong base, to give the required derivative in the form of the free base, in which A represents a 2-hydroxypropylene radical in which the hydroxy is substituted by a $C_1$-$C_4$ alkyl group whilst the aminoalkoxyphenyl derivative thus obtained can, if desired, be reacted with an organic or inorganic acid to form a pharmaceutically acceptable salt.

**17.** Process for the preparation of aminoalkoxyphenyl derivatives of formula (1) according to Claim 1 in which B represents a -SO- group, characterised in that a sulfide of general formula:

Cy-S-Ph-O-A-Am (1)

in which Cy, Ph, A and Am have the same meaning as in Claim 1 is treated with an oxidising agent, said sulfide being in the form of the free base or a salt, to form the required derivative in the form of the free base or salt, which is treated with a basic agent to give the required derivative in the form of the free base, the free base thus obtained being, if desired, reacted with an organic or inorganic acid to form a pharmaceutically acceptable salt of this derivative.

**18.** Process according to Claim 17, characterised in that the oxidizing agent is sodium periodate, potassium permanganate or 3-chloro-perbenzoic acid.

**19.** Process for the preparation of aminoalkoxyphenyl derivatives of formula (1) according to Claim 1, in which $R_7$, $R_8$, $R_9$, $R_{10}$ or $R_{11}$ represent a hydroxy group, characterised in that a compound of formula (1) according to Claim 1, in which $R_7$, $R_8$, $R_9$, $R_{10}$ or $R_{11}$ represent a benzyloxy group, is hydrogenated in a suitable solvent and in the presence of a catalyst to give the required derivative which can, if necessary, be reacted with a suitable organic or inorganic acid to form a pharmaceutically acceptable salt.

**20.** Process for the preparation of aminoalkoxyphenyl derivatives of formula (1) according to Claim 1, in which $R_7$, $R_8$, $R_9$, $R_{10}$ or $R_{11}$ represent an amino, a lower alkylamino or lower dialkylamino group, characterised in that a compound of formula (1) according to Claim 1, in which $R_7$, $R_8$, $R_9$, $R_{10}$ or $R_{11}$ represent a nitro group, is hydrogenated in a suitable solvent and in the presence of a catalyst to give:

- the desired compound in the form of the free base in which $R_7$, $R_8$, $R_9$, $R_{10}$ or $R_{11}$ represent an amino group,
- an aminoalkoxyphenyl derivative which can be reacted with an appropriate quantity of an alkyl halide having from 1 to 4 carbon atoms in the presence of an alkaline agent to give the desired derivative in the form of the free base, in which $R_7$, $R_8$, $R_9$, $R_{10}$ or $R_{11}$ represent a ($C_1$-$C_4$ alkyl)amino or ($C_1$-$C_4$ dialkyl)amino group,

whilst the aminoalkoxyphenyl derivative thus obtained can, if desired, be reacted with an organic or inorganic acid to form a pharmaceutically acceptable salt.

**21.** Pharmaceutical or veterinary compositions containing, as active principle, at least one aminoalkoxyphenyl derivative according to any one of Claims 1 to 13 in combination with a suitable pharmaceutical carrier or excipient.

**22.** Pharmaceutical or veterinary compositions according to claim 21, for treating pathological syndromes of the cardiovascular system, containing 50 mg to 500 mg of active principle.

**23.** The use of at least one aminoalkoxyphenyl derivative according to any one of Claims 1 to 13 for the production of a medicament for treating pathological syndromes of the cardiovascular system.

**24.** The use of at least one aminoalkoxyphenyl derivative according to any one of Claims 1 to 13 for the production of a medicament for treating ocular diseases.

**Claims for the following Contracting State : ES**

**1.** Process for preparing aminoalkoxyphenyl derivatives of general formula:

Cy-B-Ph-O-A-Am (1)

as well as the pharmaceutically acceptable salts thereof, in which B represents a -S-, -SO- or -$SO_2$ group, Ph represents a radical of formula:

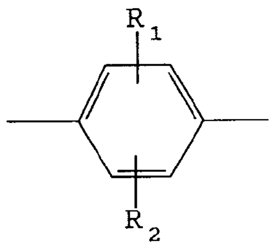

or

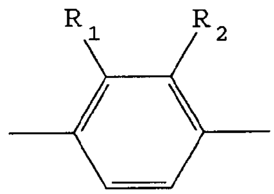

(D) (E)

$R_1$ and $R_2$, which are identical or different, each represents hydrogen, a methyl or ethyl radical or halogen,
A represents a straight-chained or branched alkylene radical having from 2 to 5 carbon atoms or a 2-hydroxypropylene radical in which the hydroxy is optionally substituted by a $C_1$-$C_4$ alkyl radical,
Am represents a group:

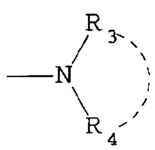

or

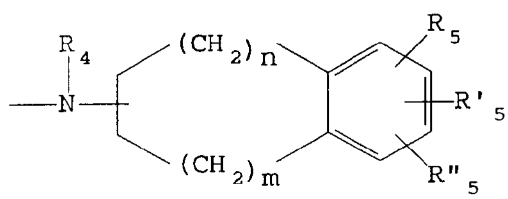

(F) (G)

or

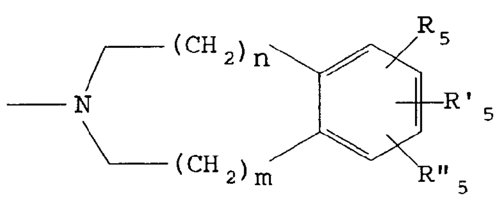

(H)

in which
$R_3$ represents a $C_1$-$C_8$ alkyl or a $C_3$-$C_6$ cycloalkyl radical, or a radical of formula:

-Alk-Ar

in which Alk represents a single bond or a straight-chained or branched alkylene radical having from 1 to 5 carbon atoms and Ar represents a pyridyl, phenyl, 2,3-methylenedioxyphenyl or 3,4-methylenedioxyphenyl radical or a phenyl group substituted by one or more identical or different substituents selected from halogen atoms,
$C_1$-$C_1$ alkyl groups or $C_1$-$C_4$ alkoxy groups,
$R_4$ represents hydrogen or a $C_1$-$C_8$ alkyl radical, or $R_3$ and $R_4$, when they are taken together, represent an alkylene or alkenylene radical having from 3 to 6 carbon atoms and optionally substituted by a phenyl radical or optionally interrupted by -O-, -N= or

$$-\overset{|}{N}-R_6,$$

$R_6$ representing hydrogen, a $C_1$-$C_4$ alkyl radical, a $C_3$-$C_6$ cycloalkyl radical, a phenyl radical optionally substituted by a halogen atom or by a $C_1$-$C_4$ alkyl group or a $C_1$-$C_4$ alkoxy group,
$R_5$, $R'_5$ and $R''_5$, which are identical or different, each represents hydrogen, a halogen atom, a $C_1$-$C_4$ alkyl group or a $C_1$-$C_4$ alkoxy group,
n and m, identical or different, each denotes 0, 1, 2, 3, Cy represents one of the groups of formula:

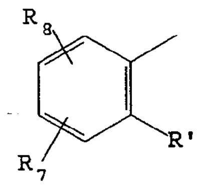

(I)

or

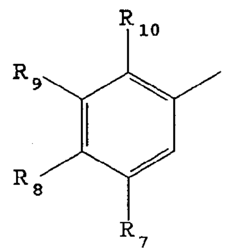

(I')

or

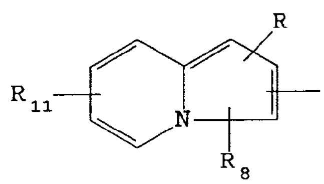

(J)

or

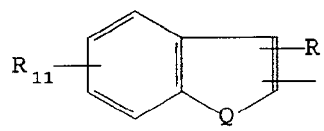

(K)

or

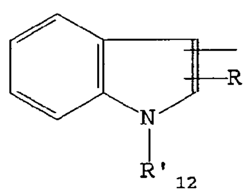

(K')

or

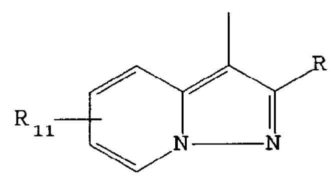

(L)

or

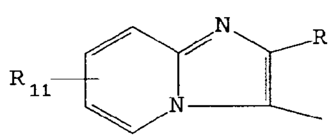

(M)

or

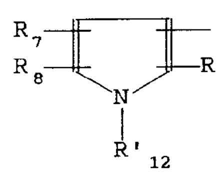

(N)

or

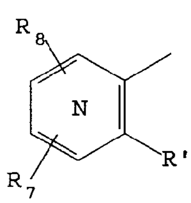

(Q)

or

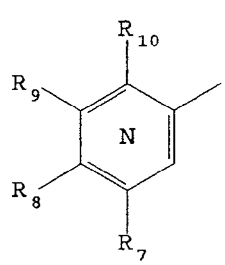

(Q')

in which:

R represents hydrogen, a $C_1$-$C_8$ alkyl radical, a $C_3$-$C_6$ cycloalkyl radical, a benzyl radical or a phenyl radical

optionally substituted by one or more substituents which may be identical or different, selected from halogen atoms or from $C_1$-$C_4$ alkyl, $C_1$-$C_4$ alkoxy or nitro groups,
R' represents a $C_1$-$C_8$ alkyl radical, a $C_3$-$C_6$ cycloalkyl radical, a benzyl radical or a phenyl radical optionally substituted by one or more substituents which may be identical or different, selected from halogen atoms or from $C_1$-$C_4$ alkyl, $C_1$-$C_4$ alkoxy or nitro groups,
$R_7$, $R_8$ and $R_9$, identical or different, each represents hydrogen, a $C_1$-$C_4$ alkyl radical, a $C_1$-$C_4$ alkoxy radical, a halogen atom, a hydroxy, benzyloxy, nitro, amino, ($C_1$-$C_4$ alkyl)-amino, di($C_1$-$C_4$ alkyl)-amino, sulfonamido, ($C_1$-$C_4$ alkyl)-sulfonamido, phenylsulfonamido, cyano, ($C_1$-$C_4$ alkoxy)-carbonyl group or ($C_1$-$C_4$ alkyl)-carbonyl group,
$R_{10}$ denotes hydrogen, a $C_1$-$C_4$ alkoxy radical, a halogen atom, a hydroxy, benzyloxy, nitro, amino, ($C_1$-$C_4$ alkyl)amino, di($C_1$-$C_4$ alkyl)amino, sulfonamido, ($C_1$-$C_4$ alkyl)-sulfonamido, phenylsulfonamido, cyano, ($C_1$-$C_4$ alkoxy)carbonyl or ($C_1$-$C_4$ alkyl)carbonyl group,
the groups $R_7$ and $R_8$, on the one hand, and $R_7$, $R_8$, $R_9$ and $R_{10}$, on the other, never simultaneously representing hydrogen and the radical (I) or (I') not representing a monohalogenophenyl radical;
$R_{11}$ represents a $C_1$-$C_4$ alkyl radical, a halogen atom, a hydroxy, benzyloxy, nitro, amino, ($C_1$-$C_4$ alkyl)amino, di($C_1$-$C_4$ alkyl)amino, sulfonamido, ($C_1$-$C_4$ alkyl)sulfonamido, phenylsulfonamido, cyano, ($C_1$-$C_4$ alkoxy)carbonyl or ($C_1$-$C_4$ alkyl)carbonyl group,
Q represents -N-$R_{12}$ in which $R_{12}$ represents hydrogen, a $C_1$-$C_4$ alkyl group, a $C_3$-$C_6$ cycloalkyl group, benzyl, halogenobenzyl, an -A-Am group as defined above, a ($C_1$-$C_4$ alkyl)sulfonyl group or a phenylsulfonyl group optionally substituted by one or more halogen atoms or $C_1$-$C_4$ alkyl or $C_1$-$C_4$ alkoxy groups,
$R'_{12}$ represents an -A-Am group as defined above, an alkylsulfonyl group optionally substituted by one or more halogen atoms, $C_1$-$C_4$ alkyl or $C_1$-$C_4$ alkoxy groups, characterised in that:

A/ When A represents an alkylene radical and B represents an -S- or $SO_2$-group, either in the presence of an acid acceptor in a polar or non-polar solvent and at a temperature between room temperature and reflux temperature, a 4-alkoxyphenyl derivative of general formula

Cy-B'-Ph-O-A-X (2)

in which B' represents an -S- or -$SO_2$- group, Cy and Ph have the same meaning as in Claim 1, A has the same meaning as above and X represents a halogen atom or an alkylsulfonyloxy group having from 1 to 4 carbon atoms or an arylsulfonyloxy group having from 6 to 10 carbon atoms, is condensed with an amine of general formula:

H-Am (3)

in which Am has the same meaning as in Claim 1, to form the desired derivative,
or, in the presence of a basic agent, a 4-hydroxyphenyl derivative of general formula:

Cy-B'-Ph-OH (4)

in which B' represents an -S- or -$SO_2$- group and Cy and Ph have the same meaning as in Claim 1, is reacted with a compound of general formula:

X-A-Am (31)

in which X denotes a halogen atom or an alkylsulfoxy group having from 1 to 4 carbon atoms or an arylsulfoxy group having from 6 to 10 carbon atoms, A represents an alkylene radical and Am has the same meaning as in Claim 1, the reaction being carried out by refluxing in a suitable solvent
B/ When A represents an optionally substituted 2-hydroxypropylene radical and B represents an -S- or -$SO_2$- group, an oxiranylmethoxy derivative of general formula:

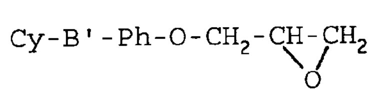 (33)

in which B' represents an -S- or -$SO_2$- group and Cy and Ph have the same meaning as in Claim 1, is refluxed with an amine of general formula:

H-Am (3)

in which Am has the same meaning as in Claim 1, in a polar solvent or in an excess of the said amine to give:

- the required derivative, in the form of the free base, in which A represents a 2-hydroxypropylene radical,
- an aminoalkoxyphenyl derivative which can be reacted with an alkyl halide having from 1 to 4 carbon atoms and in the presence of a strong base, to give the required derivative in the form of the free base, in which A represents a 2-hydroxypropylene radical in which the hydroxy is substituted by a $C_1$-$C_4$ alkyl group and

C/ When B represents an -SO- group, a sulfide of general formula:

Cy-S-Ph-O-A-Am (1)

in which Cy, Ph, A and Am have the same meaning as in Claim 1 is treated with an oxidising agent, said sulfide being in the form of the free base or a salt, to form the required derivative in the form of the free base or salt, which is treated with a basic agent to give the required derivative in the form of the free base, the free base thus obtained being, if desired, reacted with an organic or inorganic acid to form a pharmaceutically acceptable salt of this derivative.

2. Process according to Claim 1 , characterised in that the oxidizing agent is sodium periodate, potassium permanganate or 3-chloro-perbenzoic acid.

3. Process for the preparation of aminoalkoxyphenyl derivatives of formula (1) according to Claim 1, in which $R_7$, $R_8$, $R_9$, $R_{10}$ or $R_{11}$ represent a hydroxy group, characterised in that a compound of formula (1) according to Claim 1, in which $R_7$, $R_8$, $R_9$, $R_{10}$ or $R_{11}$ represent a benzyloxy group, is hydrogenated in a suitable solvent and in the presence of a catalyst to give the required derivative which can, if necessary, be reacted with a suitable organic or inorganic acid to form a pharmaceutically acceptable salt.

4. Process for the preparation of aminoalkoxyphenyl derivatives of formula (1) according to Claim 1, in which $R_7$, $R_8$, $R_9$, $R_{10}$ or $R_{11}$ represent an amino, a lower alkylamino or lower dialkylamino group, characterised in that a compound of formula (1) according to Claim 1, in which $R_7$, $R_8$, $R_9$, $R_{10}$ or $R_{11}$ represent a nitro group, is hydrogenated in a suitable solvent and in the presence of a catalyst to give:
   - the desired compound in the form of the free base in which $R_7$, $R_8$, $R_9$, $R_{10}$ or $R_{11}$ represent an amino group,
   - an aminoalkoxyphenyl derivative which can be reacted with an appropriate quantity of an alkyl halide having from 1 to 4 carbon atoms in the presence of an alkaline agent to give the desired derivative in the form of the free base, in which $R_7$, $R_8$, $R_9$, $R_{10}$ or $R_{11}$ represent a ($C_1$-$C_4$ alkyl)amino or ($C_1$-$C_4$ dialkyl)amino group,

   whilst the aminoalkoxyphenyl derivative thus obtained can, if desired, be reacted with an organic or inorganic acid to form a pharmaceutically acceptable salt.

5. Process according to claim 1, characterised in that the oxidising agent is sodium periodate, potassium permanganate or 3-chloro-perbenzoic acid.

6. Process according to claim 1, characterised in that aminoalkoxyphenyl derivatives of formula (1) are prepared wherein B represents an -$SO_2$- group.

7. Process according to claim 1, characterised in that aminoalkoxyphenyl derivatives of formula (1) are prepared wherein $R_1$ and $R_2$ each represent hydrogen.

8. Process according to claim 1, characterised in that aminoalkoxyphenyl derivatives of formula (1) are prepared wherein R represents an isopropyl or a cyclopropyl group.

9. Process according to claim 1, characterised in that aminoalkoxyphenyl derivatives of formula (1) are prepared wherein $R_8$, $R'_5$ and $R''_5$, which may be identical or different, each represent hydrogen, chlorine, or a methyl or methoxy group.

10. Process according to claim 1, characterised in that aminoalkoxyphenyl derivatives of formula (1) are prepared wherein $R_3$ represents an Alk-Ar radical.

11. Process according to claim 1, characterised in that aminoalkoxyphenyl derivatives of formula (1) are prepared wherein the -O-A-$NR_3$-$R_4$ chain represents an N-methyl-N-3,4-dimethoxy-β-phenethyl-amino)-propoxy or (N-methyl-N-3,5-dimethoxy-β-phenethyl-amino)-propoxy group.

12. Process according to claim 1, characterised in that aminoalkoxyphenyl derivatives of formula (1) are prepared wherein Ph represents a (D) group.

13. Process according to claim 1, characterised in that aminoalkoxyphenyl derivatives of formula (1) are prepared wherein Cy represents a (J) group.

14. Process according to claim 1, characterised in that aminoalkoxyphenyl derivatives of formula (1) are prepared wherein Cy represents a (K) or (K') group.

**15.** Process according to claim 1, characterised in that aminoalkoxyphenyl derivatives of formula (1) are prepared wherein Cy represents an (L) group.

**16.** Process according to claim 1, characterised in that aminoalkoxyphenyl derivatives of formula (1) are prepared, the pharmaceutically acceptable salt being the oxalate, hydrochloride or fumarate.

**17.** Process according to claim 1, characterised in that aminoalkoxyphenyl derivatives of formula (1) are prepared selected from the following compounds:
2-isopropyl-8-methyl-1-{4-[3-(N-methyl-N-3,5-dimethoxy-β-phenethyl-amino)-propoxy] benzenesulfonyl} indolizine;
2-isopropyl-8-methyl-1-{4-[3-(N-methyl-N-3,4-dimethoxy-β-phenethyl-amino)-propoxy] benzenesulfonyl} indolizine;
1-{4-[3-(di-n-butylamino)-propoxy] benzenesulfonyl}-2-isopropyl-8-methyl-indolizine;
2-isopropyl-5-methyl-1-{4-[3-(N-methyl-N-3,4-dimethoxy-β-phenethyl-amino)-propoxy] benzenesulfonyl} indolizine;
2-{4-[3-(N-methyl-N-3,4-dimethoxy-β-phenethylamino)-propoxy] benzenesulfonyl}-5-chloro-3-isopropyl-1-methyl indole
and the pharmaceutically acceptable salts thereof.

**18.** Process for preparing pharmaceutical or veterinary compositions, characterised in that at least one aminoalkoxyphenyl derivative prepared according to any one of the preceding claims is added as active principle to a suitable pharmaceutical carrier or excipient.

**19.** Process for preparing pharmaceutical or veterinary compositions according to claim 18, containing 50 to 500 mg of active principle for treating pathological syndromes of the cardiovascular system.

**20.** Process for preparing pharmaceutical or veterinary compositions according to claim 18, for treating ocular diseases.

**Claims for the following Contracting State : GR**

**1.** Aminoalkoxyphenyl derivatives of general formula:

Cy-B-Ph-O-A-Am (1)

as well as the pharmaceutically acceptable salts thereof, in which:
B represents a -S-, -SO- or $-SO_2$ group,
Ph represents a radical of formula:

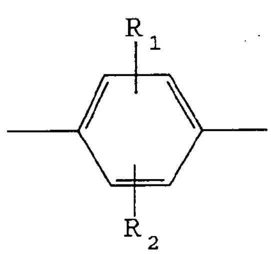

or

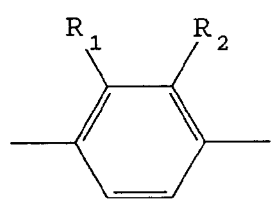

(D) (E)

$R_1$ and $R_2$, which are identical or different, each represents hydrogen, a methyl or ethyl radical or halogen,
A represents a straight-chained or branched alkylene radical having from 2 to 5 carbon atoms or a 2-hydroxypropylene radical in which the hydroxy is optionally substituted by a $C_1$-$C_4$ alkyl radical,
Am represents a group:

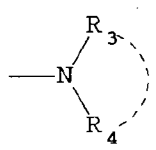

(F)

or

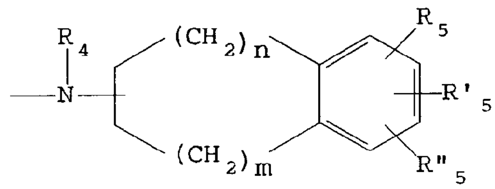

(G)

or

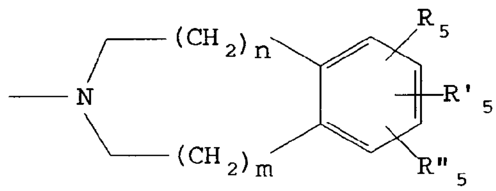

(H)

in which

$R_3$ represents a $C_1$-$C_8$ alkyl or a $C_3$-$C_6$ cycloalkyl radical, or a radical of formula:

-Alk-Ar

in which Alk represents a single bond or a straight-chained or branched alkylene radical having from 1 to 5 carbon atoms and Ar represents a pyridyl, phenyl, 2,3-methylenedioxyphenyl or 3,4-methylenedioxyphenyl radical or a phenyl group substituted by one or more identical or different substituents selected from halogen atoms,

$C_1$-$C_4$ alkyl groups or $C_1$-$C_4$ alkoxy groups,

$R_4$ represents hydrogen or a $C_1$-$C_8$ alkyl radical, or $R_3$ and $R_4$, when they are taken together, represent an alkylene or alkenylene radical having from 3 to 6 carbon atoms and optionally substituted by a phenyl radical or optionally interrupted by -O-, -N= or

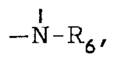,

$R_6$ representing hydrogen, a $C_1$-$C_4$ alkyl radical, a $C_3$-$C_6$ cycloalkyl radical, a phenyl radical optionally substituted by a halogen atom or by a $C_1$-$C_4$ alkyl group or a $C_1$-$C_4$ alkoxy group,

$R_5$, $R'_5$ and $R''_5$, which are identical or different, each represents hydrogen, a halogen atom, a $C_1$-$C_4$ alkyl group or a $C_1$-$C_4$ alkoxy group,

n and m, identical or different, each denotes 0, 1, 2, 3, Cy represents one of the groups of formula:

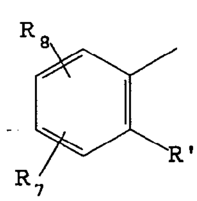

(I)

or

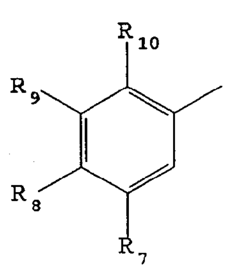

(I')

or 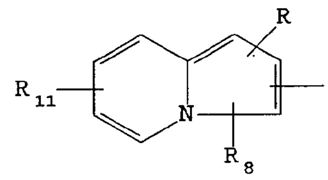 or 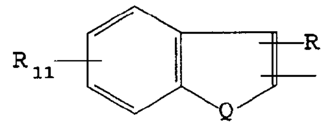

(J) (K)

or 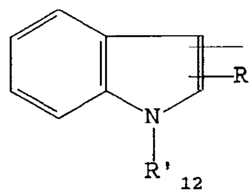 or 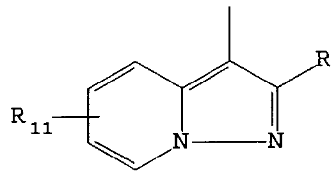

(K') (L)

or 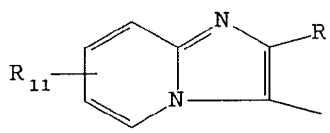 or 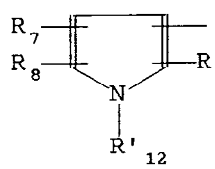

(M) (N)

or 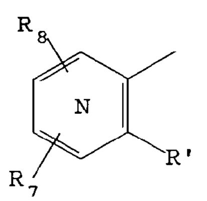 or 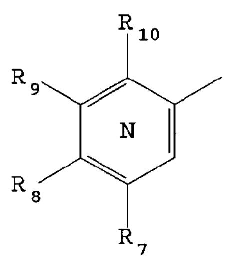

(Q) (Q')

in which:

R represents hydrogen, a $C_1$-$C_8$ alkyl radical, a $C_3$-$C_6$ cycloalkyl radical, a benzyl radical or a phenyl radical optionally substituted by one or more substituents which may be identical or different, selected from halogen atoms or from $C_1$-$C_4$ alkyl, $C_1$-$C_4$ alkoxy or nitro groups, R' represents a $C_1$-$C_8$ alkyl radical, a $C_3$-$C_6$ cycloalkyl radical, a benzyl radical or a phenyl radical optionally substituted by one or more substituents which may be identical or different, selected from halogen atoms or from $C_1$-$C_4$ alkyl, $C_1$-$C_4$ alkoxy or nitro groups,

$R_7$, $R_8$ and $R_9$, identical or different, each represents hydrogen, a $C_1$-$C_4$ alkyl radical, a $C_1$-$C_4$ alkoxy radical, a halogen atom, a hydroxy, benzyloxy, nitro, amino, ($C_1$-$C_4$ alkyl)-amino, di($C_1$-$C_4$ alkyl)-amino, sulfonamido, ($C_1$-$C_4$ alkyl)-sulfonamido, phenylsulfonamido, cyano, ($C_1$-$C_4$ alkoxy)-carbonyl group or ($C_1$-$C_4$ alkyl)-carbonyl group, $R_{10}$ denotes hydrogen, a $C_1$-$C_4$ alkoxy radical, a halogen atom, a hydroxy, benzyloxy, nitro, amino, ($C_1$-$C_4$ alkyl)amino, di($C_1$-$C_4$ alkyl)amino, sulfonamido, ($C_1$-$C_4$ alkyl)-sulfonamido, phenylsulfonamido, cyano, ($C_1$-$C_4$ alkoxy)carbonyl or ($C_1$-$C_4$ alkyl)carbonyl group,

the groups $R_7$ and $R_8$, on the one hand, and $R_7$, $R_8$, $R_9$ and $R_{10}$, on the other, never simultaneously representing hydrogen and the radical (I) or (I') not representing a monohalogenophenyl radical; $R_{11}$ represents a $C_1$-$C_4$ alkyl radical, a halogen atom, a hydroxy, benzyloxy, nitro, amino, ($C_1$-$C_4$ alkyl)amino, di($C_1$-$C_4$ alkyl)amino, sulfonamido, ($C_1$-$C_4$ alkyl)sulfonamido, phenylsulfonamido, cyano, ($C_1$-$C_4$ alkoxy)carbonyl or ($C_1$-$C_4$ alkyl)carbonyl group,
Q represents -N-$R_{12}$ in which $R_{12}$ represents hydrogen, a $C_1$-$C_4$ alkyl group, a $C_3$-$C_6$ cycloalkyl group, benzyl, halogenobenzyl, an -A-Am group as defined above, a ($C_1$-$C_4$ alkyl)sulfonyl group or a phenylsulfonyl group optionally substituted by one or more halogen atoms or $C_1$-$C_4$ alkyl or $C_1$-$C_4$ alkoxy groups,
$R'_{12}$ represents an -A-Am group as defined above, an alkylsulfonyl group optionally substituted by one or more halogen atoms, $C_1$-$C_4$ alkyl or $C_1$-$C_4$ alkoxy groups.

**2.** Aminoalkoxyphenyl derivatives according to Claim 1, in which B represents an -$SO_2$- group.

**3.** Aminoalkoxyphenyl derivatives according to Claim 1 or 2, characterised in that $R_1$ and $R_2$ each represents hydrogen.

**4.** Aminoalkoxyphenyl derivatives according to any one of Claims 1 to 3, characterised in that R represents an isopropyl or a cyclopropyl group.

**5.** Aminoalkoxyphenyl derivatives according to any one of Claims 1 to 4, characterised in that $R_5$, $R'_5$ and $R''_5$, identical or different, each represents hydrogen, chlorine, or a methyl or methoxy group.

**6.** Aminoalkoxyphenyl derivatives according to any one of Claims 1 to 5, characterised in that $R_3$ represents an -Alk-Ar radical.

**7.** Aminoalkoxyphenyl derivatives according to any one of Claims 1 to 6, characterised in that the -O-A-$NR_3$-$R_4$ chain represents an
(N-methyl-N-3,4-dimethoxy-β-phenethylamino)-propoxy or (N-methyl-N-3,5-dimethoxy-β-phenethylamino)-propoxy group.

**8.** Aminoalkoxyphenyl derivatives according to any one of Claims 1 to 7, characterised in that Ph represents a (D) group.

**9.** Aminoalkoxyphenyl derivatives according to any one of Claims 1 to 7, characterised in that Cy represents a (J) group.

**10.** Aminoalkoxyphenyl derivatives according to any one of Claims 1 to 7, characterised in that Cy represents a (K) or (K') group.

**11.** Aminoalkoxyphenyl derivatives according to any one of Claims 1 to 7, characterised in that Cy represents an (L) group.

**12.** Aminoalkoxyphenyl derivatives according to any one of Claims 1 to 11, characterised in that the pharmaceutically acceptable salt is the oxalate, hydrochloride or fumarate.

**13.** Aminoalkoxyphenyl derivatives according to Claim 1 selected from the following compounds:
2-isopropyl-8-methyl-1-{4-[3-(N-methyl-N-3,5-dimethoxy-β-phenethyl-amino)-propoxy] benzenesulfonyl} indolizine;
2-isopropyl-8-methyl-1-{4-[3-(N-methyl N-3,4-dimethoxy-β-phenethyl-amino)-propoxy] benzenesulfonyl} indolizine;
1-{4[3-(di-n-butylamino)-propoxy] benzenesulfonyl}-2-isopropyl-8-methyl-indolizine;
2-isopropyl-8-methyl-1-{4-[3-(6,7-dimethoxy-1,2,3,4- tetrahydroisoquinolin-2-yl)-propoxy] benzenesulfonyl} indolizine;
2-isopropyl-5-methyl-1-{4-[3-(N-methyl-N-3,4-dimethoxy-β-phenethyl-amino)-propoxy] benzenesulfonyl} indolizine;
2-{4-[3-(N-methyl-N-3,4-dimethoxy-β-phenethylamino)- propoxy] benzenesulfonyl}-5-chloro-3-isopropyl-1-methyl indole
and the pharmaceutically acceptable salts thereof.

**14.** Process for the preparation of aminoalkoxyphenyl derivatives of formula (1) according to Claim 1 in which A denotes an alkylene radical and B denotes a -S- or -$SO_2$- group, characterised in that, in the presence

of an acid acceptor in a polar or non-polar solvent and at a temperature between room temperature and reflux temperature, a 4-alkoxyphenyl derivative of general formula:

Cy-B'-Ph-O-A-X (2)

in which B' represents an -S- or $-SO_2-$ group, Cy and Ph have the same meaning as in Claim 1, A has the same meaning as above and X represents a halogen atom or an alkylsulfonyloxy group having from 1 to 4 carbon atoms or an arylsulfonyloxy group having from 6 to 10 carbon atoms, is condensed with an amine of general formula:

H-Am (3)

in which Am has the same meaning as in Claim 1, to form the desired derivative which can be reacted, if necessary, with an organic or inorganic acid to form a pharmaceutically acceptable salt of this derivative.

**15.** Process for the preparation of aminoalkoxyphenyl derivatives of formula (1) according to Claim 1 in which B represents an -S- or $-SO_2-$ group, characterised in that, in the presence of a basic agent, a 4-hydroxyphenyl derivative of general formula:

Cy-B'-Ph-OH (4)

in which B' represents an -S- or $-SO_2-$ group and Cy and Ph have the same meaning as in Claim 1, is reacted with a compound of general formula:

X-A-Am (31)

in which X represents a halogen atom or an alkylsulfoxy group having from 1 to 4 carbon atoms or an arylsulfoxy group having from 6 to 10 carbon atoms, A represents an alkylene radical and Am has the same meaning as in Claim 1, the reaction being carried out by refluxing in a suitable solvent to give the desired derivative which can be reacted, if desired, with a suitable organic or inorganic acid to form a pharmaceutically acceptable salt.

**16.** Process for the preparation of aminoalkoxyphenyl derivatives of formula (1) according to claim 1, in which A represents an optionally substituted 2-hydroxypropylene radical and B represents an -S- or $-SO_2-$ group, characterised in that an oxiranylmethoxy derivative of general formula:

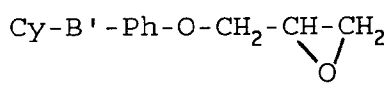

(33)

in which B' represents an -S- or $-SO_2-$ group and Cy and Ph have the same meaning as in Claim 1, is refluxed with an amine of general formula:

H-Am (3)

in which Am has the same meaning as in Claim 1, in a polar solvent or in an excess of the said amine to give:

- the required derivative, in the form of the free base, in which A represents a 2-hydroxypropylene radical,
- an aminoalkoxyphenyl derivative which can be reacted with an alkyl halide having from 1 to 4 carbon atoms and in the presence of a strong base, to give the required derivative in the form of the free base, in which A represents a 2-hydroxypropylene radical in which the hydroxy is substituted by a $C_1$-$C_4$ alkyl group whilst the aminoalkoxyphenyl derivative thus obtained can, if desired, be reacted with an organic or inorganic acid to form a pharmaceutically acceptable salt.

**17.** Process for the preparation of aminoalkoxyphenyl derivatives of formula (1) according to Claim 1 in which B represents a -SO- group, characterised in that a sulfide of general formula:

Cy-S-Ph-O-A-Am (1)

in which Cy, Ph, A and Am have the same meaning as in Claim 1 is treated with an oxidising agent, said sulfide being in the form of the free base or a salt, to form the required derivative in the form of the free base or salt, which is treated with a basic agent to give the required derivative in the form of the free base, the free base thus obtained being, if desired, reacted with an organic or inorganic acid to form a pharmaceutically acceptable salt of this derivative.

**18.** Process according to Claim 17, characterised in that the oxidizing agent is sodium periodate, potassium permanganate or 3-chloro-perbenzoic acid.

**19.** Process for the preparation of aminoalkoxyphenyl derivatives of formula (1) according to Claim 1, in which

$R_7$, $R_8$, $R_9$, $R_{10}$ or $R_{11}$ represent a hydroxy group, characterised in that a compound of formula (1) according to Claim 1, in which $R_7$, $R_8$, $R_9$, $R_{10}$ or $R_{11}$ represent a benzyloxy group, is hydrogenated in a suitable solvent and in the presence of a catalyst to give the required derivative which can, if necessary, be reacted with a suitable organic or inorganic acid to form a pharmaceutically acceptable salt.

**20.** Process for the preparation of aminoalkoxyphenyl derivatives of formula (1) according to Claim 1, in which $R_7$, $R_8$, $R_9$, $R_{10}$ or $R_{11}$ represent an amino, a lower alkylamino or lower dialkylamino group, characterised in that a compound of formula (1) according to Claim 1, in which $R_7$, $R_8$, $R_9$, $R_{10}$ or $R_{11}$ represent a nitro group, is hydrogenated in a suitable solvent and in the presence of a catalyst to give:

- the desired compound in the form of the free base in which $R_7$, $R_8$, $R_9$, $R_{10}$ or $R_{11}$ represent an amino group,
- an aminoalkoxyphenyl derivative which can be reacted with an appropriate quantity of an alkyl halide having from 1 to 4 carbon atoms in the presence of an alkaline agent to give the desired derivative in the form of the free base, in which $R_7$, $R_8$, $R_9$, $R_{10}$ or $R_{11}$ represent a ($C_1$-$C_4$ alkyl)amino or ($C_1$-$C_4$ dialkyl)amino group,

  whilst the aminoalkoxyphenyl derivative thus obtained can, if desired, be reacted with an organic or inorganic acid to form a pharmaceutically acceptable salt.

**21.** Process for preparing pharmaceutical or veterinary compositions, characterised in that at least one aminoalkoxyphenyl derivative according to any one of claims 1 to 13 is combined with a suitable pharmaceutical carrier or excipient.

**22.** Process for preparing pharmaceutical or veterinary compositions according to claim 21, for treating pathological syndromes of the cardiovascular system containing 50 mg to 500 mg of active principle.

**23.** Process for preparing pharmaceutical or veterinary compositions according to claim 21 intended for treating ocular diseases.

## Patentansprüche

### Patentansprüche für folgende Vertragsstaaten : CH, DE, DK, FR, GB, IT, LI, LU, NL, SE, AT, BE

**1.** Aminoalkoxyphenyl-Derivate der allgemeinen Formel:

Cy-B-PH-O-A-Am (1)

sowie deren pharmazeutisch annehmbare Salze, worin

B eine Gruppe -S-, -SO- oder -$SO_2$-,

Ph eine Gruppe der Formel

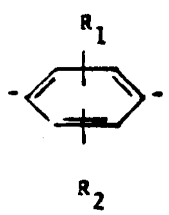

(D)

oder

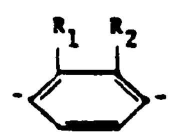

(E)

$R_1$ und $R_2$, die gleichartig oder verschieden sein können, jeweils Wasserstoffatome. Methyl- oder Ethylgruppen oder Halogenatome,

A eine geradkettige oder verzweigte Alkylengruppe mit 2 bis 5 Kohlenstoffatomen oder den 2-Hydroxypropylenrest, worin die Hydroxylgruppe gegebenenfalls durch eine $C_1$-$C_4$-Alkylgruppe substituiert ist,

Am eine Gruppe der Formeln

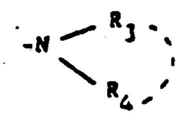

oder

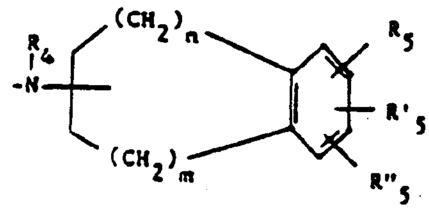

(F)

(G)

oder

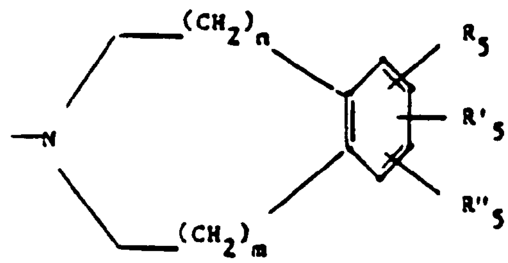

(H)

worin

$R_3$ eine $C_1$-$C_8$-Alkylgruppe, eine $C_3$-$C_6$-Cycloalkylgruppe oder eine Gruppe der Formel

- Alk - Ar

in der Alk eine Einfachbindung oder eine geradkettige oder verzweigte Alkylengruppe mit 1 bis 5 Kohlenstoffatomen und Ar einen Pyridyl-, Phenyl-, 2,3-Methylendioxy-phenyl- oder 3,4-Methylendioxyphenylrest oder eine Phenylgruppe, die durch einen oder mehrere gleichartige oder verschiedene Substituenten ausgewählt aus Halogenatomen, $C_1$-$C_4$-Alkylgruppen oder $C_1$-$C_4$-Alkoxygruppen substituiert ist, darstellen,

$R_4$ ein Wasserstoffatom oder eine $C_1$-$C_8$-Alkylgruppe oder

$R_3$ und $R_4$ gemeinsam eine Alkylen- oder Alkenylengruppe mit 3 bis 6 Kohlenstoffatomen, die gegebenenfalls durch einen Phenylrest substituiert oder gege- benenfalls durch -O-, -N= oder

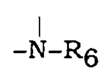

unterbrochen sein kann, worin $R_6$ ein Wasserstoffatom, eine $C_1$-$C_4$-Alkylgruppe, eine $C_3$-$C_6$-Cycloalkylgruppe, eine durch ein Halogenatom oder eine $C_1$-$C_4$-Alkylgruppe oder eine $C_1$-$C_4$-Alkoxygruppe substituierte Phenylgruppe darstellt,

$R_5$, $R'_5$ und $R''_5$, die gleichartig oder verschieden sein können, jeweils Wasserstoffatome, Halogenatome, $C_1$-$C_4$-Alkylgruppen oder $C_1$-$C_4$-Alkoxygruppen,

n und m, die gleichartig oder verschieden sein können, jeweils 0, 1, 2 oder 3 darstellen und

Cy eine der Gruppen der Formel:

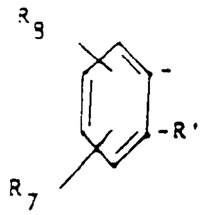

(I)

oder

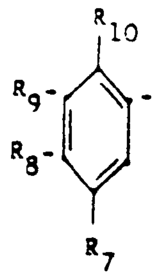

(I')

oder

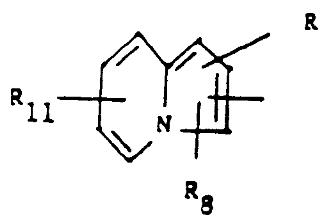

(J)

oder

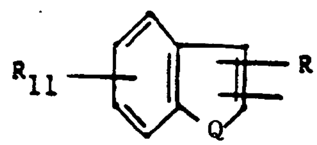

(K)

oder

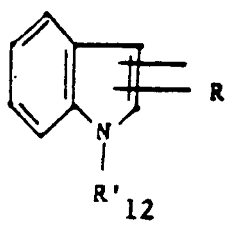

(K')

oder

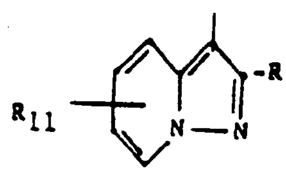

(L)

oder

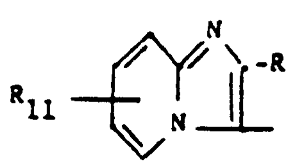

(M)

oder

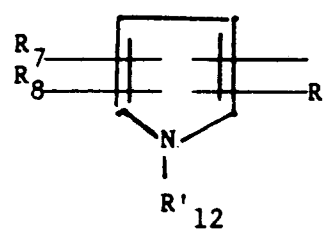

(N)

oder 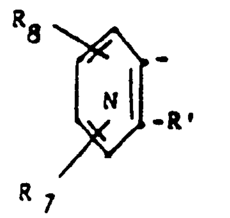 oder 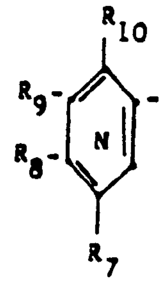

(Q) (Q')

worin

R ein Wasserstoffatom, eine $C_1$-$C_8$-Alkylgruppe, eine $C_3$-$C_6$-Cycloalkylgruppe, eine Benzylgruppe oder eine Phenylgruppe, die gegebenenfalls durch einen oder mehrere Substituenten, die gleichartig oder verschieden sein können und aus Halogenatomen, $C_1$-$C_4$-Alkylgruppen, $C_1$-$C_4$-Alkoxygruppen oder Nitrogruppen ausgewählt sind. substituiert ist,

R' eine $C_1$-$C_8$-Alkylgruppe, eine $C_3$-$C_6$-Cycloalkylgruppe, eine Benzylgruppe oder eine Phenylgruppe, die gegebenenfalls durch einen oder mehrere Substituenten, die gleichartig oder verschieden sein können, und aus Halogenatomen, $C_1$-$C_4$-Alkylgruppen, $C_1$-$C_4$-Alkoxygruppen oder Nitrogruppen ausgewählt sind, substituiert ist,

$R_7$, $R_8$ und $R_9$, die gleichartig oder verschieden sein können, jeweils Wasserstoffatome, $C_1$-$C_4$-Alkylgruppen, $C_1$-$C_4$-Alkoxygruppen, Halogenatome, Hydroxylgruppen, Benzyloxygruppen, Nitrogruppen, Aminogruppen, ($C_1$-$C_4$-Alkyl)-aminogruppen, Di-($C_1$-$C_4$-alkyl)-aminogruppen, Sulfonamidogruppen, ($C_1$-$C_4$-Alkyl)-sulfonamidogruppen, Phenylsulfonamidogruppen, Cyanogruppen, ($C_1$-$C_4$-Alkoxy)-carbonylgruppen oder ($C_1$-$C_4$-Alkyl)-carbonylgruppen, $R_{10}$ ein Wasserstoffatom, eine $C_1$-$C_4$-Alkoxygruppe, ein Halogenatom, eine Hydroxylgruppe, eine Benzyloxygruppe, eine Nitrogruppe, eine Aminogruppe, eine ($C_1$-$C_4$-Alkyl)-aminogruppe, eine Di-($C_1$-$C_4$-alkyl)-aminogruppe, eine Sulfonamidogruppe, eine ($C_1$-$C_4$-Alkyl)-sulfonamidogruppe, eine Phenylsulfonamidogruppe, eine Cyanogruppe, eine ($C_1$-$C_4$-Alkoxy)-carbonylgruppe oder eine ($C_1$-$C_4$-Alkyl)-carbonylgruppe,

wobei die Gruppen $R_7$ und $R_8$ einerseits und $R_7$, $R_8$, $R_9$ und $R_{10}$ andererseits niemals gleichzeitig Wasserstoffatome bedeuten und der Rest (I) oder (I') keinen Monohalogenophenylrest darstellt,

$R_{11}$ eine $C_1$-$C_4$-Alkylgruppe, ein Halogenatom, eine Hydroxylgruppe, eine Benzyloxygruppe, eine Nitrogruppe, eine Aminogruppe, eine ($C_1$-$C_4$-Alkyl)-aminogruppe, eine Di-($C_1$-$C_4$-alkyl)-aminogruppe, eine Sulfonamidogruppe, eine ($C_1$-$C_4$-Alkyl)-sulfonamidogruppe, eine Phenylsulfonamidogruppe, eine Cyanogruppe, eine ($C_1$-$C_4$-Alkoxy)-carbonylgruppe oder eine $C_1$-$C_4$-Alkyl)-carbonylgruppe,

Q eine Gruppe der Formel

$$-\overset{|}{N}-R_{12}.$$

worin $R_{12}$ ein Wasserstoffatom, eine $C_1$-$C_4$- Alkylgruppe, eine $C_3$-$C_6$-Cycloalkylgruppe, eine Benzylgruppe, eine Halogenbenzylgruppe, eine Gruppe der Formel -A-Am, wie sie oben definiert worden ist, eine ($C_1$-$C_4$-Alkyl)-sulfonylgruppe oder eine Phenylsulfonylgruppe, die gegebenenfalls durch eines oder mehrere Halogenatome, $C_1$-$C_4$-Alkylgruppen oder $C_1$-$C_4$-Alkoxygruppen substituiert ist, und

$R'_{12}$ eine Gruppe der Formel -A-Am, wie sie oben definiert worden ist, eine Alkylsulfonylgruppe, die gegebenenfalls durch eines oder mehrere Halogenatome,

$C_1$-$C_4$-Alkylgruppen oder $C_1$-$C_4$-Alkoxygruppen substituiert ist, darstellen, bedeuten.

**2.** Aminoalkoxyphenyl-Derivate nach Anspruch 1, worin B eine Gruppe der Formel -$SO_2$- bedeutet.

**3.** Aminoalkoxyphenyl-Derivate nach Anspruch 1 oder 2, **dadurch gekennzeichnet**, daß $R_1$ und $R_2$ jeweils Wasserstoff bedeuten.

**4.** Aminoalkoxyphenyl-Derivate nach einem der Ansprüche 1 bis 3, **dadurch gekennzeichnet**, daß R eine Isopropylgruppe oder eine Cyclopropylgruppe bedeutet.

**5.** Aminoalkoxyphenyl-Derivate nach einem der Ansprüche 1 bis 4, **dadurch gekennzeichnet**, daß $R_8$, $R'_5$

und R"$_5$, die gleichartig oder verschieden sein können, jeweils Wasserstoff, Chlor, eine Methylgruppe oder eine Methoxygruppe bedeuten.

6. Aminoalkoxyphenyl-Derivate nach einem der Ansprüche 1 bis 5, **dadurch gekennzeichnet**, daß $R_3$ eine Gruppe der Formel -Alk-Ar bedeutet.

7. Aminoalkoxyphenyl-Derivate nach einem der Ansprüche 1 bis 6, **dadurch gekennzeichnet**, daß die Kette -O-A-$NR_3$-$R_4$ eine [N-Methyl-N-(3,4-dimethoxy-β-phenethyl)-amino]-propoxygruppe oder eine [N-Methyl-N-(3,5-dimethoxy-β-phenethyl)-amino]-propoxygruppe bedeutet.

8. Aminoalkoxyphenyl-Derivate nach einem der Ansprüche 1 bis 7, **dadurch gekennzeichnet**, daß Ph eine Gruppe (D) bedeutet.

9. Aminoalkoxyphenyl-Derivate nach einem der Ansprüche 1 bis 7, **dadurch gekennzeichnet**, daß Cy eine Gruppe (J) bedeutet.

10. Aminoalkoxyphenyl-Derivate nach einem der Ansprüche 1 bis 7, **dadurch gekennzeichnet**, daß Cy eine Gruppe (K) oder (K') bedeutet.

11. Aminoalkoxyphenyl-Derivate nach einem der Ansprüche 1 bis 7, **dadurch gekennzeichnet**, daß Cy eine Gruppe (L) bedeutet.

12. Aminoalkoxyphenyl-Derivate nach einem der Ansprüche 1 bis 11, **dadurch gekennzeichnet**, daß das pharmazeutisch annehmbare Salz das Oxalat, Hydrochlorid oder Fumarat ist.

13. Aminoalkoxyphenyl-Derivate nach Anspruch 1, ausgewählt aus den folgenden Verbindungen:
2-Isopropyl-8-methyl-1-[4-{3-[N-methyl-N-(3,5-dimethoxy-β-phenethyl)-amino]-propoxy}-benzolsulfonyl]-indolizin,
2-Isopropyl-8-methyl-1-[4-{3-[N-methyl-N-(3,4-dimethoxy-β-phenethyl)-amino]-propoxy}-benzolsulfonyl]-indolizin,
1-{4-[3-(Di-n-butylamino)-propoxy]-benzolsulfonyl}-2-isopropyl-8-methyl-indolizin,
2-Isopropyl-8-methyl-1-{4-[3-(6,7-dimethoxy-1,2,3,4-tetrahydro-isochinolin-2-yl)-propoxy]-benzolsulfonyl}-indolizin,
2-Isopropyl-5-methyl-1-[4-{3-[N-methyl-N-(3,4-dimethoxy-β-phenethyl)-amino]-propoxy}-benzolsulfonyl]-indolizin,
2-[4-{3-[N-Methyl-N-(3,4-dimethoxy-β-phenethyl)-amino]-propoxy}-benzolsulfonyl]-5-chlor-3-isopropyl-4-methyl-indol,
und deren pharmazeutisch annehmbaren Salzen.

14. Verfahren zur Herstellung der Aminoalkoxyphenyl-Derivate der Formel (1) nach Anspruch 1, worin A eine Alkylengruppe und B eine Gruppe -S- oder -$SO_2$- bedeuten, **dadurch gekennzeichnet**, daß man ein 4-Alkoxyphenyl-Derivat der allgemeinen Formel

Cy-B'-Ph-O-A-X (2)

in der B' eine Gruppe -S- oder -$SO_2$- bedeutet, Cy und Ph die in Anspruch 1 angegebenen Bedeutungen besitzen, A die oben angegebene Bedeutung besitzt und X ein Halogenatom oder eine Alkylsulfonyloxygruppe mit 1 bis 4 Kohlenstoffatomen oder eine Arylsulfonyloxygruppe mit 6 bis 10 Kohlenstoffatomen darstellt, in Gegenwart eines Säureakzeptors in einem polaren oder nichtpolaren Lösungsmittel bei einer Temperatur zwischen Raumtemperatur und der Rückflußtemperatur mit einem Amin der allgemeinen Formel:

H-Am (3)

worin Am die in Anspruch 1 angegebenen Bedeutungen besitzt, kondensiert zur Bildung des gewünschten Derivats. welches man erforderlichenfalls mit einer organischen oder anorganischen Säure umsetzen kann zur Bildung eines pharmazeutisch annehmbaren Salzes dieses Derivats.

15. Verfahren zur Herstellung der Aminoalkoxyphenyl-Derivate der Formel (1) nach Anspruch 1, worin B eine Gruppe -S- oder -$SO_2$- bedeutet, **dadurch gekennzeichnet**, daß man ein 4-Hydroxyphenyl-Derivat der allgemeinen Formel:

Cy-B'-Ph-OH (4)

in der B' eine Gruppe -S- oder -$SO_2$- bedeutet und Cy und Ph die in Anspruch 1 angegebenen Bedeutungen besitzen, in Gegenwart eines basischen Mittels mit einer Verbindung der allgemeinen Formel:

X-A-Am (31)

in der X ein Halogenatom oder eine Alkylsulfoxygruppe mit 1 bis 4 Kohlenstoffatomen oder eine Arylsulfoxygruppe mit 6 bis 10 Kohlenstoffatomen und A eine Alkylengruppe bedeuten und Am die in Anspruch 1 angegebenen Bedeutungen besitzt, bei der Rückflußtemperatur in einem geeigneten Lösungsmittel umsetzt zur Bildung des gewünschten Derivats, welches man gewünschtenfalls mit einer geeigneten organischen oder anorganischen Säure umsetzen kann zur Bildung eines pharmazeutisch annehmbaren Salzes.

**16.** Verfahren zur Herstellung der Aminoalkoxyphenyl-Derivate der Formel (1) nach Anspruch 1, worin A den 2-Hydroxypropylenrest, der gegebenenfalls substituiert sein kann, und B eine Gruppe -S- oder -$SO_2$- bedeuten, **dadurch gekennzeichnet**, daß man ein Oxyranylmethoxy-Derivat der allgemeinen Formel:

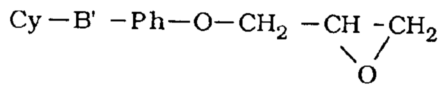

(33)

in der B' eine Gruppe -S- oder -$SO_2$- bedeutet und Cy und Ph die in Anspruch 1 angegebenen Bedeutungen besitzen, bei der Rückflußtemperatur mit einem Amin der allgemeinen Formel:

H - Am (3)

in der Am die in Anspruch 1 angegebenen Bedeutungen besitzt, in einem polaren Lösungsmittel oder in einem Überschuß des genannten Amins behandelt, so daß man

- das gewünschte Derivat in Form der freien Base, worin A eine 2-Hydroxypropylengruppe darstellt, oder
- ein Aminoalkoxyphenyl-Derivat erhält, welches man mit einem Alkylhalogenid mit 1 bis 4 Kohlenstoffatomen in Gegenwart einer starken Base umsetzen kann, so daß man das gewünschte Derivat in Form der freien Base erhält, worin A eine 2-Hydroxypropylengruppe darstellt, in der die Hydroxylgruppe durch eine Alkylgruppe mit 1 bis 4 Kohlenstoffatomen substituiert ist,

wobei man das in dieser Weise erhaltene Aminoalkoxyphenyl-Derivat gewünschtenfalls mit einer organischen oder anorganischen Säure umsetzen kann zur Bildung eines pharmazeutisch annehmbaren Salzes.

**17.** Verfahren zur Herstellung von Aminoalkoxyphenyl-Derivaten der Formel (1) nach Anspruch 1, worin B eine Gruppe -SO- bedeutet, **dadurch gekennzeichnet**, daß man ein Sulfid der allgemeinen Formel

Cy - S - Ph - O - A - Am (1)

in der Cy, Ph, A und Am die in Anspruch 1 angegebenen Bedeutungen besitzen, welches Sulfid in Form der freien Base oder eines Salzes vorliegt, mit einem Oxidationsmittel behandelt zur Bildung des gewünschten Derivats in Form der freien Base oder des Salzes, welches man mit einem basischen Mittel behandelt zur Bildung des gewünschten Derivats in Form der freien Base, welche freie Base gewünschtenfalls mit einer organischen oder anorganischen Säure umgesetzt werden kann zur Bildung eines pharmazeutisch annehmbaren Salzes dieses Derivats.

**18.** Verfahren nach Anspruch 17, **dadurch gekennzeichnet**, daß das Oxidationsmittel Natriumperiodat, Kaliumpermanganat oder 3-Chlor-perbenzoesäure ist.

**19.** Verfahren zur Herstellung von Aminoalkoxyphenyl-Derivaten der Formel (1) nach Anspruch 1, worin $R_7$, $R_8$, $R_9$, $R_{10}$ oder $R_{11}$ eine Hydroxylgruppe bedeuten, **dadurch gekennzeichnet**, daß man eine Verbindung der Formel (1) nach Anspruch 1, worin $R_7$, $R_8$, $R_9$, $R_{10}$ oder $R_{11}$ eine Benzyloxygruppe bedeuten, in einem geeigneten Lösungsmittel und in Gegenwart eines Katalysators hydriert zur Bildung des gewünschten Derivats, welches man erforderlichenfalls mit einer geeigneten organischen oder anorganischen Säure umsetzen kann zur Bildung eines pharmazeutisch annehmbaren Salzes.

**20.** Verfahren zur Herstellung von Aminoalkoxyphenyl-Derivaten der Formel (1) nach Anspruch 1, worin $R_7$, $R_8$, $R_9$, $R_{10}$ oder $R_{11}$ eine Aminogruppe, die Niedrigalkylaminogruppe oder eine Diniedrigalkylaminogruppe bedeuten, **dadurch gekennzeichnet**, daß man eine Verbindung der Formel (1) nach Anspruch 1, worin $R_7$, $R_8$, $R_9$, $R_{10}$ oder $R_{11}$ eine Nitrogruppe bedeuten, in einem geeigneten Lösungsmittel in Gegenwart eines Katalysators hydriert, so daß man

- die gewünschte Verbindung in Form der freien Base, in der $R_7$, $R_8$, $R_9$, $R_{10}$ oder $R_{11}$ eine Aminogruppe bedeuten, oder
- ein Aminoalkoxyphenyl-Derivat erhält, welches man mit einer geeigneten Menge eines Alkylhalogenids mit 1 bis 4 Kohlenstoffatomen und in Gegenwart eines alkalischen Mittels umsetzen kann zur Bildung des gewünschten Derivats in Form der freien Base, in dem $R_7$, $R_8$, $R_9$, $R_{10}$ oder $R_{11}$ eine ($C_1$-$C_4$-Alkyl)-aminogruppe oder eine Di-($C_1$-$C_4$-alkyl)-aminogruppe bedeuten,

welches in dieser Weise erhaltene Aminoalkoxyphenyl-Derivat man gewünschtenfalls mit einer organischen oder anorganischen Säure umsetzen kann zur Bildung eines pharmazeutisch annehmbaren Salzes.

**21.** Pharmazeutische oder veterinärmedizinische Zubereitungen enthaltend als Wirkstoff mindestens ein Aminoalkoxyphenyl-Derivat nach einem der Ansprüche 1 bis 13 in Kombination mit einem geeigneten pharmazeutischen Trägermaterial oder Bindemittel.

**22.** Pharmazeutische oder veterinärmedizinische Zubereitungen nach Anspruch 21 zur Behandlung von pathologischen Syndromen des kardiovaskulären Systems, enthaltend 50 mg bis 500 mg des Wirkstoffs.

**23.** Verwendung mindestens eines Aminoalkoxyphenyl-Derivats nach einem der Ansprüche 1 bis 13 zur Herstellung eines Arzneimittels zur Behandlung von pathologischen Syndromen des kardiovaskulären Systems.

**24.** Verwendung mindestens eines Aminoalkoxyphenyl-Derivats nach einem der Ansprüche 1 bis 13 zur Herstellung eines Arzneimittels zur Behandlung von pathologischen Zuständen des Auges.

**Patentansprüche für folgenden Vertragsstaat : ES**

**1.** Verfahren zur Herstellung von Aminoalkoxyphenyl-Derivaten der allgemeinen Formel:

Cy - B - PH -O- A - Am (1)

sowie deren pharmazeutisch annehmbare Salze, worin
B eine Gruppe -S-, -SO- oder -$SO_2$-,
Ph eine Gruppe der Formel

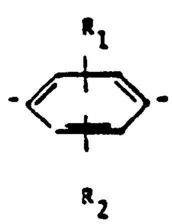

(D)

oder

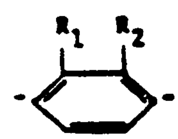

(E)

$R_1$ und $R_2$, die gleichartig oder verschieden sein können, jeweils Wasserstoffatome, Methyl- oder Ethylgruppen oder Halogenatome,
A eine geradkettige oder verzweigte Alkylengruppe mit 2 bis 5 Kohlenstoffatomen oder den 2-Hydroxypropylenrest, worin die Hydroxylgruppe gegebenenfalls durch eine $C_1$-$C_4$-Alkylgruppe substituiert ist,
Am eine Gruppe der Formeln

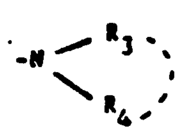

(F)

oder

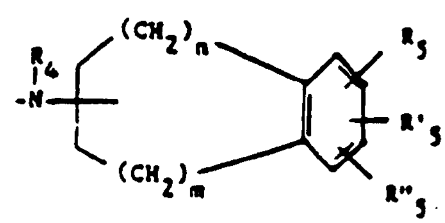

(G)

oder 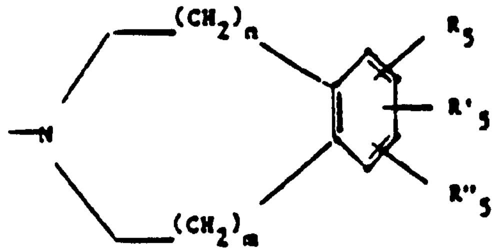

(H)

worin

$R_3$ eine $C_1$-$C_8$-Alkylgruppe, eine $C_3$-$C_6$-Cycloalkylgruppe oder eine Gruppe der Formel

Alk - Ar

in der Alk eine Einfachbindung oder eine geradkettige oder verzweigte Alkylengruppe mit 1 bis 5 Kohlenstoffatomen und Ar einen Pyridyl-, Phenyl-, 2,3-Methylendioxy-phenyl- oder 3,4-Methylendioxyphenylrest oder eine Phenylgruppe, die durch einen oder mehrere gleichartige oder verschiedene Substituenten ausgewählt aus Halogenatomen, $C_1$-$C_4$-Alkylgruppen oder $C_1$-$C_4$-Alkoxygruppen substituiert ist, darstellen,

$R_4$ ein Wasserstoffatom oder eine $C_1$-$C_8$-Alkylgruppe oder

$R_3$ und $R_4$ gemeinsam eine Alkylen- oder Alkenylengruppe mit 3 bis 6 Kohlenstoffatomen, die gegebenenfalls durch einen Phenylrest substituiert oder gegebenenfalls durch -O-, -N= oder

$$-\overset{|}{N}-R_6$$

unterbrochen sein kann, worin $R_6$ ein Wasserstoffatom, eine $C_1$-$C_4$-Alkylgruppe, eine $C_3$-$C_6$-Cycloalkylgruppe, eine durch ein Halogenatom oder eine $C_1$-$C_4$-Alkylgruppe oder eine $C_1$-$C_4$-Alkoxygruppe substituierte Phenylgruppe darstellt,

$R_5$, $R'_5$ und $R''_5$, die gleichartig oder verschieden sein können, jeweils Wasserstoffatome, Halogenatome, $C_1$-$C_4$-Alkylgruppen oder $C_1$-$C_4$-Alkoxygruppen,

n und m, die gleichartig oder verschieden sein können, jeweils 0, 1, 2 oder 3 darstellen und

Cy eine der Gruppen der Formel:

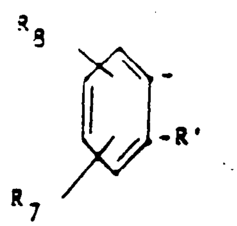

(I)

oder

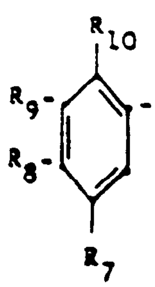

(I')

oder 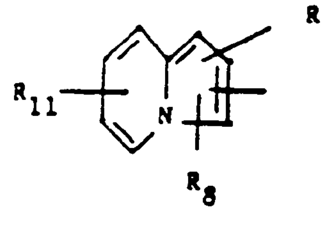

(J)

oder 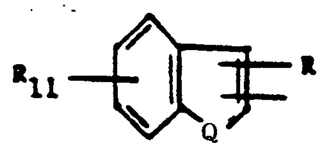

(K)

oder 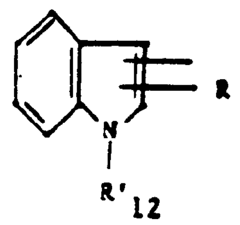

(K')

oder 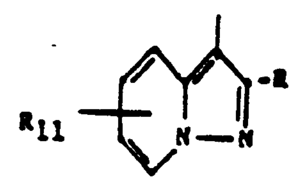

(L)

oder 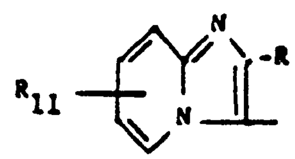

(M)

oder 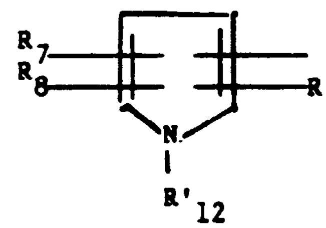

(N)

·oder 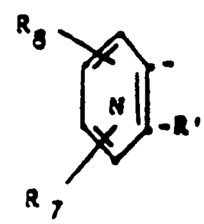

(Q)

oder 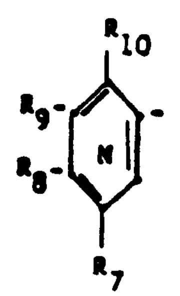

(Q')

worin

R ein Wasserstoffatom, eine $C_1$-$C_8$-Alkylgruppe, eine $C_3$-$C_6$-Cycloalkylgruppe, eine Benzylgruppe oder eine Phenylgruppe, die gegebenenfalls durch einen oder mehrere Substituenten, die gleichartig oder verschieden sein können und aus Halogenatomen, $C_1$-$C_4$-Alkylgruppen, $C_1$-$C_4$-Alkoxygruppen oder Nitrogruppen ausgewählt sind, substituiert ist,

R' eine $C_1$-$C_8$-Alkylgruppe, eine $C_3$-$C_6$-Cycloalkylgruppe, eine Benzylgruppe oder eine Phenylgruppe, die gegebenenfalls durch einen oder mehrere Substituenten, die gleichartig oder verschieden sein können, und aus Halogenatomen, $C_1$-$C_4$-Alkylgruppen, $C_1$-$C_4$-Alkoxygruppen oder Nitrogruppen ausgewählt sind, substituiert ist,

$R_7$, $R_8$ und $R_9$, die gleichartig oder verschieden sein können, jeweils Wasserstoffatome, $C_1$-$C_4$-Alkylgruppen, $C_1$-$C_4$-Alkoxygruppen, Halogenatome. Hydroxylgruppen, Benzyloxygruppen, Nitrogruppen, Aminogruppen, ($C_1$-$C_4$-Alkyl)-aminogruppen, Di-($C_1$-$C_4$-alkyl)-aminogruppen, Sulfonamidogruppen, ($C_1$-$C_4$-Alkyl)-sulfonamidogruppen, Phenylsulfonamidogruppen, Cyanogrup- pen, ($C_1$-$C_4$-Alkoxy)-carbonylgruppen oder ($C_1$-$C_4$-Alkyl)-carbonylgruppen,

$R_{10}$ ein Wasserstoffatom, eine $C_1$-$C_4$-Alkoxygruppe, ein Halogenatom, eine Hy- droxylgruppe, eine Benzyloxygruppe, eine Nitrogruppe, eine Aminogruppe, eine ($C_1$-$C_4$-Alkyl)-aminogruppe, eine Di-($C_1$-$C_4$-alkyl)-aminogruppe, eine Sulfonamidogruppe, eine ($C_1$-$C_4$-Alkyl)-sulfonamidogruppe, eine Phenylsulfonamidogruppe, eine Cyanogruppe, eine ($C_1$-$C_4$-Alkoxy)-carbonylgruppe oder eine ($C_1$-$C_4$-Al-

kyl)-carbonylgruppe,
wobei die Gruppen $R_7$ und $R_8$ einerseits und $R_7$, $R_8$, $R_9$ und $R_{10}$ andererseits niemals gleichzeitig Wasserstoffatome bedeuten und der Rest (I) oder (I') keinen Monohalogenophenylrest darstellt,
$R_{11}$ eine $C_1$-$C_4$-Alkylgruppe, ein Halogenatom, eine Hydroxylgruppe, eine Benzyloxygruppe, eine Nitrogruppe, eine Aminogruppe, eine ($C_1$-$C_4$-Alkyl)-aminogruppe, eine Di-($C_1$-$C_4$-alkyl)-aminogruppe, eine Sulfonamidogruppe, eine ($C_1$-$C_4$-Alkyl)-sulfonamidogruppe, eine Phenylsulfonamidogruppe, eine Cyanogruppe, eine ($C_1$-$C_4$-Alkoxy)-carbonylgruppe oder eine $C_1$-$C_4$-Alkyl)-carbonylgruppe,
Q eine Gruppe der Formel

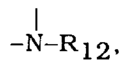

worin $R_{12}$ ein Wasserstoffatom, eine $C_1$-$C_4$-Alkylgruppe, eine $C_3$-$C_6$-Cycloalkylgruppe, eine Benzylgruppe, eine Halogenbenzylgruppe, eine Gruppe der Formel -A-Am, wie sie oben definiert worden ist, eine ($C_1$-$C_4$-Alkyl)-sulfonylgruppe oder eine Phenylsulfonylgruppe, die gegebenenfalls durch eines oder mehrere Halogenatome, $C_1$-$C_4$-Alkylgruppen oder $C_1$-$C_4$-Alkoxygruppen substituiert ist, und
$R'_{12}$ eine Gruppe der Formel -A-Am, wie sie oben definiert worden ist, eine Alkylsulfonylgruppe, die gegebenenfalls durch eines oder mehrere Halogenatome, $C_1$-$C_4$-Alkylgruppen oder $C_1$-$C_4$-Alkoxygruppen substituiert ist, darstellen, bedeuten,
**dadurch gekennzeichnet**, daß man

A/ wenn A eine Alkylengruppe und B eine Gruppe -S- oder -$SO_2$- bedeuten, entweder
ein 4-Alkoxyphenyl-Derivat der allgemeinen Formel:

Cy - B' - Ph - O - A - X (2)

worin B' eine Gruppe -S- oder -$SO_2$- darstellt, Cy und Ph die oben angegebenen Bedeutungen besitzen, A die oben angegebenen Bedeutungen besitzt und X ein Halogenatom oder eine Alkylsulfonyloxygruppe mit 1 bis 4 Kohlenstoffatomen oder eine Arylsulfonyloxygruppe mit 6 bis 10 Kohlenstoffatomen darstellt,
in einem polaren oder nichtpolaren Lösungsmittel bei einer Temperatur zwischen Raumtemperatur und der Rückflußtemperatur mit einem Amin der allgemeinen Formel:

H - Am (3)

in der Am die oben angegebenen Bedeutungen besitzt, kondensiert zur Bildung des gewünschten Derivats,

- oder ein 4-Hydroxyphenyl-Derivat der allgemeinen Formel:

Cy - B' - Ph - OH (4)

in der B' eine Gruppe -S- oder -$SO_2$- darstellt und Cy und Ph die oben angegebenen Bedeutungen besitzen, in Gegenwart eines basischen Mittels mit einer Verbindung der allgemeinen Formel:

X - A - Am (31)

in der X ein Halogenatom oder eine Alkylsulfoxygruppe mit 1 bis 4 Kohlenstoffatomen oder eine Arylsulfoxygruppe mit 6 bis 10 Kohlenstoffatomen darstellt, A eine Alkylengruppe bedeutet und Am die oben angegebenen Bedeutungen besitzt, bei der Rückflußtemperatur in einem geeigneten Lösungsmittel umsetzt;

B/ wenn A eine gegebenenfalls substituierte 2-Hydroxypropylengruppe und B eine Gruppe -S- oder -$SO_2$- bedeuten, ein Oxyranylmethoxy-Derivat der allgemeinen Formel:

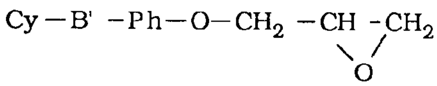 (33)

in der B' eine Gruppe -S- oder -$SO_2$- darstellt und Cy und Ph die oben angegebe- nen Bedeutungen besitzen, bei der Rückflußtemperatur mit einem Amin der all- gemeinen Formel:

H - Am (3)

in der Am die oben angegebenen Bedeutungen besitzt, in einem polaren Lösungsmittel oder in einem Überschuß dieses Amins behandelt, so daß man

- das gewünschte Derivat in Form der freien Base, worin A eine 2-Hydroxypropylengruppe darstellt, oder
- ein Aminoalkoxyphenyl-Derivat erhält, welches man mit einem Alkylhalogenid mit 1 bis 4 Kohlenstoffatomen in Gegenwart einer starken Base umsetzen kann zur Bildung des gewünschten Derivats in Form der freien Base, worin A eine 2-Hydroxypropylengruppe darstellt, in der die Hydroxylgruppe durch eine Alkylgruppe mit 1 bis 4 Kohlenstoffatomen substituiert ist, und

C/ wenn B eine Gruppe -SO- darstellt, ein Sulfid der allgemeinen Formel:

$$Cy - S - Ph - O - A - Am \qquad (1)$$

in der Cy, Ph, A und Am die oben angegebenen Bedeutungen besitzen, welches Sulfid in Form der freien Base oder des Salzes vorliegt, mit einem Oxidationsmittel behandelt zur Bildung des gewünschten Derivats in Form der freien Base oder des Salzes, welches man mit einem basischen Mittel behandelt zur Bildung des gewünschten Derivats in Form der freien Base, welche freie Base gewünschtenfalls mit einer organischen oder anorganischen Säure umgesetzt werden kann zur Bildung eines pharmazeutisch annehmbaren Salzes dieses Derivats.

2. Verfahren nach Anspruch 1, **dadurch gekennzeichnet**, daß das Oxidationsmittel Natriumperiodat, Kaliumpermanganat oder 3-Chlor-perbenzoesäure ist.

3. Verfahren zur Herstellung von Aminoalkoxyphenyl-Derivaten der Formel (1) nach Anspruch 1, worin $R_7$, $R_8$, $R_9$, $R_{10}$ oder $R_{11}$ eine Hydroxylgruppe bedeuten, **dadurch gekennzeichnet**, daß man eine Verbindung der Formel (1) nach Anspruch 1, worin $R_7$, $R_8$, $R_9$, $R_{10}$ oder $R_{11}$ eine Benzyloxygruppe bedeuten, in einem geeigneten Lösungsmittel und in Gegenwart eines Katalysators hydriert zur Bildung des gewünschten Derivats, welches man erforderlichenfalls mit einer geeigneten organischen oder anorganischen Säure umsetzen kann zur Bildung eines pharmazeutisch annehmbaren Salzes.

4. Verfahren zur Herstellung von Aminoalkoxyphenyl-Derivaten der Formel (1) nach Anspruch 1, worin $R_7$, $R_8$, $R_9$, $R_{10}$ oder $R_{11}$ eine Aminogruppe, die Niedrigalkylaminogruppe oder eine Diniedrigalkylaminogruppe bedeuten, **dadurch gekennzeichnet**, daß man eine Verbindung der Formel (1) nach Anspruch 1. worin $R_7$, $R_8$, $R_9$, $R_{10}$ oder $R_{11}$ eine Nitrogruppe bedeuten, in einem geeigneten Lösungsmittel in Gegenwart eines Katalysators hydriert, so daß man
   - die gewünschte Verbindung in Form der freien Base, in der $R_7$, $R_8$, $R_9$, $R_{10}$ oder $R_{11}$ eine Aminogruppe bedeuten, oder
   - ein Aminoalkoxyphenyl-Derivat erhält, welches man mit einer geeigneten Menge eines Alkylhalogenids mit 1 bis 4 Kohlenstoffatomen und in Gegenwart eines alkalischen Mittels umsetzen kann zur Bildung des gewünschten Derivats in Form der freien Base, in dem $R_7$, $R_8$, $R_9$, $R_{10}$ oder $R_{11}$ eine ($C_1$-$C_4$-Alkyl)-aminogruppe oder eine Di-($C_1$-$C_4$-alkyl)-aminogruppe bedeuten,

   welches in dieser Weise erhaltene Aminoalkoxyphenyl-Derivat man gewünschtenfalls mit einer organischen oder anorganischen Säure umsetzen kann zur Bildung eines pharmazeutisch annehmbaren Salzes.

5. Verfahren nach Anspruch 1, **dadurch gekennzeichnet**, daß das Oxidationsmittel Natriumperiodat, Kaliumpermanganat oder 3-Chlor-perbenzoesäure ist.

6. Verfahren nach Anspruch 1, **dadurch gekennzeichnet**, daß man Aminoalkoxyphenyl-Derivate der Formel (1) herstellt, in der B eine Gruppe $-SO_2-$ darstellt.

7. Verfahren nach Anspruch 1, **dadurch gekennzeichnet**, daß man Aminoalkoxyphenyl-Derivate der Formel (1) herstellt, in der $R_1$ und $R_2$ jeweils Wasserstoff bedeuten.

8. Verfahren nach Anspruch 1, **dadurch gekennzeichnet**, daß man Amino- alkoxyphenyl-Derivate der Formel (1) herstellt, in der R eine Isopropylgruppe oder eine Cyclopropylgruppe bedeutet.

9. Verfahren nach Anspruch 1, **dadurch gekennzeichnet**, daß man Aminoalkoxyphenyl-Derivate der Formel (1) herstellt, in der $R_5$, $R'_5$ und $R''_5$, die gleichartig oder verschieden sind, jeweils Wasserstoffatome, Chloratome, Methylgruppen oder Methoxygruppen bedeuten, herstellt.

10. Verfahren nach Anspruch 1, **dadurch gekennzeichnet**, daß man Aminoalkoxyphenyl-Derivate der Formel (1) herstellt, in der $R_3$ eine Gruppe -Alk-Ar darstellt.

11. Verfahren nach Anspruch 1, **dadurch gekennzeichnet**, daß man Aminoalkoxyphenyl-Derivate der Formel (1) herstellt, in der die Kette $-O-A-NR_3-R_4$ eine [N-Methyl-N-(3,4-dimethoxy-β-phenethyl)-amino]-propoxygruppe oder eine [N-Methyl-N-(3,5-dimethoxy-β-phenethyl)-amino]-propoxygruppe bedeutet.

12. Verfahren nach Anspruch 1, **dadurch gekennzeichnet**, daß man Aminoalkoxyphenyl-Derivate der Formel (1) herstellt, in der Ph eine Gruppe (D) darstellt.

13. Verfahren nach Anspruch 1, **dadurch gekennzeichnet**, daß man Aminoalkoxyphenyl-Derivate der Formel (1) herstellt, in der Cy eine Gruppe (J) bedeutet.

14. Verfahren nach Anspruch 1, **dadurch gekennzeichnet**, daß man Aminoalkoxyphenyl-Derivate der Formel (1) herstellt, in der Cy eine Gruppe (K) oder (K') bedeutet.

15. Verfahren nach Anspruch 1, **dadurch gekennzeichnet**, daß man Aminoalkoxyphenyl-Derivate der Formel (1) herstellt, in der Cy eine Gruppe (L) bedeutet.

16. Verfahren nach Anspruch 1, **dadurch gekennzeichnet**, daß manAminoalkoxyphenyl-Derivate der Formel (1) herstellt, in der das pharmazeutisch annehmbare Salz das Oxalat, Hydrochlorid oder Fumarat ist.

17. Verfahren nach Anspruch 1, **dadurch gekennzeichnet**, daß manAminoalkoxyphenyl-Derivate der Formel (1) herstellt, ausgewählt aus den folgenden Verbindungen:
2-Isopropyl-8-methyl-1-[4-{3-[N-methyl-N-(3,5-dimethoxy-β-phenethyl)-amino]-propoxy}-benzolsulfonyl]-indolizin,
2-Isopropyl-8-methyl-1-[4-{3-[N-methyl-N-(3,4-dimethoxy-β-phenethyl)-amino]-propoxy}-benzolsulfonyl]-indolizin,
1-{4-[3-(Di-n-butylamino)-propoxy]-benzolsulfonyl}-2-isopropyl-8-methyl-indolizin,
2-Isopropyl-8-methyl-1-{4-[3-(6,7-dimethoxy-1,2,3,4-tetrahydro-isochinolin-2-yl)-propoxy]-benzolsulfonyl}-indolizin,
2-Isopropyl-5-methyl-1-[4-{3-[N-methyl-N-(3,4-dimethoxy-β-phenethyl)-amino]-propoxy}-benzolsulfonyl]-indolizin,
2-[4-{3-[N-Methyl-N-(3,4-dimethoxy-β-phenethyl)-amino]-propoxy}-benzolsulfonyl]-5-chlor-3-isopropyl-4-methyl-indol,
und deren pharmazeutisch annehmbaren Salzen.

18. Verfahren zur Herstellung von pharmazeutischen oder veterinärmedizinischen Zubereitungen, **dadurch gekennzeichnet**, daß man als Wirkstoff mindestens ein Aminoalkoxyphenyl-Derivat, hergestellt nach einem der vorhergehenden Ansprüche, zu einem geeigneten pharmazeutischen Trägermaterial oder Bindemittel zusetzt.

19. Verfahren zur Herstellung von pharmazeutischen oder veterinärmedizinischen Zubereitungen nach Anspruch 18, enthaltend 50 mg bis 500 mg des Wirkstoffs zur Behandlung von pathologischen Syndromen des kardiovaskulären Systems.

20. Verfahren zur Herstellung von pharmazeutischen oder veterinärmedizinischen Zubereitungen nach Anspruch 18 zur Behandlung von pathologischen Zuständen der Augen.

**Patentansprüche für folgenden Vertragsstaat : GR**

1. Aminoalkoxyphenyl-Derivate der allgemeinen Formel:

$$Cy - B - PH - O - A - Am \qquad (1)$$

sowie deren pharmazeutisch annehmbare Salze, worin
B eine Gruppe -S-, -SO- oder $-SO_2-$,
Ph eine Gruppe der Formel

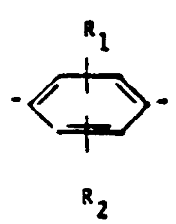

(D)

oder

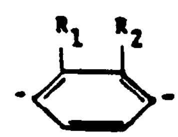

(E)

$R_1$ und $R_2$, die gleichartig oder verschieden sein können, jeweils Wasserstoffatome, Methyl- oder Ethylgruppen oder Halogenatome,
A eine geradkettige oder verzweigte Alkylengruppe mit 2 bis 5 Kohlenstoffatomen oder den 2-Hydroxypropylenrest, worin die Hydroxylgruppe gegebenenfalls durch eine $C_1$-$C_4$-Alkylgruppe substituiert ist,
Am eine Gruppe der Formeln

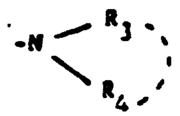

(F)

oder

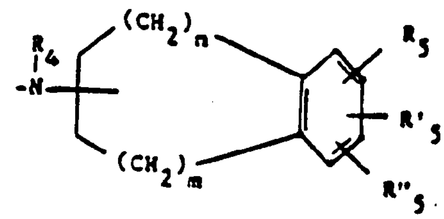

(G)

oder

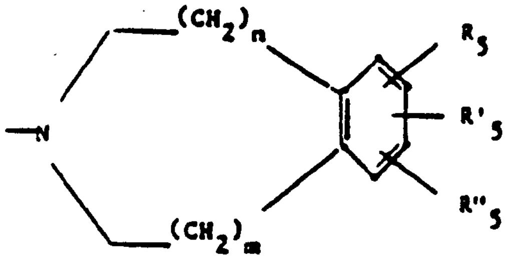

(H)

worin
$R_3$ eine $C_1$-$C_8$-Alkylgruppe, eine $C_3$-$C_6$-Cycloalkylgruppe oder eine Gruppe der Formel

- Alk - Ar

in der Alk eine Einfachbindung oder eine geradkettige oder verzweigte Alkylengruppe mit 1 bis 5 Kohlenstoffatomen und Ar einen Pyridyl-, Phenyl-, 2,3-Methylendioxy-phenyl- oder 3,4-Methylendioxyphenylrest oder eine Phenylgruppe, die durch einen oder mehrere gleichartige oder verschiedene Substituenten ausgewählt aus Halogenatomen, $C_1$-$C_4$-Alkylgruppen oder $C_1$-$C_4$-Alkoxygruppen substituiert ist, darstellen,
$R_4$ ein Wasserstoffatom oder eine $C_1$-$C_8$-Alkylgruppe oder
$R_3$ und $R_4$ gemeinsam eine Alkylen- oder Alkenylengruppe mit 3 bis 6 Kohlenstoffatomen, die gegebenenfalls durch einen Phenylrest substituiert oder gegebenenfalls durch -O-, -N= oder

$$-\overset{|}{N}-R_6$$

unterbrochen sein kann, worin $R_6$ ein Wasserstoffatom, eine $C_1$-$C_4$-Alkylgruppe, eine $C_3$-$C_6$-Cycloalkylgruppe, eine durch ein Halogenatom oder eine $C_1$-$C_4$-Alkylgruppe oder eine $C_1$-$C_4$-Alkoxygruppe substituierte Phenylgruppe darstellt,
$R_5$, $R'_5$ und $R''_5$, die gleichartig oder verschieden sein können, jeweils Wasserstoffatome, Halogenatome, $C_1$-$C_4$-Alkylgruppen oder $C_1$-$C_4$-Alkoxygruppen,
n und m, die gleichartig oder verschieden sein können, jeweils 0, 1, 2 oder 3 darstellen und
Cy eine der Gruppen der Formel:

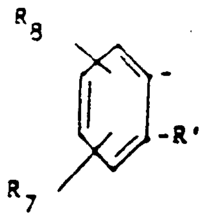

(I)

oder

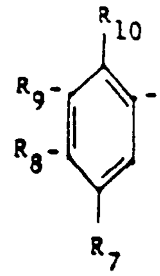

(I')

oder

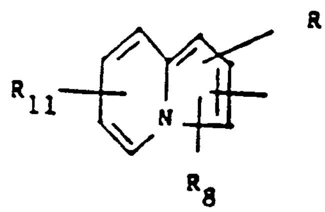

(J)

oder

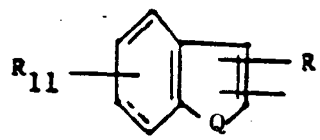

(K)

oder

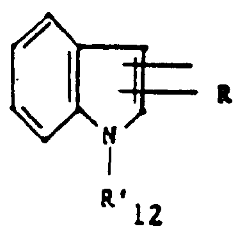

(K')

oder

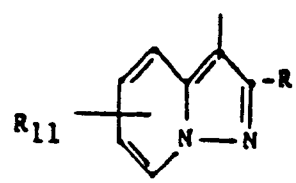

(L)

oder

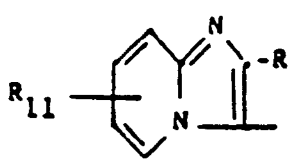

(M)

oder

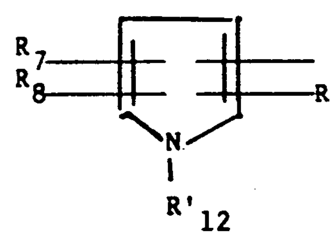

(N)

oder 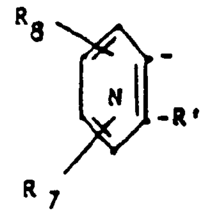 oder 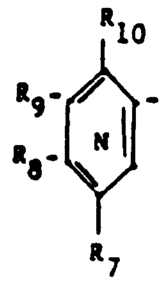

(Q) (Q')

worin
R ein Wasserstoffatom, eine $C_1$-$C_8$-Alkylgruppe, eine $C_3$-$C_6$-Cycloalkylgruppe, eine Benzylgruppe oder eine Phenylgruppe, die gegebenenfalls durch einen oder mehrere Substituenten, die gleichartig oder verschieden sein können und aus Halogenatomen, $C_1$-$C_4$-Alkylgruppen, $C_1$-$C_4$-Alkoxygruppen oder Nitrogruppen ausgewählt sind, substituiert ist,
R' eine $C_1$-$C_8$-Alkylgruppe, eine $C_3$-$C_6$-Cycloalkylgruppe, eine Benzylgruppe oder eine Phenylgruppe, die gegebenenfalls durch einen oder mehrere Substituenten, die gleichartig oder verschieden sein können, und aus Halogenatomen, $C_1$-$C_4$-Alkylgruppen, $C_1$-$C_4$-Alkoxygruppen oder Nitrogruppen ausgewählt sind, substituiert ist,
$R_7$, $R_8$ und $R_9$, die gleichartig oder verschieden sein können, jeweils Wasserstoffatome, $C_1$-$C_4$-Alkylgruppen, $C_1$-$C_4$-Alkoxygruppen, Halogenatome, Hydroxylgruppen, Benzyloxygruppen, Nitrogruppen, Aminogruppen, ($C_1$-$C_4$-Alkyl)-aminogruppen, Di-($C_1$-$C_4$-alkyl)-aminogruppen, Sulfonamidogruppen, ($C_1$-$C_4$-Alkyl)-sulfonamidogruppen, Phenylsulfonamidogruppen, Cyanogruppen, (C1-$C_4$-Alkoxy)-carbonylgruppen oder ($C_1$-$C_4$-Alkyl)-carbonylgruppen,
$R_{10}$ ein Wasserstoffatom, eine $C_1$-$C_4$-Alkoxygruppe, ein Halogenatom, eine Hydroxylgruppe, eine Benzyloxygruppe, eine Nitrogruppe, eine Aminogruppe, eine ($C_1$-$C_4$-Alkyl)-aminogruppe, eine Di-($C_1$-$C_4$-alkyl)-aminogruppe, eine Sulfonamidogruppe, eine ($C_1$-$C_4$-Alkyl)-sulfonamidogruppe, eine Phenylsulfonamidogruppe, eine Cyanogruppe, eine ($C_1$-$C_4$-Alkoxy)-carbonylgruppe oder eine ($C_1$-$C_4$-Alkyl)-carbonylgruppe,
wobei die Gruppen $R_7$ und $R_8$ einerseits und $R_7$, $R_8$, $R_9$ und $R_{10}$ andererseits niemals gleichzeitig Wasserstoffatome bedeuten und der Rest (I) oder (I') keinen Monohalogenophenylrest darstellt,
$R_{11}$ eine $C_1$-$C_4$-Alkylgruppe, ein Halogenatom. eine Hydroxylgruppe, eine Benzyloxygruppe, eine Nitrogruppe, eine Aminogruppe, eine ($C_1$-$C_4$-Alkyl)-aminogruppe, eine Di-($C_1$-$C_4$-alkyl)-aminogruppe, eine Sulfonamidogruppe, eine ($C_1$-$C_4$-Alkyl)-sulfonamidogruppe, eine Phenylsulfonamidogruppe, eine Cyanogruppe, eine ($C_1$-$C_4$-Alkoxy)-carbonylgruppe oder eine $C_1$-$C_4$-Alkyl)-carbonylgruppe,
Q eine Gruppe der Formel

$$-\overset{|}{N}-R_{12},$$

worin $R_{12}$ ein Wasserstoffatom, eine $C_1$-$C_4$- Alkylgruppe, eine $C_3$-$C_6$-Cycloalkylgruppe, eine Benzylgruppe, eine Halogenbenzylgruppe, eine Gruppe der Formel -A-Am, wie sie oben definiert worden ist, eine ($C_1$-$C_4$-Alkyl)-sulfonylgruppe oder eine Phenylsulfonylgruppe, die gegebenenfalls durch eines oder mehrere Halogenatome, $C_1$-$C_4$-Alkylgruppen oder $C_1$-$C_4$-Alkoxygruppen substituiert ist, und
$R'_{12}$ eine Gruppe der Formel -A-Am, wie sie oben definiert worden ist, eine Alkylsulfonylgruppe, die gegebenenfalls durch eines oder mehrere Halogenatome, $C_1$-$C_4$-Alkylgruppen oder $C_1$-$C_4$-Alkoxygruppen substituiert ist, darstellen, bedeuten.

**2.** Aminoalkoxyphenyl-Derivate nach Anspruch 1, worin B eine Gruppe der Formel -$SO_2$- bedeutet.

**3.** Aminoalkoxyphenyl-Derivate nach Anspruch 1 oder 2, **dadurch gekennzeichnet**, daß $R_1$ und $R_2$ jeweils Wasserstoff bedeuten.

**4.** Aminoalkoxyphenyl-Derivate nach einem der Ansprüche 1 bis 3, **dadurch gekennzeichnet**, daß R eine Isopropylgruppe oder eine Cyclopropylgruppe bedeutet.

5. Aminoalkoxyphenyl-Derivate nach einem der Ansprüche 1 bis 4, **dadurch gekennzeichnet**, daß $R_5$, $R'_5$ und $R''_5$, die gleichartig oder verschieden sein können, jeweils Wasserstoff, Chlor, eine Methylgruppe oder eine Methoxygruppe bedeuten.

6. Aminoalkoxyphenyl-Derivate nach einem der Ansprüche 1 bis 5, **dadurch gekennzeichnet**, daß $R_3$ eine Gruppe der Formel -Alk-Ar bedeutet.

7. Aminoalkoxyphenyl-Derivate nach einem der Ansprüche 1 bis 6, **dadurch gekennzeichnet**, daß die Kette -O-A-$NR_3$-$R_4$ eine [N-Methyl-N-(3,4-dimethoxy-β-phenethyl)-amino]-propoxygruppe oder eine [N-Methyl-N-(3,5-dimethoxy-β-phenethyl)-amino]-propoxygruppe bedeutet.

8. Aminoalkoxyphenyl-Derivate nach einem der Ansprüche 1 bis 7, **dadurch gekennzeichnet**, daß Ph eine Gruppe (D) bedeutet.

9. Aminoalkoxyphenyl-Derivate nach einem der Ansprüche 1 bis 7, **dadurch gekennzeichnet**, daß Cy eine Gruppe (J) bedeutet.

10. Aminoalkoxyphenyl-Derivate nach einem der Ansprüche 1 bis 7, **dadurch gekennzeichnet**, daß Cy eine Gruppe (K) oder (K') bedeutet.

11. Aminoalkoxyphenyl-Derivate nach einem der Ansprüche 1 bis 7, **dadurch gekennzeichnet**, daß Cy eine Gruppe (L) bedeutet.

12. Aminoalkoxyphenyl-Derivate nach einem der Ansprüche 1 bis 11, **dadurch gekennzeichnet**, daß das pharmazeutisch annehmbare Salz das Oxalat, Hydrochlorid oder Fumarat ist.

13. Aminoalkoxyphenyl-Derivate nach Anspruch 1, ausgewählt aus den folgenden Verbindungen:
2-Isopropyl-8-methyl-1-[4-{3-[N-methyl-N-(3,5-dimethoxy-β-phenethyl)-amino]-propoxy}-benzolsulfonyl]-indolizin,
2-Isopropyl-8-methyl-1-[4-{3-[N-methyl-N-(3,4-dimethoxy-β-phenethyl)-amino]-propoxy}-benzolsulfonyl]-indolizin,
1-{4-[3-(Di-n-butylamino)-propoxy]benzolsulfonyl}-2-isopropyl-8-methyl-indolizin,
2-Isopropyl-8-methyl-1-{4-[3-(6,7-dimethoxy-1,2,3,4-tetrahydro-isochinolin-2-yl)-propoxy]-benzolsulfonyl}-indolizin,
2-Isopropyl-5-methyl-1-[4-{3-[N-methyl-N-(3,4-dimethoxy-β-phenethyl)-amino]-propoxy}-benzolsulfonyl]-indolizin,
2-[4-{3-[N-Methyl-N-(3,4-dimethoxy-β-phenethyl)-amino]-propoxy}-benzolsulfonyl]-5-chlor-3-isopropyl-4-methyl-indol,
und deren pharmazeutisch annehmbaren Salzen.

14. Verfahren zur Herstellung der Aminoalkoxyphenyl-Derivate der Formel (1) nach Anspruch 1, worin A eine Alkylengruppe und B eine Gruppe -S- oder -$SO_2$- bedeuten, **dadurch gekennzeichnet**, daß man ein 4-Alkoxyphenyl-Derivat der allgemeinen Formel

$$Cy - B' - Ph - O - A - X \qquad (2)$$

in der B' eine Gruppe -S- oder -$SO_2$- bedeutet, Cy und Ph die in Anspruch 1 angegebenen Bedeutungen besitzen, A die oben angegebene Bedeutung besitzt und X ein Halogenatom oder eine Alkylsulfonyloxygruppe mit 1 bis 4 Kohlenstoffatomen oder eine Arylsulfonyloxygruppe mit 6 bis 10 Kohlenstoffatomen darstellt, in Gegenwart eines Säureakzeptors in einem polaren oder nichtpolaren Lösungsmittel bei einer Temperatur zwischen Raumtemperatur und der Rückflußtemperatur mit einem Amin der allgemeinen Formel:

$$H - Am \qquad (3)$$

worin Am die in Anspruch 1 angegebenen Bedeutungen besitzt, kondensiert zur Bildung des gewünschten Derivats, welches man erforderlichenfalls mit einer organischen oder anorganischen Säure umsetzen kann zur Bildung eines pharmazeutisch annehmbaren Salzes dieses Derivats.

15. Verfahren zur Herstellung der Aminoalkoxyphenyl-Derivate der Formel (1) nach Anspruch 1, worin B eine Gruppe -S- oder -$SO_2$- bedeutet, **dadurch gekennzeichnet**, daß man ein 4-Hydroxyphenyl-Derivat der allgemeinen Formel:

$$Cy - B' - Ph - OH \qquad (4)$$

in der B' eine Gruppe -S- oder $-SO_2-$ bedeutet und Cy und Ph die in Anspruch 1 angegebenen Bedeutungen besitzen, in Gegenwart eines basischen Mittels mit einer Verbindung der allgemeinen Formel:

X - A - Am (31)

in der X ein Halogenatom oder eine Alkylsulfoxygruppe mit 1 bis 4 Kohlenstoffatomen oder eine Arylsulfoxygruppe mit 6 bis 10 Kohlenstoffatomen und A eine Alkylengruppe bedeuten und Am die in Anspruch 1 angegebenen Bedeutungen besitzt, bei der Rückflußtemperatur in einem geeigneten Lösungsmittel umsetzt zur Bildung des gewünschten Derivats, welches man gewünschtenfalls mit einer geeigneten organischen oder anorganischen Säure umsetzen kann zur Bildung eines pharmazeutisch annehmbaren Salzes.

**16.** Verfahren zur Herstellung der Aminoalkoxyphenyl-Derivate der Formel (1) nach Anspruch 1, worin A den 2-Hydroxypropylenrest, der gegebenenfalls substituiert sein kann, und B eine Gruppe -S- oder $-SO_2-$ bedeuten, **dadurch gekennzeichnet**, daß man ein Oxyranylmethoxy-Derivat der allgemeinen Formel:

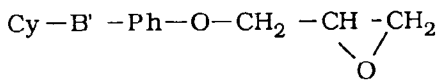

(33)

in der B' eine Gruppe -S- oder $-SO_2-$ bedeutet und Cy und Ph die in Anspruch 1 angegebenen Bedeutungen besitzen, bei der Rückflußtemperatur mit einem Amin der allgemeinen Formel:

H - Am (3)

in der Am die in Anspruch 1 angegebenen Bedeutungen besitzt, in einem polaren Lösungsmittel oder in einem Überschuß des genannten Amins behandelt, so daß man

- das gewünschte Derivat in Form der freien Base, worin A eine 2-Hydroxypropylengruppe darstellt, oder
- ein Aminoalkoxyphenyl-Derivat erhält, welches man mit einem Alkylhalogenid mit 1 bis 4 Kohlenstoffatomen in Gegenwart einer starken Base umsetzen kann, so daß man das gewünschte Derivat in Form der freien Base erhält, worin A eine 2-Hydroxypropylengruppe darstellt, in der die Hydroxylgruppe durch eine Alkylgruppe mit 1 bis 4 Kohlenstoffatomen substituiert ist,

wobei man das in dieser Weise erhaltene Aminoalkoxyphenyl-Derivat gewünschtenfalls mit einer organischen oder anorganischen Säure umsetzen kann zur Bildung eines pharmazeutisch annehmbaren Salzes.

**17.** Verfahren zur Herstellung von Aminoalkoxyphenyl-Derivaten der Formel (1) nach Anspruch 1, worin B eine Gruppe -SO- bedeutet, **dadurch gekennzeichnet**, daß man ein Sulfid der allgemeinen Formel

Cy - S - Ph - O - A - Am (1)

in der Cy, Ph, A und Am die in Anspruch 1 angegebenen Bedeutungen besitzen, welches Sulfid in Form der freien Base oder eines Salzes vorliegt, mit einem Oxidationsmittel behandelt zur Bildung des gewünschten Derivats in Form der freien Base oder des Salzes, welches man mit einem basischen Mittel behandelt zur Bildung des gewünschten Derivats in Form der freien Base, welche freie Base gewünschtenfalls mit einer organischen oder anorganischen Säure umgesetzt werden kann zur Bildung eines pharmazeutisch annehmbaren Salzes dieses Derivats.

**18.** Verfahren nach Anspruch 17, **dadurch gekennzeichnet**, daß das Oxidationsmittel Natriumperiodat, Kaliumpermanganat oder 3-Chlor-perbenzoesäure ist.

**19.** Verfahren zur Herstellung von Aminoalkoxyphenyl-Derivaten der Formel (1) nach Anspruch 1, worin $R_7$, $R_8$, $R_9$, $R_{10}$ oder $R_{11}$ eine Hydroxylgruppe bedeuten, **dadurch gekennzeichnet**, daß man eine Verbindung der Formel (1) nach Anspruch 1, worin $R_7$, $R_8$, $R_9$, $R_{10}$ oder $R_{11}$ eine Benzyloxygruppe bedeu- ten, in einem geeigneten Lösungsmittel und in Gegenwart eines Katalysators hydriert zur Bildung des gewünschten Derivats, welches man erforderlichenfalls mit einer geeigneten organischen oder anorganischen Saure umsetzen kann zur Bildung eines pharmazeutisch annehmbaren Salzes.

**20.** Verfahren zur Herstellung von Aminoalkoxyphenyl-Derivaten der Formel (1) nach Anspruch 1, worin $R_7$, $R_8$, $R_9$, $R_{10}$ oder $R_{11}$ eine Aminogruppe, die Nie- drigalkylaminogruppe oder eine Diniedrigalkylaminogruppe bedeuten, **dadurch gekennzeichnet**, daß man eine Verbindung der Formel (I) nach Anspruch 1, worin $R_7$, $R_8$, $R_9$, $R_{10}$ oder $R_{11}$ eine Nitrogruppe bedeuten, in einem geeigneten Lösungsmittel in Gegenwart eines Katalysators hydriert, so daß man

- die gewünschte Verbindung in Form der freien Base, in der $R_7$, $R_8$, $R_9$, $R_{10}$ oder $R_{11}$ eine Aminogruppe bedeuten, oder
- ein Aminoalkoxyphenyl-Derivat erhält, welches man mit einer geeigneten Menge eines Alkylhalogenids mit 1 bis 4 Kohlenstoffatomen und in Gegenwart eines alkalischen Mittels umsetzen kann zur Bildung des gewünschten Derivats in Form der freien Base, in dem $R_7$, $R_8$, $R_9$, $R_{10}$ oder $R_{11}$ eine ($C_1$-$C_4$-Alkyl)-aminogruppe oder eine Di-($C_1$-$C_4$-alkyl)-aminogruppe bedeuten,

welches in dieser Weise erhaltene Aminoalkoxyphenyl-Derivat man gewünschtenfalls mit einer organischen oder anorganischen Säure umsetzen kann zur Bildung eines pharmazeutisch annehmbaren Salzes.

**21.** Verfahren zur Herstellung von pharmazeutische oder veterinärme- dizinische Zubereitungen dadurch gekennzeichnet daB man mindestens ein Aminoalkoxyphenyl-Derivat nach einem der Ansprüche 1 bis 13 zu einem geeigneten pharmazeutischen Trägermaterial oder Bindemittel zusetzt.

**22.** Verfahren zur Herstellung von pharmazeutische oder vetererinärmedizinische Zubereitungen nach Anspruch 21 zur Behandlung von pathologischen Syndromen des kardiovaskulären Systems, enthaltend 50 mg bis 500 mg des Wirkstoffs.

**23.** Verfahren zur Herstellung von pharmazeutische oder veterinärme- dizinische Zubereitungen nach Ansprüche 21 zur Behandlung von patholo- gischen Zuständen des Auges.